# EUROPEAN PATENT SPECIFICATION

(11) **EP 1 831 149 B1**
(45) Date of publication and mention of the grant of the patent: **25.01.2012**
(21) Application number: 05824484.9
(22) Date of filing: 21.12.2005
(51) Int. Cl.: C07C 229/52, C07C 233/54, C07D 295/155, C07D 207/27, C07D 207/325

(54) **NOVEL LIGANDS THAT MODULATE RAR RECEPTORS AND USE THEREOF IN HUMAN MEDICINE AND IN COSMETICS**
NEUE LIGANDEN ALS RAR-REZEPTORMODULATOREN UND DEREN VERWENDUNG IN DER HUMANMEDIZIN UND KOSMETIK
NOUVEAUX LIGANDS QUI MODULENT LES RECEPTEURS RAR, ET LEUR UTILISATION EN MEDECINE ET EN COSMETIQUE

(30) Priority: 23.12.2004 FR 0413848; 28.01.2005 US 647383 P
(43) Date of publication of application: 12.09.2007
(73) Proprietor: GALDERMA RESEARCH & DEVELOPMENT, 06410 Biot (FR)
(72) Inventor: BIADATTI, Thibaud, F-06650 Opio (FR); DUMAIS, Laurence, F-06650 Le Rouret (FR); SOULET, Catherine, F-06600 Antibes (FR); TALANO, Sandrine, F-06140 Vence (FR); DAVER, Sébastien, F-06600 Antibes (FR)
(74) Representative: Allab, Myriam
(86) International application number: PCT/EP2005/014217
(87) International publication number: WO 2006/066978

(56) References cited:
- WO-A-99/10308
- US-A- 6 150 413

## Description

The invention relates to novel compounds as novel and useful industrial products, which are ligands that modulate RAR receptors. The invention also relates to compositions containing them, to processes for preparing them and to their use in pharmaceutical compositions for use in human or veterinary medicine, or alternatively in cosmetic compositions, and to the non-therapeutic use of these compositions.

Compounds with activity of retinoid type (vitamin A and its derivatives) are widely described in the literature as having activity in cell proliferation and differentiation processes. These properties give this class of compounds high potential in the treatment or prevention of numerous pathologies, and more particularly in dermatology and cancer. Many biological effects of retinoids are mediated by modulating the nuclear retinoic acid receptors (RAR).

The RAR receptors activate transcription by binding to DNA sequence elements, known as RAR response elements (RARE), in the form of a heterodimer with the retinoid X receptors (known as RXRs).

Three subtypes of human RARs have been identified and described: RARα, RARβ and RARγ.

The prior art contains a large number of chemical compounds that are RAR type receptor ligands. Among the prior art documents, examples that may be mentioned include patent US 6 150 413, which describes triaromatic compounds, patent US 6 214 878, which describes stilbene compounds, or patent US 6 218 128, which describes a family of bicyclic or tricyclic molecules.

The Applicant has invented novel compounds that modulate retinoic acid receptors.

Thus, the present invention relates to compounds corresponding to the general formula (I) below: 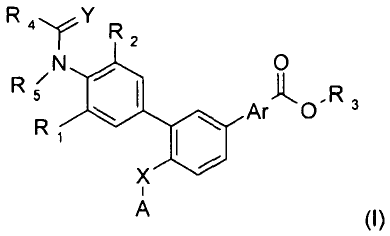 in which:
- R₁ is a hydrogen atom, an alkyl radical of 1 to 4 carbon atoms or a -CF₃ radical;
- R₂ is a hydrogen atom, an alkyl or alkoxy radical of 1 to 4 carbon atoms or a chlorine atom;
- R₃ is a hydrogen atom, a linear or branched alkyl of 1 to 10 carbon atoms and preferably 1 to 6 carbon atoms optionally substituted with a methoxy group, or alternatively a linear or branched alkyl radical of 1 to 10 carbon atoms and preferably 1 to 6 carbon atoms containing an ether function;
- R₄ is a hydrogen atom or an alkyl radical of 1 to 3 carbon atoms;
- R₅ is a hydrogen atom or an alkyl radical of 1 to 3 carbon atoms;
- or alternatively R₄ and R₅ form, together with the bond -N-C(=Y)-, a ring of pyrrolidine, pyrrolidinone, piperidine or piperidinone type;
- Y represents two hydrogen atoms or a hetero atom, for instance oxygen or sulfur;
- Ar represents a 1,4-phenyl, 2,5-pyridyl, 5,2-pyridyl or 2,5-thiophenyl ring;
- X represents an oxygen atom optionally substituted with an alkyl or alkylamine chain or a C-C single bond;
- A represents a hydrogen atom or the following formula: 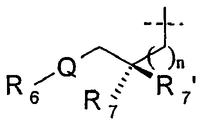
in which,
○ Q is an oxygen atom or an -NH- bond;
○ R₆ represents a hydrogen atom, an alkyl radical of 1 to 6 carbon atoms, a cycloalkyl radical of 3 to 6 carbon atoms or a -C(O)CH₃ or -C(O)CH₂CH₃ radical;
○ R₇ and R₇' represent, independently of each other, a hydrogen atom or a hydroxyl group, on condition that R₇ and R₇' are not simultaneously a hydroxyl group;
○ n is 0, 1, 2, 3, 4 or 5;
and the salts of the compounds of formula (I) when R₃ represents a hydrogen atom, and also the geometrical isomers of the said compounds of formula (I).

When the compounds according to the invention are in the form of a salt, it is preferably an alkali metal or alkaline-earth metal salt, or alternatively a zinc salt or a salt of an organic amine or of an acidic partner when the compound is itself basic.

According to the present invention, the alkyl radicals of 1 to 3 carbon atoms are preferably chosen from methyl, ethyl, i-propyl and n-propyl radicals.

According to the present invention, the alkyl radicals of 1 to 4 carbon atoms are preferably chosen from methyl, ethyl, i-propyl, i-butyl and t-butyl radicals.

According to the present invention, the alkyl radicals of 1 to 6 carbon atoms are preferably chosen from methyl, ethyl, propyl, i-propyl, butyl, i-butyl, t-butyl, pentyl and hexyl.

According to the present invention, the alkyl radicals of 1 to 10 carbon atoms are linear or branched chains preferably chosen from methyl, ethyl, propyl, i-propyl, butyl, i-butyl, t-butyl, pentyl, hexyl, heptyl, octyl, nonyl and dodecyl.

Finally, the term "alkoxy radical containing from 1 to 4 carbon atoms" means an alkyl radical containing from 1 to 4 carbon atoms linked to the rest of the molecule via an oxygen atom. Preferably, the alkoxy radical is chosen from methoxy, ethoxy, isopropyloxy and tert-butoxy radicals.

According to the present invention, the cycloalkyl radicals of 3 to 6 carbon atoms are preferably chosen from cyclopropyl, cyclopentyl and cyclohexyl.

According to the present invention, the compounds of formula (I) that are more particularly preferred are those for which at least one, and preferably all, of the conditions below are satisfied:
- R1 is a hydrogen atom or a t-butyl or i-propyl radical;
- R2 is a hydrogen atom or a t-butyl or i-propyl radical;
- R3 is a hydrogen atom or an ethyl radical;
- R4 and R5 are, independently of each other, a methyl or ethyl radical or together form a pyrrolidine ring;
- A is as defined above in which R₆ represents a hydrogen atom, an i-propyl or t-butyl radical, a cycloalkyl radical of 3 to 6 carbon atoms or a -C(O)CH₃ or -C(O)CH₂CH₃ radical.

Among the compounds of formula (I) falling within the context of the present invention, mention may be made especially of the following compounds:
1. ethyl 3"-*tert*-butyl-4"-diethylamino-4'-hydroxy[1,1';3',1"]terphenyl-4-carboxylate
2. ethyl 3"-*tert*-butyl-4"-diethylamino-4'-(4-hydroxybutoxy)-[1,1';3',1"]terphenyl-4-carboxylate
3. 3"-*tert*-butyl-4"-diethylamino-4'-(4-hydroxybutoxy)-[1,1';3',1"]terphenyl-4-carboxylic acid
4. ethyl 4"-(acetylethylamino)-3"-*tert*-butyl-4'-(4-hydroxybutoxy)-[1,1';3',1"]terphenyl-4-carboxylate
5. 4"-(acetylethylamino)-3"-*tert*-butyl-4'-(4-hydroxybutoxy)-[1,1';3',1"]terphenyl-4-carboxylic acid
6. 3"-*tert* butyl-4"-diethylamino-4'-(2-hydroxyethoxy)-[1,1';3',1"]terphenyl-4-carboxylic acid
7. ethyl 3"-*tert*-butyl-4"-diethylamino-4'-(3-hydroxypropoxy)-[1,1';3',1"]terphenyl-4-carboxylate
8. 3"-*tert*-butyl-4"-diethylamino-4'-(3-hydroxypropoxy)-[1,1';3',1"]terphenyl-4-carboxylic acid
9. ethyl 4"-diethylamino-4'-(3-hydroxypropoxy)-[1,1';3',1"]terphenyl-4-carboxylate
10. 4"-diethylamino-4'-(3-hydroxypropoxy)-[1,1';3',1"]terphenyl-4-carboxylic acid
11. ethyl 4"-diethylamino-3"-ethyl-4'-(3-hydroxypropoxy)-[1,1';3',1"]terphenyl-4-carboxylate
12. 4"-diethylamino-3"-ethyl-4'-(3-hydroxypropoxy)-[1,1';3',1"]terphenyl-4-carboxylic acid
13. 4"-diethylamino-3"-ethyl-4'-(2-hydroxyethoxy)-[1,1';3',1"]terphenyl-4-carboxylic acid
14. ethyl 4"-diethylamino-3"-ethyl-4'-(2-hydroxyethoxy)-[1,1';3',1"]terphenyl-4-carboxylate
15. 3"-*tert*-butyl-4"-diethylamino-4'-(2-hydroxyethoxy)-[1,1';3',1"]terphenyl-4-carboxylate
16. ethyl 4"-diethylamino-4'-(3-hydroxypropoxy)-3"-methyl[1,1';3',1"]terphenyl-4-carboxylate
17. 4"-diethylamino-4'-(3-hydroxypropoxy)-3"-methyl[1,1';3',1"]terphenyl-4-carboxylic acid
18. ethyl 3"-*tert*-butyl-4'-(4-hydroxybutoxy)-4"-pyrrolidin-1-yl[1,1';3',1"]terphenyl-4-carboxylate
19. 3"-*tert*-butyl-4'-(4-hydroxybutoxy)-4"-pyrrolidin-1-yl[1,1';3',1"]terphenyl-4-carboxylic acid
20. ethyl 4'-(4-hydroxybutoxy)-4"-pyrrolidin-1-yl-3"-trifluoromethyl[1,1';3',1"]terphenyl-4-carboxylate
21. 4'-(4-hydroxybutoxy)-4"-pyrrolidin-1-yl-3"-trifluoromethyl[1,1';3',1"]terphenyl-4-carboxylic acid
22. ethyl 3"-*tert*-butyl-4'-(3-hydroxypropoxy)-4"-pyrrolidin-1-yl[1,1';3',1"]terphenyl-4-carboxylate
23. 3"-*tert*-butyl-4'-(3-hydroxypropoxy)-4"-pyrrolidin-1-yl[1,1';3',1"]terphenyl-4-carboxylic acid
24. ethyl 3"-*tert*-butyl-4'-(2-hydroxyethoxy)-4"-pyrrolidin-1-yl[1,1';3',1"]terphenyl-4-carboxylate
25. 3"-*tert*-butyl-4'-(2-hydroxyethoxy)-4"-pyrrolidin-1-yl[1,1';3',1"]terphenyl-4-carboxylic acid
26. 4'-(3-hydroxypropoxy)-4"-pyrrolidin-1-yl[1,1';3',1"]terphenyl-4-carboxylic acid
27. 3"-*tert*-butyl-4"-diethylamino-4'-hydroxy[1,1';3',1"]terphenyl-4-carboxylic acid
28. ethyl 4"-diethylamino-4'-hydroxy-3"-trifluoromethyl[1,1';3',1"]terphenyl-4-carboxylate
29. 4"-diethylamino-4'-hydroxy-3"-trifluoromethyl[1,1';3',1"]terphenyl-4-carboxylic acid
30. 3"-*tert*-butyl-4"-diethylamino-4'-(4-isopropylaminobutoxy)-[1,1';3',1"]terphenyl-4-carboxylic acid
31. 3"-*tert*-butyl-4'-(4-isopropylaminobutoxy)-4"-pyrrolidin-1-yl[1,1';3',1"]terphenyl-4-carboxylic acid
32. ethyl 3"-*tert*-butyl-4"-diethylamino-4'-(3-hydroxypropyl)-[1,1';3',1"]terphenyl-4-carboxylate
33. 3"-*tert*-butyl-4"-diethylamino-4'-(3-hydroxypropyl)-[1,1';3',1"]terphenyl-4-carboxylic acid
34. ethyl 3"-*tert*-butyl-4"-diethylamino-4'-(2,3-dihydroxypropyl)-[1,1';3',1"]terphenyl-4-carboxylate
35. 3"-*tert*-butyl-4"-diethylamino-4'-(2,3-dihydroxypropyl)-[1,1';3',1"]terphenyl-4-carboxylic acid
36. ethyl 4"-(acetylethylamino)-3"-*tert*-butyl-4'-(3-hydroxypropyl)-[1,1';3',1"]terphenyl-4-carboxylate
37. 4"-(acetylethylamino)-3"-*tert*-butyl-4'-(3-hydroxypropyl)-[1,1';3',1"]terphenyl-4-carboxylic acid
38. ethyl 3"-*tert*-butyl-4'-(3-cyclopropylaminopropyl)-4"-diethylamino[1,1';3',1"]terphenyl-4-carboxylate
39. 3"-*tert*-butyl-4'-(3-cyclopropylaminopropyl)-4"-diethylamino[1,1';3',1"]terphenyl-4-carboxylic acid
40. ethyl 3"-*tert*-butyl-4'-(3-cyclopentylaminopropyl)-4"-diethylamino[1,1';3',1"]terphenyl-4-carboxylate
41. 3"-*tert*-butyl-4'-(3-cyclopentylaminopropyl)-4"-diethylamino[1,1';3',1"]terphenyl-4-carboxylic acid
42. ethyl 3"-*tert*-butyl-4'-(3-cyclohexylaminopropyl)-4"-diethylamino[1,1';3',1"]terphenyl-4-carboxylate
43. 3"-*tert*-butyl-4'-(3-cyclohexylaminopropyl)-4"-diethylamino[1,1';3',1"]terphenyl-4-carboxylic acid
44. ethyl 3"-*tert*-butyl-4'-(3-tert-butylaminopropyl)-4"-diethylamino[1,1';3',1"]terphenyl-4-carboxylate
45. 3"-*tert*-butyl-4'-(3-*tert*-butylaminopropyl)-4"-diethylamino[1,1';3',1"]terphenyl-4-carboxylic acid
46. ethyl 4"-(acetylethylamino)-3"-*tert*-butyl-4'-(3-cyclopropylaminopropyl)-[1,1';3',1"]terphenyl-4-carboxylate
47. 4"-(acetylethylamino)-3"-*tert*-butyl-4'-(3-cyclopropylaminopropyl)-[1,1';3',1"]terphenyl-4-carboxylic acid
48. ethyl 3"-*tert*-butyl-4"-diethylamino-4'-(3-isopropylaminopropyl)-[1,1';3',1"]terphenyl-4-carboxylate
49. 3"-*tert*-butyl-4"-diethylamino-4'-(3-isopropylaminopropyl)-[1,1';3',1"]terphenyl-4-carboxylic acid
50. ethyl 4'-(3-aminopropyl)-3"-*tert* butyl-4"-diethylamino[1,1';3',1"]terphenyl-4-carboxylate
51. 4'-(3-aminopropyl)-3"-*tert*-butyl-4"-diethylamino[1,1';3',1"]terphenyl-4-carboxylic acid
52. [3"-*tert*-butyl-4-carboxy-4'-(3-hydroxypropyl)-[1,1';3',1"]terphenyl-4"-yl]diethylamine hydrochloride
53. 3"-tert-butyl-4'-(2-hydroxyethoxy)-4"-(2-oxopyrrolidin-1-yl)-[1,1';3',1"]terphenyl-4-carboxylic acid
54. 3"-tert-butyl-4"-ethylamino-4'-(3-hydroxypropoxy)-[1,1';3',1"]terphenyl-4-carboxylic acid
55. 4'-(3-acetoxypropoxy)-3"-tert-butyl-4"-diethylamino[1,1';3',1"]terphenyl-4-carboxylic acid
56. 3"-*tert*-butyl-4"-diethylamino-4'-(3-propionyloxypropoxy)-[1,1';3',1"]terphenyl-4-carboxylic acid
57. methyl 3"-*tert*-butyl-4"-diethylamino-4'-(3-hydroxypropoxy)-[1,1';3',1"]terphenyl-4-carboxylate
58. isopropyl 3"-*tert*-butyl-4"-diethylamino-4'-(3-hydroxypropoxy)-[1,1';3',1"]terphenyl-4-carboxylate
59. isobutyl 3"-*tert*-butyl-4"-diethylamino-4'-(3-hydroxypropoxy)-[1,1';3',1"]terphenyl-4-carboxylate
60. 3"-*tert-*butyl-4"-diethylamino-4'-(3-hydroxypropoxy)-5"-methyl[1,1;3',1"]terphenyl-4-carboxylic acid
61. 4"-diethylamino-4'-(3-hydroxypropoxy)-3"-isopropyl-5"-methyl[1,1';3',1"]terphenyl-4-carboxylic acid
62. 3"-*tert*-butyl-5"-chloro-4"-diethylamino-4'-(3-hydroxypropoxy)-[1,1';3',1"]terphenyl-4-carboxylic acid
63. 4"-diethylamino-4'-(3-hydroxypropoxy)-3",5"-diisopropyl[1,1'; 3',1"]terphenyl-4-carboxylic acid
64. 3",5"-di-*tert*-butyl-4"-diethylamino-4'-(3-hydroxypropoxy)-[1,1';3',1"]terphenyl-4-carboxylic acid
65. 4"-diethylamino-4'-(3-hydroxypropoxy)-3"-trifluoromethyl[1,1';3',1"]terphenyl-4-carboxylic acid
66. 3"-*tert*-butyl-4"-(ethylmethylamino)-4'-(2-hydroxyethoxy)-[1,1';3',1"]terphenyl-4-carboxylic acid
67. 3"-*tert*-butyl-4"-dimethylamino-4'-(2-hydroxyethoxy)-[1,1';3',1"]terphenyl-4-carboxylic acid
68. 3"-*tert*-butyl-4"-(ethylisopropylamino)-4'-(2-hydroxyethoxy)-[1,1';3',1"]terphenyl-4-carboxylic acid
69. 3"-*tert*-butyl-4"-(ethylpropylamino)-4'-(2-hydroxyethoxy)-[1,1';3',1"]terphenyl-4-carboxylic acid
70. 3"-*tert*-butyl-4"-dipropylamino-4'-(2-hydroxyethoxy)-[1,1';3',1"]terphenyl-4-carboxylic acid
71. 3"-*tert*-butyl-4"-(ethylpropionylamino)-4'-(2-hydroxyethoxy)-[1,1';3',1"]terphenyl-4-carboxylic acid
72. 6-[3'-*tert*-butyl-4'-diethylamino-6-(2-hydroxyethoxy)biphenyl-3-yl]nicotinic acid
73. 5-[3'-*ter*t-butyl-4'-diethylamino-6-(2-hydroxyethoxy)biphenyl-3-yl]pyridine-2-carboxylic acid
74. 5-[3'-*tert* butyl-4'-diethylamino-6-(2-hydroxyethoxy)biphenyl-3-yl]thiophene-2-carboxylic acid
75. 3"-*tert*-butyl-4'-(2-hydroxyethoxy)-5"-methyl-4"-pyrrolidin-1-yl[1,1';3',1"]terphenyl-4-carboxylic acid
76. 3"-*tert* butyl-5"-chloro-4'-(2-hydroxyethoxy)-4"-pyrrolidin-1-yl[1,1';3',1"]terphenyl-4-carboxylic acid
77. 4'-(2-hydroxyethoxy)-3"-isopropyl-4"-pyrrolidin-1-yl[1,1';3',1"]terphenyl-4-carboxylic acid
78. 3"-ethyl-4'-(2-hydroxyethoxy)-4"-pyrrolidin-1-yl[1,1';3',1"]terphenyl-4-carboxylic acid
79. 4'-(2-hydroxyethoxy)-3",5"-diisopropyl-4"-pyrrolidin-1-yl[1,1';3',1"]terphenyl-4-carboxylic acid
80. 3",5"-diethyl-4'-(2-hydroxyethoxy)-4"-pyrrolidin-1-yl[1,1';3',1"]terphenyl-4-carboxylic acid
81. 3",5"-dimethyl-4'-(2-hydroxyethoxy)-4"-pyrrolidin-1-yl[1,1';3',1"]terphenyl-4-carboxylic acid
82. 4'-(2-acetoxyethoxy)-3"-*tert*-butyl-4"-pyrrolidin-1-yl[1,1';3',1"]terphenyl-4-carboxylic acid
83. 4'-(2-propionyloxyethoxy)-3"-*tert*-butyl-4"-pyrrolidin-1-yl[1,1';3',1"]terphenyl-4-carboxylic acid
84. methyl 3"-*tert*-butyl-4'-(2-hydroxyethoxy)-4"-pyrrolidin-1-yl[1,1';3',1"]terphenyl-4-carboxylate
85. isopropyl 3"-*tert*-butyl-4'-(2-hydroxyethoxy)-4"-pyrrolidin-1-yl[1,1';3',1"]terphenyl-4-carboxylate
86. isobutyl 3"-*tert*-butyl-4'-(2-hydroxyethoxy)-4"-pyrrolidin-1-yl[1,1';3',1"]terphenyl-4-carboxylate
87. ethyl 3"-*tert*-butyl-4'-(3-hydroxypropoxy)-4"-pyrrolidin-1-yl[1,1';3',1"]terphenyl-4-carboxylate
88. ethyl 3"-*tert*-butyl-5"-chloro-4'-(2-hydroxyethoxy)-4"-pyrrolidin-1-yl[1,1';3',1"]terphenyl-4-carboxylate
89. 6-[3'-tert-butyl-6-(2-hydroxyethoxy)-4'-pyrrolidin-1-ylbiphenyl-3-yl]nicotinic acid
90. 5-[3'-tert-butyl-6-(2-hydroxyethoxy)-4'-pyrrolidin-1-ylbiphenyl-3-yl]pyridine-2-carboxylic acid
91. ethyl 6-[3'-*tert*-butyl-6-(2-hydroxyethoxy)-4'-pyrrolidin-1-ylbiphenyl-3-yl]nicotinate
92. ethyl 3"-*tert*-butyl-4'-(3-hydroxypropyl)-4"-pyrrolidin-1-yl[1,1';3',1"]terphenyl-4-carboxylate
93. 3"-*tert*-butyl-4'-(3-hydroxypropyl)-4"-pyrrolidin-1-yl[1,1';3',1"]terphenyl-4-carboxylic acid
94. 3"-*tert*-butyl-4'-(2-hydroxyethoxy)-4"-(2-oxopyrrolidin-1-yl)-[1,1';3',1"]terphenyl-4-carboxylic acid
95. 3"-*tert*-butyl-4'-(2-hydroxyethoxy)-4"-(2-oxopiperid-1-yl)-[1,1';3',1"]terphenyl-4-carboxylic acid
96. 3"-*tert*-butyl-4'-(2-hydroxyethoxy)-4"-piperid-1-yl[1,1';3',1"]terphenyl-4-carboxylic acid
97. 5-[3'-*tert*-butyl-6-(2-hydroxyethoxy)-4'-pyrrolidin-1-ylbiphenyl-3-yl]thiophene-2-carboxylic acid.

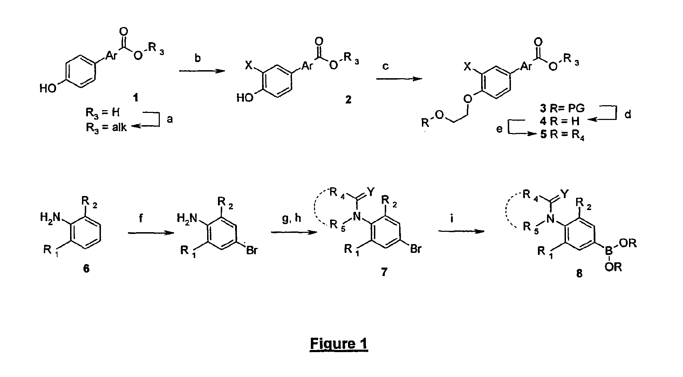

A subject of the present invention is also the processes for preparing the compounds of formula (I), in particular according to the reaction schemes given in Figures 1, 2 and 3.

### - Synthesis of advanced fragments (Figure 1)

The intermediates of general formula **1** or **2** are prepared from the commercial starting material **1**. Compound **1** is subjected to a first step of esterification of the carboxylic acid function (a), performed under standard esterification conditions including, for example, the methods described in "Protective Groups in Organic Synthesis" by T.W. Greene & P. G. M. Wuts, 3rd edition (J. Wiley & sons), pages 373-377 or in "Comprehensive Organic Transformations" by R.C. Larock, 2nd edition (J. Wiley & sons), pages 1932-1941. The introduction of a halide (bromide or iodide) into an ortho position of the phenol function (b) may be performed under standard bromination conditions (for example by adding dibromine or an equivalent reagent such as a tetraalkylammonium tribromide) or standard iodination conditions (for example sodium iodide and sodium hypochlorite), many examples of which may be found in the literature: see, for example "Comprehensive Organic Transformations" by R.C. Larock, 2nd edition (J. Wiley & sons), pages 619-628.

The compounds of general formula **3** are then obtained via standard methods of etherification of phenols (c), for instance an etherification similar to a Williamson reaction starting with corresponding alkyl halides in the presence of a base, or alternatively a reaction of Mitsunobu type with the corresponding hydroxyl derivatives (see "Comprehensive Organic Transformations" by R.C. Larock, 2nd edition (J. Wiley & sons), pages 889-910 or, respectively: a. Dermer, O.C., Chem. Rev. 1934, 14, 409 or Nakatsugi, T. Synthesis, 1987, 280: b. Mitsunobu, O. Synthesis 1981, 1). The compounds of formula **3** are, in the case where the protecting group PG does not coincide with the desired group R₆, subjected to a deprotection step (d) suited to the nature of PG, the description of which will be found in "Protective Groups in Organic Synthesis" by T.W. Greene & P. G. M. Wuts, 3rd edition (J. Wiley & sons) to obtain compound **4** (R₆ = H) and is then subjected, where appropriate, to a standard esterification step (e) (see above) with the carboxylic acid or acyl halide derivative corresponding to the structure of R₄, in order to obtain the compounds of type **5** in which R₆ is other than H.

The intermediates of general formula **7** may be obtained from the compounds **6** after a first step of bromination (f) in the para position (see above) followed by alkylation or amidation of the aniline function (g) in the presence, respectively, of a dialkyl sulfate or of an alkyl halide and a base (see, for example, Dehmlow, E.V., Tet. Lett. 1985, 25, 97 or the reference below) or in the presence of an acyl chloride or a corresponding anhydride and a base (for example Et₃N) in accordance with the methods described, for example, in "Chemistry of the Amino Group" by S. Patai (Wiley-Interscience, NY 1968) pages 669-682. Alternatively, when R₄ and R₅, taken together, form a ring from among the claimed sub-structures, for example a pyrrolidine ring, the compounds **7** may be obtained after bromination of **6** and then formation of the ring (g), for example in the presence of a 1,4-dihalobutane or a 1,5-dihalopentane or the carbonyl analogues thereof, and of a base, or via a method described in "Chemistry of the Amino Group" by S. Patai (Wiley-Interscience, NY 1968) pages 669-682. Alternatively, when R₄ and R₅, taken together, form a ring from among the claimed sub-structures, the compounds of general formula **7** may also be generated after para- bromination (**f**, see above) and then formation (h) and reduction (i) of a pyrrolidinone, piperidinone, succinimide or piperidine-2,6-dione group (see, for example, Ohta, S. Heterocycles 1993, 36 (4), 743; Hubbard, J. L.; J. Heterocycl. Chem. 1992, 29 (4), 719; Akula, M. R.; Synth. Commun. 1998, 28 (11), 2063; Collins, C. J. Tetrahedron Lett. 1999, 40 (19), 3673).

Finally, the compounds of general formula **8** may be obtained via a sequence of two reactions: the first is an alkylation reaction (h) of a secondary aniline (when Y = H, H, see above) or of an acylaniline (when Y = O), in the presence of a dialkyl sulfate or an alkyl halide and of a base (see, for example, Bisarya, S.C. Synth. Commun. 1992, 22 (22), 3305 or the above references); an inversion of steps (h) and (g) when Y = O allows the same precursor of the compounds of general formula **8** to be obtained. The second reaction is the generation of a boronic acid or boronate function from the bromide group (i), for example by generating an organolithium or organomagnesium reagent trapped with a trialkyl borate (see, for example, Cladingboel, D. E. Org. Process Res. Dev. 2000, 4 (3), 153 or Li, W. J. Org. Chem. 2002, 67 (15), 5394) or alternatively by performing a coupling reaction with bis-dialkoxydiborane or dialkoxyborane in the presence of a catalyst of transition metal type (see, for example, Ishiyama, T. J. Org. Chem. 1995, 60 (23), 7508 or Murata, M., J. Org. Chem. 1997, 62 (19), 6458).

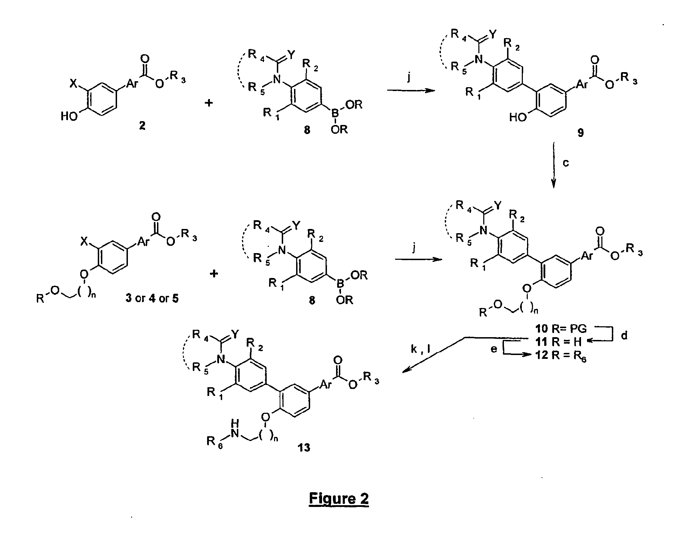

### - Synthesis of the compounds in which X = O (Figure 2)

The synthesis of the final compounds of general formulae **11** and **12** may be performed according to two parallel routes for which only the order of the reactions changes.

A first route requires the synthesis of the intermediate **9**, via a coupling reaction of Suzuki type (j) between the intermediate **2** and the boronate/boronic acid partner of formula **8**, under standard Suzuki coupling conditions (see A. Suzuki et al., Synth. Commun. 1981, 11, 513 or Sharp, M.J. Tet. Lett. 1985, 26, 5997) or alternatively, where appropriate, optimized conditions (see, for example, Littke, A.F. et al., J. Am. Chem. Soc. 2000, 122 (17), 4020-4028). The compounds **9** are obtained directly when R₃ is other than H, or after a reaction to reveal the carboxylic acid function, for example by using conditions among those described in "Comprehensive Organic Transformations" by R.C. Larock, 2nd edition (J. Wiley & sons), pages 1959-1968.

The intermediate **9** may also be subjected to the conditions (c) described above in order to obtain the compounds of general formula **10**.

These compounds of general formula **10** may also be generated via the same methods (j) described above starting with the intermediates of general formula **3**.

When PG is other than the desired group R₆, **10** may be subjected to deprotection conditions (d) mentioned above to obtain the final compounds **11** in which R₆ = H, and then, where appropriate, subjected to the conditions (e) to obtain the compounds **12** in which R₆ is other than H.

Alternatively, these same final compounds **11** and **12** may be obtained by subjecting, respectively, the intermediates **4** and **5** to the coupling conditions (j) described above.

Finally, when R₃ = H, the advanced intermediates **11** and **12** may be subjected to reactions to reveal the carboxylic acid function, for example using conditions among those described in "Comprehensive Organic Transformations" by R.C. Larock, 2nd edition (J. Wiley & sons), pages 1959-1968.

The compounds of general formula **13** may be obtained after a sequence of conversion of the primary alcohol function of **11** into an amine, for example via oxidation (k) followed by reductive amination (I) (see, for example, "Comprehensive Organic Transformations" by R.C. Larock, 2nd edition (J. Wiley & sons)) or alternatively conversion of the alcohol into a halide and substitution of the halogen atom with an amine.

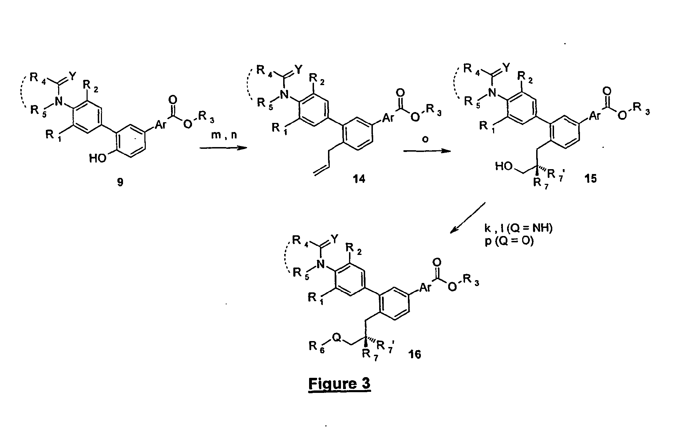

### - Synthesis of the claimed compounds in which X is a single bond (Figure 3)

When X is a single bond, the intermediates of general formula **9** are first converted into suitable sulfonyl esters under standard conditions, for example into triflates (see, for example, Robl, J. A. Tetrahedron Lett. 1990, 31 (24), 3421) (m), and this group is then subjected to an allylation reaction (n), for example in the presence of tributylallyltin and of a transition metal catalyst, for example tetrakis(triphenylphosphine)palladium (for an example, see Martorell, G.; Garcia-Raso, A.; Saa, J. M.; Tetrahedron Lett. 1990, 31 (16), 2357), to obtain the intermediates of type **14**.

The final compounds of general formula **15** may then be obtained via an oxidation reaction (o) of the olefin function, for instance an oxidative hydroboration reaction (see, for example, Luo, F. T.; Negishi, E.; J. Org. Chem. 1983, 48, 5144 or "Comprehensive Organic Transformations" by R.C. Larock, 2nd edition (J. Wiley & sons), pages 992-993 & 1005-1007) in the case where R₇, R₇' = H, H or alternatively via a racemic or enantioselective dihydroxylation reaction, as described, for example, in Van Rheenan, V.; Cha, D. Y.; Hartley, W. M.; Org. Synth. 1978, 58, 44 or in "Comprehensive Organic Transformations" by R.C. Larock, 2nd edition (J. Wiley & sons), pages 996-1001.

When Q = NH, the reaction sequence (k, l) described above for the conversion of the compounds **11** into compounds **13** may be applied, in order to obtain the compounds of general formula **16**. Alternatively, when Q = O and R₆ is other than H, a simple standard alkylation or acylation reaction of the primary alcohol function of the intermediates of structure **15** allows the final compounds of general formula **16** to be obtained.

The compounds according to the invention have modulatory properties on retinoic acid receptors (RAR). This activity on the RARα, β and γ receptors is measured in a transactivation test and quantified by means of the dissociation constant Kdapp (apparent), as described in Example 55.

The preferred compounds of the present invention have a dissociation constant of less than or equal to 5000 nM, advantageously less than or equal to 1000 nM and preferentially less than or equal to 1 nM.

Preferably, the compounds are at least modulators of receptors of RARγ type, selectively relative to the subtypes α and β, i.e. they have a ratio between the Kdapp for the RARα or RARβ receptors, and the Kdapp for the RARγ receptors, of greater than or equal to 5. Preferably, this ratio RARγ/RARβ or RARγ/RARα is greater than or equal to 10, advantageously greater than or equal to 50 and more advantageously greater than or equal to 100.

A subject of the present invention is also the compounds of formula (I) as described above, as medicaments.

The compounds according to the invention are particularly suitable in the following fields of treatment:
1) for treating dermatological complaints associated with a keratinization disorder relating to cell differentiation and proliferation, especially for treating common acne, comedones, polymorphonuclear leukocytes, acne rosacea, nodulocystic acne, acne conglobata, senile acne, and secondary acnes such as solar acne, medication-related acne or occupational acne;
2) for treating other types of keratinization disorders, especially ichthyosis, ichthyosiform conditions, Darier's disease, palmoplantar keratoderma, leukoplakia and leukoplakiform conditions, and cutaneous or mucous (buccal) lichen;
3) for treating other dermatological complaints with an inflammatory immunoallergic component, with or without cell proliferation disorder, and especially all forms of psoriasis, whether cutaneous, mucous or ungual psoriasis, and even psoriatic rheumatism, or cutaneous atopy, such as eczema, or respiratory atopy, or even gingival hypertrophy;
4) in the treatment of skin disorders caused by exposure to UV radiation, and also for repairing or combating ageing of the skin, whether photoinduced or chronological ageing, or for reducing actinic pigmentations and keratosis, or any pathology associated with chronological or actinic ageing, such as xerosis;
5) for treating all dermal or epidermal proliferations, whether benign or malignant, and whether of viral origin or otherwise, such as common warts, flat warts and verruciform epidermodysplasia, oral or florid papillomatoses, T lymphoma, and proliferations that may be induced by ultraviolet radiation, especially in the case of basocellular and spinocellular epithelioma, and also any precancerous skin lesion such as keratoacanthomas;
6) for treating other dermatological disorders such as immune dermatoses, such as lupus erythematosus, immune bullous diseases and collagen diseases, such as scleroderma;
7) in the treatment of dermatological or general complaints with an immunological component;
8) for treating certain ophthalmological disorders, especially corneopathies;
9) for preventing or curing the stigmata of epidermal and/or dermal atrophy induced by local or systemic corticosteroids, or any other form of cutaneous atrophy;
10) in the treatment of any cutaneous or general complaint of viral origin;
11) for combating sebaceous function disorders, such as the hyperseborrhoea of acne or simple seborrhoea;
12) for preventing or treating cicatrization disorders, or for preventing or repairing stretch marks, or alternatively for promoting cicatrization;
13) in the treatment of pigmentation disorders, such as hyperpigmentation, melasma, hypopigmentation or vitiligo;
14) in the treatment of lipid metabolism complaints, such as obesity, hyperlipidaemia, or non-insulin-dependent diabetes;
15) in the treatment of inflammatory complaints such as arthritis;
16) in the treatment or prevention of cancerous or precancerous conditions;
17) in the prevention or treatment of alopecia of various origins, especially alopecia caused by chemotherapy or radiation;
18) in the treatment of disorders of the immune system, such as asthma, type I sugar diabetes, multiple sclerosis or other selective dysfunctions of the immune system; and
19) in the treatment of complaints of the cardiovascular system, such as arteriosclerosis or hypertension.

A subject of the present invention is also a pharmaceutical composition comprising, in a physiologically acceptable medium, at least one compound of formula (I) as defined above.

A subject of the present invention is also a novel medicinal composition intended especially for treating the abovementioned complaints, which is characterized in that it comprises, in a pharmaceutically acceptable support that is compatible with the mode of administration selected for this composition, at least one compound of formula (I), an optical isomer thereof or a salt thereof.

The composition according to the invention may be administered orally, enterally, parenterally, topically or ocularly. The pharmaceutical composition is preferably packaged in a form that is suitable for topical application.

Via the oral route, the composition may be in the form of tablets, gel capsules, dragees, syrups, suspensions, solutions, powders, granules, emulsions, suspensions of microspheres or nanospheres or lipid or polymer vesicles allowing a controlled release. Via the parenteral route, the composition may be in the form of solutions or suspensions for infusion or for injection.

The compounds according to the invention are generally administered at a daily dose of about 0.01 mg/kg to 100 mg/kg of body weight, in one or more dosage intakes.

The compounds are used systemically, at a concentration generally of between 0.001% and 10% by weight and preferably between 0.01% and 1% by weight, relative to the weight of the composition.

Via the topical route, the pharmaceutical composition according to the invention is more particularly intended for treating the skin and mucous membranes and may be in liquid, pasty or solid form, and more particularly in the form of ointments, creams, milks, pomades, powders, impregnated pads, syndets, solutions, gels, sprays, mousses, suspensions, sticks, shampoos or washing bases. It may also be in the form of suspensions of microspheres or nanospheres or of lipid or polymer vesicles or gelled or polymer patches allowing a controlled release.

The compounds are used topically at a concentration generally of between 0.001% and 10% by weight and preferably between 0.01% and 1% by weight, relative to the total weight of the composition.

The compounds of formula (I) according to the invention also find an application in cosmetics, in particular in body and hair hygiene and especially for treating acne-prone skin, for promoting regrowth of the hair or for limiting hair loss, for combating the greasy appearance of the skin or the hair, in protection against the harmful aspects of sunlight or in the treatment of physiologically dry skin, and for preventing and/or combating photoinduced or chronological ageing.

A subject of the invention is thus also a cosmetic composition comprising, in a physiologically acceptable support, at least one of the compounds of formula (I).

A subject of the invention is also the non-therapeutic use of a cosmetic composition comprising at least one compound of formula (I) for preventing and/or treating the signs of ageing and/or dry skin.

A subject of the invention is also the non-therapeutic use of a cosmetic composition comprising at least one compound of formula (I) for body or hair hygiene.

The cosmetic composition according to the invention containing, in a physiologically acceptable medium, at least one compound of formula (I) or an optical or geometrical isomer thereof or a salt thereof, may be especially in the form of a cream, a milk, a gel, suspensions of microspheres or nanospheres or lipid or polymer vesicles, impregnated pads, solutions, sprays, mousses, sticks, soaps, washing bases or shampoos.

The concentration of compound of formula (I) in the cosmetic composition is preferably between 0.001 % and 3% by weight, relative to the total weight of the composition.

The term "physiologically acceptable medium" means a medium that is compatible with the skin and optionally with its integuments (eyelashes, nails or hair) and/or mucous membranes.

The pharmaceutical and cosmetic compositions as described above may also contain inert additives, or even pharmacodynamically active additives as regards the pharmaceutical compositions, or combinations of these additives, and especially:
- wetting agents;
- flavour enhancers;
- preserving agents such as para-hydroxybenzoic acid esters;
- stabilizers;
- moisture regulators;
- pH regulators;
- osmotic pressure modifiers;
- emulsifiers;
- UV-A and UV-B screening agents;
- antioxidants such as α-tocopherol, butylhydroxyanisole, butylhydroxytoluene, superoxide dismutase, ubiquinol or certain metal-chelating agents;
- depigmenting agents such as hydroquinone, azelaic acid, caffeic acid or kojic acid;
- emollients;
- moisturizers, for instance glycerol, PEG 400, thiamorpholinone and its derivatives or urea;
   antiseborrhoeic or anti-acne agents, such as S-carboxymethylcysteine, S-benzylcysteamine, salts thereof or derivatives thereof, or benzoyl peroxide;
- antibiotics, for instance erythromycin and its esters, neomycin, clindamycin and its esters, and tetracyclines;
- antifungal agents such as ketoconazole or poly-4,5-methylene-3-isothiazolidones;
- agents for promoting regrowth of the hair, for instance Minoxidil (2,4-diamino-6-piperidinopyrimidine 3-oxide) and its derivatives, Diazoxide (7-chioro-3-methyl-1,2,4-benzothiadiazine 1,1-dioxide) and Phenytoin (5,4-diphenylimidazolidine-2,4-dione);
- non-steroidal anti-inflammatory agents;
- carotenoids and especially β-carotene;
- anti-psoriatic agents such as anthralin and its derivatives;
- eicosa-5,8,11,14-tetraynoic acid and eicosa-5,8,11-triynoic acid, and esters and amides thereof;
- retinoids, i.e. natural or synthetic RXR receptor ligands;
- corticosteroids or oestrogens;
- α-hydroxy acids and α-keto acids or derivatives thereof, such as lactic acid, malic acid, citric acid, glycolic acid, mandelic acid, tartaric acid, glyceric acid or ascorbic acid, and also salts, amides or esters thereof, or β-hydroxyl acids or derivatives thereof, such as salicylic acid and its salts, amides or esters;
- ion-channel blockers such as potassium-channel blockers;
- or alternatively, more particularly for pharmaceutical compositions, in combination with medicaments known to interfere with the immune system (for example cyclosporin, FK 506, glucocorticoids, monoclonal antibodies, cytokines or growth factors, etc.).

Needless to say, a person skilled in the art will take care to select the optional compound(s) to be added to these compositions such that the advantageous properties intrinsically attached to the present invention are not, or are not substantially, adversely affected by the envisaged addition.

Another subject of the invention relates to a cosmetic process for enhancing the appearance of the skin, characterized in that a composition comprising at least one compound of formula (I) as defined above is applied to the skin.

Activation of the retinoic acid receptors with the compounds of formula (I) according to the invention makes it possible to obtain skin of enhanced surface appearance.

Several examples of the production of active compounds of formula (I) according to the invention, biological activity results and also various concrete formulations based on such compounds, will now be given, for illustrative purposes and with no limiting nature.

### EXAMPLES

### Example 1- Ethyl 3"-tert-butyl-4"-diethylamino-4'-hydroxy[1,1';3',1"]terphenyl-4-carboxylate

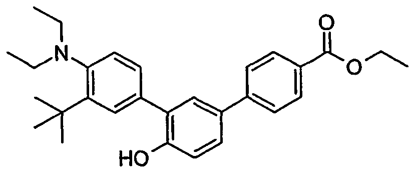

### a) 4-Bromo-2-tert-butylaniline

25 g of 2 tert-butylaniline (168 mmol) are dissolved in 250 mL of THF; the reaction mixture is stirred and cooled to 0°C, and 81g of tetrabutylammonium bromide (TBA.Br₃) (168 mmol) are then added portionwise while maintaining the temperature between 0°C and 5°C. The temperature is then allowed to rise to about room temperature and the mixture is stirred for 10 minutes.

The reaction is stopped by adding 250 mL of water and is then extracted with 250 mL of ethyl acetate. The organic phases are washed with 1 L of saturated Na₂S₂O₅ solution and then dried over magnesium sulfate. The solvents are evaporated off and the residue is filtered through a pad of silica (pure heptane, then a 3/7 heptane/ethyl acetate mixture). 43.6 g of 4-bromo-2-*tert*-butylaniline (yield = 100%) are obtained in the form of a white solid.

### b) (4-Bromo-2-tert-butylphenyl)diethylamine

6.9 g (0.17 mol) of sodium hydride are suspended in 200 mL of tetrahydrofuran. 13 g (57 mmol) of 4-bromo-2-tert-butylaniline are added, along with 200 mL of dimethyl sulfoxide, added slowly. The mixture turns blue and, after 30 minutes, 13 mL (0.17 mol) of ethyl iodide are added and the reaction medium, which has turned white, is stirred at room temperature for 13 hours. The reaction medium is then poured into saturated ammonium chloride solution and extracted with ethyl acetate, and the organic phase is then washed twice with water. It is dried and then concentrated to dryness. The residue is purified by chromatography on silica gel (eluent: 90/10 heptane/ethyl acetate). 14.8 g of (4-bromo-2-*tert*-butylphenyl)diethylamine are obtained (yield = 91%) in the form of a yellow oil.

### c) 3-tert-Butyl-4-diethylaminophenylboronic acid

9.8 g (35 mmol) of (4-bromo-2-*tert*-butylphenyl)diethylamine are dissolved in 118 mL of THF at room temperature, and the reaction mixture is then cooled to -78°C. 17.5 mL of a 2 M solution of *n*-BuLi (35 mmol) are added dropwise and the reaction medium is left stirring at -78°C for 1 hour. 12 mL of triisopropyl borate (B(OiPr)₃) (52 mmol) are added slowly and the reaction medium is stirred for 15 minutes at -70°C.

The temperature is raised to room temperature and the reaction medium is stirred for 3 hours. The reaction medium is cooled again to -70°C and 69 mL of 1M hydrochloric acid solution (69 mmol) are added. The temperature is raised to 0°C and the reaction medium is stirred for 30 minutes. The reaction is extracted after addition of 250 mL of water and 250 mL of ethyl acetate. The organic phases are washed with 800 mL of water and then dried over sodium sulfate. 6.5 g of 3-*tert*-butyl-4-diethylaminophenylboronic acid are obtained (yield = 76%) in the form of a thick oil.

### d) Ethyl 3"-tert-butyl-4'-diethylamino-4'-hydroxy[1,1;3;1']terphenyl-4-carboxylate

To 5.6 g of ethyl 3'-bromo-4'-hydroxy-biphenyl-4-carboxylate (17 mmol) are added 6.5 g of 3-tert-butyl-4-diethylaminophenylboronic acid (26 mmol) dissolved in 112 mL of toluene and 30.5 mL of 2M potassium carbonate (61 mmol). The reaction medium is stirred and heated to 40°C; 2 g of tetrakis(triphenylphosphine)palladium (Pd(PPh₃)₄) (1.74 mmol) are added and the medium is heated to 110°C and stirred for 3 hours. The reaction is stopped by adding 200 mL of water and the medium is then extracted with 200 mL of ethyl acetate. The organic phases are washed with 400 mL of water and neutralized with 200 mL of saturated NH₄Cl and then dried over magnesium sulfate. The solvents are evaporated off and the residue is then purified by chromatography on silica gel (eluent: 9/1 heptane/ethyl acetate). 1.2 g of ethyl 3"-*tert*-butyl-4"-diethylamino-4'-hydroxy[1,1';3',1"]terphenyl-4-carboxylate are obtained (yield = 16%) in the form of an orange solid.

¹H NMR (CDCl₃, 400 MHz): 1.14 (t, *J*=7.4Hz, 6H); 1.44 (t, *J*=7.6H, 3H); 1.51 (s, 9H); 2.94 (bs, 2H); 3.00 (bs, 2H); 4.42 (q, *J*=7.6Hz, 2H); 5.49 (s, 1H); 7.11 (d, *J*=8.5Hz, 1H); 7.33-7.41 (m, 2H); 7.53-7.57 (m, 3H); 7.68 (d, *J*=6.7Hz, 2H); 8.11 (d, *J*=6.7Hz, 2H).

### Example 2 - Ethyl 3"-tert-butyl-4"-diethylamino-4'-(4-hydroxybutoxy)-[1,1';3',1"]-terphenyl-4-carboxylate

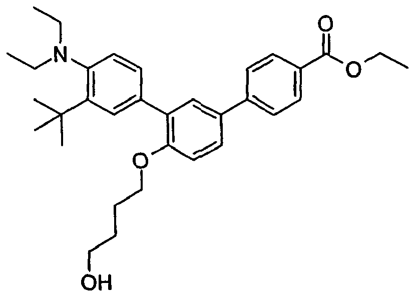

### a) Ethyl 3'-tert-butyl-4'-[4-(tert-butyldimethylsilanyloxy)butoxy]-4"-diethylamino[1,1;3;1']-terphenyl-4-carboxylate

1 g of ethyl 3"-*tert*-butyl-4"-diethylamino-4'-hydroxy[1,1';3',1"]terphenyl-4-carboxylate obtained in Example 1d (2.2 mmol) are dissolved in 20 mL of dimethylformamide under a nitrogen atmosphere. 880 mg (2.7 mmol) of caesium carbonate are added. The reaction medium stirred at room temperature turns yellow. 0.64 mL of 1-bromo-4-(*tert-*butyldimethylsilanyloxy)butane (2.4 mmol) is then added and the reaction medium is heated at 80°C for 18 hours. The reaction medium is then cooled to room temperature and then filtered. The solvents are evaporated off and the residue obtained is purified by chromatography on silica gel (eluent: 70/30 heptane/ethyl acetate).

1.4 g of ethyl 3"-*tert*-butyl-4'-[4-(tert-butyldimethylsilanyloxy)butoxy]-4"-diethylamino-[1,1';3',1"]terphenyl-4-carboxylate are obtained (yield = 99%) in the form of an oil.

### b) Ethyl 3"-tert-butyl-4"-diethylamino-4'-(4-hydroxybutoxy)-[1,1';3;1"]terphenyl-4-carboxylate

1.6 g of ethyl 3"-*tert*-butyl-4'-[4-(*tert*-butyldimethylsilanyloxy)butoxy]-4"-diethylamino-[1,1';3',1"]terphenyl-4-carboxylate (2.5 mmol) are dissolved in 20 mL of tetrahydrofuran under a nitrogen atmosphere. 3 mL of a 1M solution of tetrabutylammonium fluoride are then added dropwise. The reaction medium is stirred at room temperature for 3 hours, and the reaction is then stopped by adding 10 mL of water and then extracted with ethyl acetate. The organic phases are combined and dried over magnesium sulfate. The solvents are evaporated off and the residue is precipitated from 10 mL of heptane and filtered. 900 mg of ethyl 3"-*tert* butyl-4"-diethylamino-4'-(4-hydroxybutoxy)-[1,1';3',1 "]terphenyl-4-carboxylate are obtained (yield = 92%).

¹H NMR (CDCl₃ - 400 MHz): 1.12 (t, *J*=7.8Hz, 6H); 1.43 (t, *J*=7.5Hz, 3H); 1.51 (s, 9H); 1.64-1.71 (m, 2H); 1.85-2.07 (m, 2H); 2.91 (bs, 2H); 2.99 (bs, 2H); 3.62 (t, *J*=6.1Hz, 2H); 4.08 (t, *J*=6.1 Hz, 2H); 4.41 (t, *J*=7.5Hz, 2H); 7.07 (d, *J*=8.0Hz, 1H); 7.30 (d, *J*=8.0Hz, 1H); 7.38 (d, *J*=8.0Hz, 1H); 7.56-7.59 (m, 1H); 7.62-7.64 (m, 2H); 7.68 (d, *J*=8.2Hz, 2H); 8.11 (d, *J*=8.2Hz, 2H).

### Example 3 - 3"-tert-Butyl-4"-diethylamino-4'-(4-hydroxybutoxy)-[1,1';3',1"]terphenyl-4-carboxylic acid

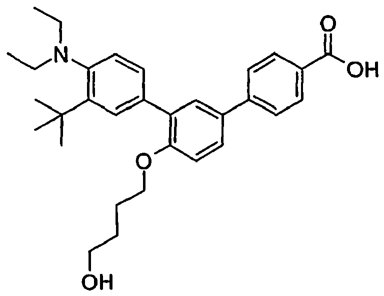

### a) 3"-tert-Butyl-4"-diethylamino-4'-(4-hydroxybutoxy)-[1,1';3',1"]terphenyl-4-carboxylic acid

0.9 mL of aqueous 1 N sodium hydroxide solution is added to a solution of 300 mg of ethyl 3"-*tert*-butyl-4"-diethylamino-4'-(4-hydroxybutoxy)-[1,1';3',1"]terphenyl-4-carboxylate (Example 2b) in a mixture of 10 mL of tetrahydrofuran and 3 mL of water. The reaction medium is stirred at room temperature for 12 hours. 0.5 mL of aqueous 1 N sodium hydroxide solution is then added. After 12 hours at room temperature, the reaction medium is heated to 50°C and then left at room temperature for 3 days. The reaction is stopped by adding 5 mL of water. The reaction medium is acidified to pH 5 by adding aqueous 1 N hydrochloric acid solution and is then extracted with ethyl acetate. The organic phase is dried over magnesium sulfate. The solid obtained is taken up in heptane and then filtered. 235 mg of 3"-*tert*-butyl-4"-diethylamino-4'-(4-hydroxybutoxy)-[1,1';3',1"]terphenyl-4-carboxylic acid are obtained in the form of a white solid (yield = 83%, m.p. = 165°C).

¹H NMR (DMSO-D₆ 400 MHz): 1.06 (t, 6H); 1.45 (s, 9H); 1.52 (m, 2H); 1.73 (m, 2H); 2.9 (m, 4H); 3.39 (t, 2H); 4.05 (t, 2H); 7.2 (dd, *J*=8.5Hz, 1H); 7.3 (dd, *J*=8.15Hz, 1H); 7.39 (d, *J*=6.93Hz, 1H); 7.6 (s, 1H); 7.64 (s, 1H); 7.66 (d, *J*=8.5Hz 2H); 7.8 (d, 2H, *J*=8.3Hz); 7.97 (d, *J*=8.3Hz, 1H).

### Example 4 - Ethyl 4"-(acetylethylaminol-3"-tert-butyl-4'-(4-hydroxybutoxyl-[1,1';3',1"]terphenyl-4-carboxylate

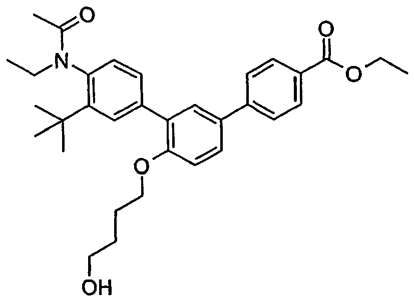

### a) (4-Bromo-2-tert-butylphenyl)ethylamine

In a 1 L three-necked flask under a nitrogen atmosphere, equipped with a magnetic stirrer, 28 g (123 mmol) of 4-bromo-2-*tert*-butylphenylamine are dissolved in 280 mL of dimethylformamide. 5.4 g (135 mmol) of sodium hydride (60%) are added portionwise and the reaction medium is stirred for 10 minutes. 140 mL of dimethyl sulfoxide are then added slowly. The reaction medium is stirred at room temperature for 2 hours, 23 g (147 mmol, 11.8 mL) of ethyl iodide are introduced dropwise and the medium is then stirred for 18 hours at room temperature. The reaction medium is poured into 1 L of water and extracted three times with ethyl acetate.

The organic phases obtained are combined and washed with water, dried over anhydrous magnesium sulfate, filtered and evaporated to give a reddish oil. This residue is purified on silica (eluent: 95/5 heptane/ethyl acetate) to give 25 g (yield = 79%) of (4-bromo-2-*tert-*butylphenyl)ethylamine in the form of a dark reddish oil.

### b) N-(4-Bromo-2-tert-butylphenyl)-N-ethylacetamide

In a 250 mL three-necked flask under a nitrogen atmosphere, equipped with a magnetic stirrer, 15 g (58.5 mmol) of (4-bromo-2-*tert*-butylphenyl)ethylamine are dissolved in 150 mL of dichloromethane. 8.9 g (87.8 mmol, 12.2 mL) of triethylamine and 0.72 g (5.9 mmol) of 4-dimethylaminopyridine are added. 11.5 g (146 mmol, 2.5 mL) of acetyl chloride are added dropwise and the reaction medium is stirred for 1 hour at room temperature. The reaction medium is poured into 250 mL of water and extracted twice with dichloromethane. The organic phases obtained are combined and washed with water, dried over anhydrous magnesium sulfate, filtered and evaporated to give a blackish oil. This oil is purified by chromatography on silica (eluent: 80/20 heptane/ethyl acetate) to give 15 g (yield = 86%) of N-(4-bromo-2-*tert*-butylphenyl)-N-ethylacetamide in the form of an orange oil.

### c) N-[2-tert-Butyl-4-(4,4,5,5-tetramethyl[1,3,2]dioxaborolan-2-yl)phenyl]-N-ethylacetamide.

In a 250 mL three-necked flask under a nitrogen atmosphere, equipped with a magnetic stirrer, 10 g (33.5 mmol) of N-(4-bromo-2-*tert*-butylphenyl)-N-ethylacetamide are dissolved in 100 mL of dimethylformamide. The solution is then degassed by sparging with nitrogen for 15 minutes. 13.2 g (134 mmol) of potassium acetate, 12.8 g (50.3 mmol) of bis(pinacolato)diborane and 2.73 g (3.35 mmol) of [1,1'-bis(diphenylphosphino)-ferrocene]dichloropalladium (II) are then added; the reaction medium is heated at 80°C and stirred at this temperature for 3 hours. The reaction medium is poured into 500 mL of water and extracted twice with ethyl acetate. The organic phases obtained are combined and washed with water, dried over anhydrous magnesium sulfate, filtered and evaporated to give a blackish oil. This oil is purified by chromatography on silica (eluent: 90/10 heptane/ethyl acetate) to give 12 g (yield = 100%) of N-[2-*tert*-butyl-4-(4,4,5,5-tetramethyl[1,3,2]dioxaborolan-2-yl)phenyl]-N-ethylacetamide in the form of a beige-coloured powder.

### d) Ethyl 4"-(acetylethylamino)-3"-tert-butyl-4'-hydroxy[1,1'; 3',1"]terphenyl-4-carboxylate.

In a manner similar to that of Example 1d, by reacting 2 g of ethyl 3'-bromo-4'-hydroxybiphenyl-4-carboxylate (6 mmol) with 2.7 g of N-[2-*tert*-butyl-4-(4,4,5,5-tetramethyl-[1,3,2]dioxaborolan-2-yl)phenyl]-N-ethylacetamide (9 mmol) in the presence of tetrakis-(triphenylphosphine)palladium. 1.9 g of ethyl 4"-(acetylethylamino)-3"-*tert*-butyl-4'-hydroxy[1,1';3',1"]terphenyl-4-carboxylate (yield = 70%) are obtained in the form of a beige-coloured powder.

### e) Ethyl 4"-(acetylethylamino)-3"-tert-butyl-4'-[4-(tert-butyldimethylsilanyloxy)butoxy]-[1,1';3,1"]terphenyl-4-carboxylate

In a manner similar to that of Example 2a, by reacting 260 mg of ethyl 4"-(acetylethylamino)-3"-*tert*-butyl-4'-hydroxy[1,1';3',1"]terphenyl-4-carboxylate (0.56 mmol) in 12 mL of dimethylformamide with 225 mg of caesium carbonate and 0.16 mL of 1-bromo-4-(*tert*-butyldimethylsilanyloxy)butane, 310 mg of ethyl 4"-(acetylethylamino)-3"-*tert*-butyl-4'-[4-(*tert*-butyldimethylsilanyloxy)butoxy]-[1,1';3',1"]terphenyl-4-carboxylate (yield = 99%) are obtained in the form of an oil.

### f) Ethyl 4"-(acetylethylamino)-3"-tert-butyl-4'-(4-hydroxybutoxy)-[1,1';3',1"]terphenyl-4-carboxylate

In a manner similar to that of Example 2b, by reacting 320 mg of ethyl 4"-(acetylethylamino)-3"-*tert-*butyl-4'-[4-(tert-butyldimethylsilanyloxy)butoxy]-[1,1';3',1"]-terphenyl-4-carboxylate in 10 mL of tetrahydrofuran with 0.5 mL of 1 M tetrabutylammonium fluoride, 270 mg of ethyl 4"-(acetylethylamino)-3"-*tert*-butyl-4'-(4-hydroxybutoxy)-[1,1';3',1"]terphenyl-4-carboxylate are obtained (yield = 99%).

¹H NMR (CDCl₃ - 400 MHz): 1.27 (t, 3H); 1.42 (s, 9H); 1.43 (t, 3H); 1.63-1.69 (m, 2H); 1.89 (s, 3H); 1.87-1.91 (m, 2H); 2.85-2.91 (m, 2H); 3.60-3.64 (m, 2H); 4.11 (t, *J*=6.1Hz, 2H); 4.42 (t, *J*=7.5Hz, 2H); 7.06 (d, *J*=8.0Hz, 1H); 7.10 (d, *J*=8.0Hz, 1H); 7.42 (d, *J*=8.0Hz, 1H); 7.61-7.63 (m, 2H); 7.68 (d, *J*=8.2Hz, 2H); 7.81 (s, 1H); 8.12 (d, *J*=8.2Hz, 2H).

### Example 5 - 4"-(Acetylethylaminol-3"-tert-butyl-4'-(4-hydroxybutoxyl-[1,1';3',1"]-terphenyl-4-carboxylic acid

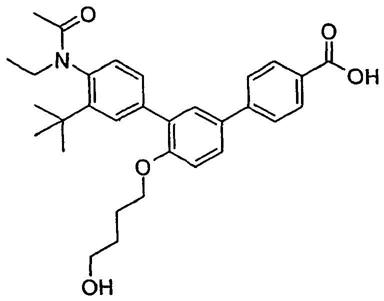

### a) 4'-(Acetylethylamino)-3"-tert-butyl-4'-(4-hydroxybutoxy)-[1,1';3',1"]terphenyl-4-carboxylic acid

In a manner similar to that of Example 3, by reacting 270 mg of ethyl 4"-(acetyl-ethylamino)-3"-*tert*-butyl-4'-(4-hydroxybutoxy)-[1,1';3',1"]terphenyl-4-carboxylate with 0.8 mL and then 0.4 mL of aqueous 1 N sodium hydroxide solution in a mixture of 8 mL of tetrahydrofuran and 2 mL of water. 195 mg of 4"-(acetylethylamino)-3"-*tert*-butyl-4'-(4-hydroxybutoxy)-[1,1';3',1"]terphenyl-4-carboxylic acid are obtained in the form of a white solid (yield = 95%, m.p. = 75°C).

¹H NMR (DMSO, 400 MHz): 1.26 (m, 3H); 1.43 (s, 9H); 1.67 (m, 2H); 1.87 (s, 3H); 1.89 (m, 2H); 2.92 (m, 1H); 3.63 (m, 2H); 4.11 (m, 2H); 4.4 (m, 1H); 7.0 (m, 1H); 7.36 (m, 1H); 7.58 (m, 4H); 7.77 (m, 1H); 8.12 (m, 2H).

### Example 6 3"-tert-Butyl-4"-diethylamino-4'-(2-hydroxyethoxy)-[1,1';3',1"]-terphenyl-4-carboxylic acid

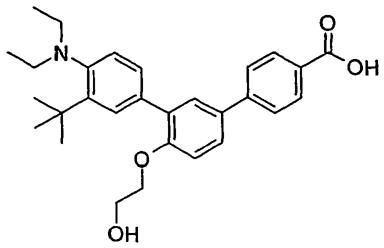

### a) Ethyl 4'-(2-acetoxyethoxy)-3"-tert-butyl-4"-diethylamino[1,1';3',1"]terphenyl-4-carboxylate

850 mg (1.9 mmol) of ethyl 3"-*tert*-butyl-4"-diethylamino-4'-hydroxy[1,1';3',1"]terphenyl-4-carboxylate (obtained in Example 1d) are dissolved in dimethylformamide under nitrogen. 92 mg (2.3 mmol) of 60% sodium hydride are added. After 20 minutes at room temperature, 0.25 mL (2.3 mmol) of 2-bromoethan-l-ol acetate are added and the mixture is stirred for 24 hours at room temperature and then poured into saturated ammonium chloride solution and extracted with ethyl acetate. The organic phase is washed with water and then dried and evaporated. The residue obtained is purified by chromatography on silica gel (eluent: 70/30 heptane/ethyl acetate). 1 g of ethyl 4'-(2-acetoxyethoxy)-3"-*tert*-butyl-4"-diethylamino[1,1';3',1"]terphenyl-4-carboxylate is obtained (yield = 99%) in the form of a colourless oil.

### b) 3"-tert-Butyl-4"-diethylamino-4-(2-hydroxyethoxy)-[1,1';3',1"]terphenyl-4-carboxylic acid

1 g (1.9 mmol) of ethyl 4'-(2-acetoxyethoxy)-3"-*tert*-butyl-4"-diethylamino[1,1';3',1"]-terphenyl-4-carboxylate is placed in 50 mL of tetrahydrofuran and 19 mL (19 mmol) of 1 N sodium hydroxide solution are added. The mixture is stirred at reflux for 12 hours. The yellow solution obtained is poured into saturated ammonium chloride solution, the pH is adjusted to 5-6 with 1 N hydrochloric acid solution and the mixture is then extracted with ethyl acetate. The organic phase is dried over magnesium sulfate and then concentrated to dryness. 455 mg of 3"-*tert*-butyl-4"-diethylamino-4'-(2-hydroxyethoxy)-[1,1';3',1"]-terphenyl-4-carboxylic acid are obtained in the form of a white solid (yield = 52%, m.p. = 216°C)

¹H NMR (DMSO, 400 MHz): 1.06 (t, *J*=7.2Hz, 6H); 1.47 (s, 9H); 2.84 (m, 2H); 2.94 (m, 2H); 4.11 (m, 2H); 7.23 (d, *J*=8Hz, 1H); 7.32 (d, *J*=8Hz, 1H); 7.48 (dd, *J*=1.6Hz, 8.4Hz, 1H); 7.66 (dd, *J*=1.2Hz, 4Hz, 2H); 7.69 (d, *J*=2Hz, 1H); 7.99 (d, *J*=8Hz, 2H); 7.82 (d, *J*=8Hz, 2H).

### Example 7 - Ethyl 3"-tert-butyl-4"-diethylamino-4'-(3-hydroxypropoxyl-[1,1';3',1"]-terphenyl-4-carboxylate

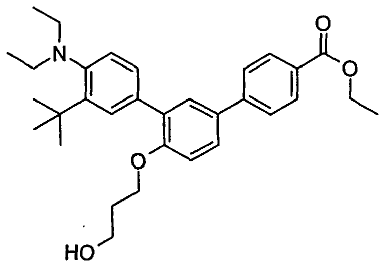

### a) Ethyl 3"-tert-butyl-4'-[3-(tert-butyldimethylsilanyloxy)propoxy]-4"-diethylamino-[1,1;3;1"]terphenyl-4-carboxylate

In a manner similar to that of Example 2a, by reacting 1 g of ethyl 3"-*tert*-butyl-4"-diethylamino-4'-hydroxy[1,1';3',1"]terphenyl-4-carboxylate (obtained in Example 1d) (2.2 mmol) in 20 mL of dimethylformamide with 880 mg (2.7 mmol) of caesium carbonate and 0.6 mL of 1-bromo-3-(tert-butyldimethylsilanyloxy)propane (2.4 mmol). 1.35 g of ethyl 3"-*tert-*butyl-4'-[4-(*tert*-butyldimethylsilanyloxy)propoxy]-4"-diethylamino[1,1';3',1"]terphenyl-4-carboxylate are obtained (yield = 99%) in the form of an oil.

### b) Ethyl 3"-tert-butyl-4"-diethylamino-4'-(4-hydroxypropoxy)-[1,1';3',1"]terphenyl-4-carboxylate

In a manner similar to that of Example 2b, by reacting 1 g of ethyl 3"-*tert*-butyl-4'-[4-(*tert* butyldimethylsilanyloxy)propoxy]-4"-diethylamino[1,1';3',1"]terphenyl-4-carboxylate (1.62 mmol) in 25 mL of tetrahydrofuran with 2 mL of 1 M tetrabutylammonium fluoride. 790 mg of ethyl 3"-*tert*-butyl-4"-diethylamino-4'-(4-hydroxypropoxy)-[1,1';3',1"]terphenyl-4-carboxylate are obtained (yield = 97%, m.p. = 114-115°C).

¹H NMR (DMSO, 400 MHz): 1.10 (t, *J*=7.1 Hz, 6H); 1.41 (t, *J*=7.1Hz, 3H); 1.49 (s, 9H); 1.81 (m, 1H); 2.90 (bs, 2H); 3.0 (bm, 2H); 3.88 (m, 2H); 4.13 (t, *J*=4.4Hz, 2H); 4.40 (q, *J*=7.1 Hz, 2H); 7.08 (d, *J*=8.5Hz, 1H); 7.30 (m, 1H); 7.36 (m, 1H); 7.56 (m, 1H); 7.62 (m, 2H); 7.66 (d, *J*=8.4Hz, 2H); 8.09 (d, *J*=8.1 Hz, 2H).

### Example 8 - 3"-tert-Butyl-4"-diethylamino-4'-(2-hydroxypropoxy)-(1,1';3',1"]-terphenyl-4-carboxylic acid

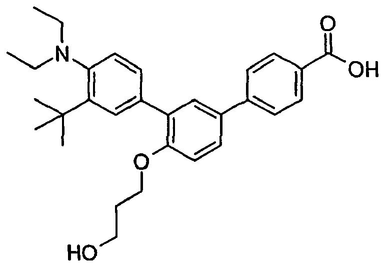

In a manner similar to that of Example 3, by reacting 870 mg of ethyl 3"-*tert*-butyl-4"-diethylamino-4'-(4-hydroxypropoxy)-[1,1';3',1"]terphenyl-4-carboxylate (Example 6b, 1.7 mmol) with 15 mL of 1N sodium hydroxide solution. 410 mg of 3"-*tert*-butyl-4"-diethylamino-4'-(2-hydroxypropoxy)-[1,1';3',1"]terphenyl-4-carboxylic acid are obtained in the form of a white solid (yield = 51%, m.p. = 193°C).

¹H NMR (DMSO, 400 MHz): 1.05 (t, *J*=7Hz, 6H); 1.46 (s, 9H); 1.85 (t, *J*=6Hz, 2H); 2.50 (m, 2H); 2.95 (m, 2H); 3.51(t, *J*=8Hz, 2H); 4.12 (t, *J*=4Hz, 2H); 4.50 (s, 1H); 7.22 (d, *J*=8Hz, 1H); 7.32 (d, *J*=8Hz, 1H); 7.41 (dd, *J*=1.6Hz, 8Hz, 1H); 7.61 (d, *J*=1.6Hz, 1H); 7.65 (d, *J*=2.4Hz, 1H); 7.70 (dd, *J*=2Hz, 8Hz, 1H); 7.83 (d, *J*=8.4Hz, 2H); 7.99 (d, *J*=8Hz, 2H); 13.1 (s, 1H).

### Example 9 - Ethyl 4"-diethylamino-4'-(3-hydroxypropoxy)-[1,1';3',1"]terphenyl-4-carboxylate

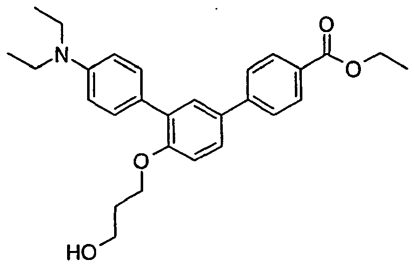

### a) 4- Diethylaminophenylboronic acid

In a manner similar to that of Example 1c, by reacting 5 g of (4-bromophenyl)diethylamine (21.9 mmol) with 13 mL of 2.0 M n-butyllithium solution and 6 mL (26 mmol) of triisopropyl borate. 4 g of 4-diethylaminophenylboronic acid (yield = 94 %) are obtained in the form of a white solid.

### b) Ethyl 3'-bromo-4'-[3-(tert-butyldimethylsilanyloxy)propoxy]biphenyl-4-carboxylate

In a manner similar to that of Example 2a, by reacting 3 g of ethyl 3'-bromo-4'-hydroxybiphenyl-4-carboxylate (9.3 mmol) in 100 mL of dimethylformamide with 448 mg (11 mmol) of sodium hydride and 2.6 mL of 1-bromo-3-(tert-butyldimethylsilanyloxy)-propane (11 mmol). 2.95 g of ethyl 3'-bromo-4'-[3-(*tert*-butyldimethylsilanyloxy)propoxy]-biphenyl-4-carboxylate are obtained (yield = 64%) in the form of an oil.

### c) Ethyl 4'-[3-(tert-butyldimethylsilanyloxy)propoxy]-4"-diethylamino[1,1;3;1"]terphenyl-4-carboxylate

In a manner similar to that of Example 1d, by reacting 1 g of ethyl 3'-bromo-4'-[3-(*tert-*butyldimethylsilanyloxy)propoxy]biphenyl-4-carboxylate (2 mmol) with 580 mg of 4-diethylaminophenylboronic acid (3 mmol) in the presence of tetrakis(triphenylphosphine)-palladium. 1.15 g of ethyl 4'-[3-(tert-butyldimethylsilanyloxy)propoxy]-4"-diethylamino-[1,1';3',1"]terphenyl-4-carboxylate (yield = 100 %) are obtained in the form of a colourless oil.

### d) Ethyl 4"-diethylamino-4'-(3-hydroxypropoxy)-[1,1';3,1"]terphenyl-4-carboxylate

In a manner similar to that of Example 2b, by reacting 730 mg of ethyl 4'-[3-(*tert-*butyldimethylsilanyloxy)propoxy]-4"-diethylamino[1,1';3',1"]terphenyl-4-carboxylate (1.3 mmol) in 25 mL of tetrahydrofuran with 1.6 mL of 1M tetrabutylammonium fluoride. 430 mg of ethyl 4"-diethylamino-4'-(3-hydroxypropoxy)-[1,1';3',1"]terphenyl-4-carboxylate are obtained (yield = 74%).

¹H NMR (CDCl₃, 400 MHz): 1.22 (t, *J*=7.4Hz, 6H); 1.43 (t, *J*=7.6H, 3H); 2.05 (m, 2H); 3.42 (q, *J*=7.4Hz, 4H); 3.81 (m, 2H); 4.17 (t, *J*=5.7Hz, 2H); 4.41 (q, *J*=7.6Hz, 2H); 6.76 (d, *J*=8.9Hz, 1H); 7.08 (d, *J*=8.1Hz, 2H); 7.45-7.47 (m, 2H); 7.52 (dd, J₁=2.3Hz, J₂=8.6Hz, 1H); 7.62 (d, *J*=2.4Hz, 1H); 7.67 (d, *J*=6.7Hz, 2H); 8.10 (d, *J*=6.7Hz, 2H).

### Example 10 - 4"-Diethylamino-4'-(3-hydroxypropoxy)-[1,1';3',1"]terphenyl-4-carboxylic acid

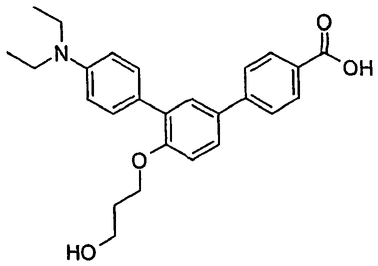

In a manner similar to that of Example 3, by reacting 430 mg of ethyl 4"-diethylamino-4'-(3-hydroxypropoxy)-[1,1';3',1"]terphenyl-4-carboxylate (Example 8d, 0.9 mmol) with 10 mL of 1N sodium hydroxide solution, 100mg of 4"-diethylamino-4'-(3-hydroxypropoxy)-[1,1;3',1"]terphenyl-4-carboxylic acid are obtained in the form of a yellow solid (yield = 25%, m.p. = 200°C).

**HPLC** Thermo Aquasil C₁₈, 3 microns, 2 x 150 mm, mobile phase: A (CH₃CN / 0.1 v/v HCO₂H); B (H₂O / 0.1 v/v HCO₂H), Flow rate: 0.5 mL/minutes. Gradient: 0 min: 90% B, 0-20 min: 90-5% B, 20-30 min: 5% B; retention time: 10.01 min, purity: 96%, MS (ESI) m/z 420.22 (M+H)⁺

### Example 11 - Ethyl 4"-diethylamino-3"-ethyl-4'-(3-hydroxypropoxy)-[1,1';3',1"]-terphenyl-4-carboxylate

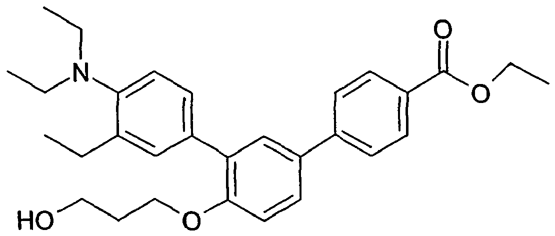

### a) (4-Bromo-2-ethylphenyl)diethylamine

In a manner similar to that of Example 1b, by reacting 6 g (0.15 mol) of sodium hydride with 10 g (50 mmol) of 4-bromo-2-ethylaniline, 11.3 g of (4-bromo-2-ethylphenyl)-diethylamine are obtained (yield = 88 %) in the form of a yellow oil.

### b) 4-Diethylamino-3-ethylphenylboronic acid

In a manner similar to that of Example 1c, by reacting 3 g of (4-bromo-2-ethylphenyl)diethylamine (11.7 mmol) with 5.2 mL of a 2.5 M solution of *n*-butyllithium and 3.2 mL (14 mmol) of triisopropyl borate, 0.9 g of 4-diethylamino-3-ethylphenylboronic acid (yield = 35 %) is obtained in the form of a white solid.

### c) Ethyl 4'-[3-(tert-butyldimethylsilanyloxy)propoxy]-4"-diethylamino-3"-ethyl[1,1';3',1"]-terphenyl-4-carboxylate

In a manner similar to that of Example 1d, by reacting 1.4 g (2.9 mmol) of ethyl 3'-bromo-4'-[3-(*tert*-butyldimethylsilanyloxy)propoxy]biphenyl-4-carboxylate (obtained in Example 5b) with 900 mg of 4-diethylamino-3-ethylphenylboronic acid (4 mmol) in the presence of tetrakis(triphenylphosphine)palladium, 674 mg of ethyl 4'-[3-(*tert*-butyldimethylsilanyloxy)-propoxy]-4"-diethylamino-3"-ethyl[1,1';3',1"]terphenyl-4-carboxylate (yield = 39 %) are obtained in the form of a colourless oil.

### d) Ethyl 4"-diethylamino-3"-ethyl-4'-(3-hydroxypropoxy)-[1,1';3',1"]terphenyl-4-carboxylate

In a manner similar to that of Example 2b, by reacting 660 mg of ethyl 4'-[3-(*tert-*butyldimethylsilanyloxy)propoxy]-4"-diethylamino-3"-ethyl[1,1';3',1"]terphenyl-4-carboxylate (1.1 mmol) in 25 mL of tetrahydrofuran with 1.2mL of 1M tetrabutylammonium fluoride. 280 mg of ethyl 4"-diethylamino-3"-ethyl-4'-(3-hydroxypropoxy)-[1,1';3',1"]terphenyl-4-carboxylate are obtained (yield = 52%).

¹H NMR (CDCl₃, 400 MHz): 1.05 (t, *J*=7.4Hz, 6H); 1.28 (t, *J*=7.5Hz, 3H); 1.44 (t, *J*=7.6H, 3H); 2.02 (m, 2H); 2.80 (q, *J*=7.5Hz, 2H); 3.02 (q, *J*=7.4Hz, 4H); 3.77 (q, *J*=5.6Hz, 2H); 4.17 (t, *J*=5.7Hz, 2H); 4.42 (q, *J*=7.6Hz, 2H); 7.10 (d, *J*=8.5Hz, 1H); 7.18 (d, *J*=8.1Hz, 1H); 7.35 (dd, *J*₁=2.1 Hz, *J*₂=8.2Hz, 1H); 7.44 (d, *J*=2.1 Hz, 1H); 7.57 (dd, *J*₁=2.4Hz, *J*₂=8.5Hz, 1H); 7.63 (d, *J*=2.4Hz, 1H); 7.57 (dd, *J*₁=2.4Hz, *J*₂=8.5Hz, 1H); 7.64 (d, *J*=2.4Hz, 1H); 7.69 (d, *J*=6.7Hz, 2H); 8.11 (d, *J*=6.7Hz, 2H).

### Example 12 - 4"-Diethylamino-3"-ethyl-4'-(3-hydroxypropoxyl-[1,1';3',1"]terphenyl-4-carboxylic acid

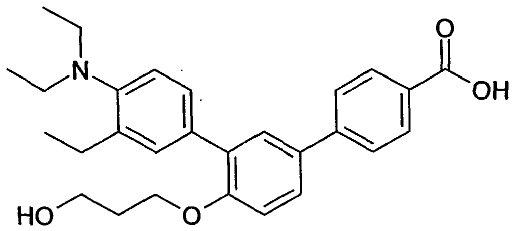

### a) 4"-Diethylamino-3"-ethyl-4'-(3-hydroxypropoxy)-[1,1;3',1"]terphenyl-4-carboxylic acid

Into a 25 mL three-necked flask under a nitrogen atmosphere, equipped with a magnetic stirrer, are placed 270 mg (0.57 mmol) of ethyl 4"-diethylamino-3"-ethyl-4'-(3-hydroxypropoxy)-[1,1';3',1"]terphenyl-4-carboxylate (Example 10d) and 22 mg (5.7 mmol) of solid sodium hydroxide in 5.4 mL of tetrahydrofuran. The reaction medium is heated at the reflux temperature of the tetrahydrofuran for 5 hours. The reaction medium is concentrated under vacuum. The crude reaction product obtained is taken up in water and the pH of the medium thus obtained is adjusted to pH=4 by addition of hydrochloric acid solution in a proportion of 1 mol/L. The precipitate obtained, after stirring for 30 minutes, is purified by chromatography on silica (eluent: 40/60 heptane/ethyl acetate) to give, after evaporation of the fractions, 80 mg (yield = 31%) of 4"-diethylamino-3"-ethyl-4'-(3-hydroxypropoxy)-[1,1';3',1"]terphenyl-4-carboxylic acid in the form of a cream-coloured powder (m.p. = 204°C).

¹H NMR (DMSO, 400 MHz): 1.02 (t, *J*=7.2 Hz, 6H); 1.25 (t, *J*=7.2 Hz, 3H); 2.02 (m, 2H); 2.78 (q, *J*=7.6 Hz, 2H); 3.01 (q, *J*=6.8 Hz, 4H); 3.76 (t, *J*=5.6 Hz, 2H); 4.18 (t, *J*=5.6 Hz, 2H); 7.08 (d, *J*=8.4 Hz, 1H); 7.16 (d, *J*=8.0 Hz, 1H); 7.33 (dd, J₁=2.0 Hz, J₂=8.4 Hz, 1H); 7.42 (d, *J*=1.6 Hz, 1H); 7.56 (dd, J₁=2.0 Hz, J₂=8.4 Hz, 1H); 7.63 (d, *J*=2.4 Hz, 1H); 7.70 (d, *J*=8.4 Hz, 2H); 8.14 (d, *J*=8.4 Hz, 2H).

### Example 13 - 4"-Diethylamino-3"-ethyl-4'-(2-hydroxyethoxy)-[1,1';3',1"]terphenyl-4-carboxylic acid

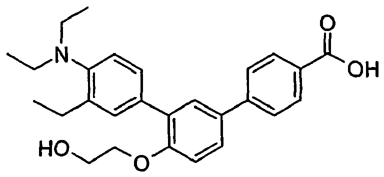

### a) Ethyl 4'-(2-acetoxyethoxy)-3'-bromobiphenyl-4-carboxylate

In a manner similar to that of Example 6a, by reacting 2 g (6 mmol) of ethyl 3'-bromo-4'-hydroxybiphenyl-4-carboxylate with 320 mg (8 mmol) of 60% sodium hydride and 0.7 mL (7 mmol) 2-bromoethan-1-ol acetate, 2.2 g of ethyl 4'-(2-acetoxyethoxy)-3'-bromobiphenyl-4-carboxylate are obtained (yield = 95%) in the form of a colourless oil.

### b) Ethyl 2'-(2-acetoxyethoxy)-3-tert-butyl-4-diethylamino[1,1;4',1"]terphenyl-3"-carboxylate

In a manner similar to that of Example 1d, by reacting 400 mg of ethyl 4'-(2-acetoxyethoxy)-3'-bromobiphenyl-4-carboxylate (1 mmol) with 340 mg of 4-diethylamino-3-ethylphenylboronic acid (1.5 mmol) in the presence of tetrakis(triphenylphosphine)palladium, 500 mg of ethyl 2'-(2-acetoxyethoxy)-3-*tert*-butyl-4-diethylamino-[1,1';4',1"]terphenyl-3"-carboxylate (yield = 97 %) are obtained in the form of a white solid.

### c) 4"-Diethylamino-3"-ethyl-4'-(2-hydroxyethoxy)-[1,1';3',1"]terphenyl-4-carboxylic acid

In a manner similar to that of Example 6b, by reacting 690 mg (1.4 mmol) of ethyl 2'-(2-acetoxyethoxy)-3-*tert*-butyl-4-diethylamino[1,1';4',1"]terphenyl-3"-carboxylate with 550 mg (14 mmol) of sodium hydroxide, 470 mg of 4"-diethylamino-3"-ethyl-4'-(2-hydroxyethoxy)-[1,1';3',1"]terphenyl-4-carboxylic acid are obtained (yield = 79 %) in the form of a white solid (m.p. = 206°C).

¹H NMR (DMSO, 400 MHz): 1.05 (t, *J*=7.0 Hz, 6H); 1.27 (t, *J*=7.6 Hz, 3H); 2.80 (q, *J*=7.2 Hz, 2H); 3.03 (q, *J*=7.2 Hz, 4H); 3.90 (m, 2H), 4.15 (m, 2H); 7.09 (m, 1H); 7.18 (m, 1H); 7.38 (m, 1H); 7.47 (m, 1H); 7.56 (m, 1H); 7.7 (m, 3H); 8.18 (d, *J*=8.4 Hz, 2H).

### Example 14 - Ethyl 4"-diethylamino-3"-ethyl-4'-(2-hydroxyethoxy)-[1,1';3',1"]-terphenyl-4-carboxylate

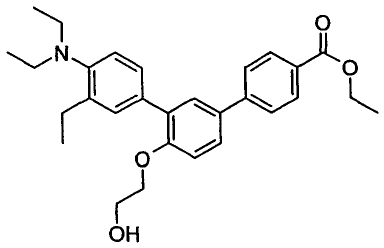

### a) Ethyl 4"-diethylamino-3"-ethyl-4'-(2-hydroxyethoxy)-[1,1';3',1"]terphenyl-4-carboxylate

6.6 g of ethyl 2'-(2-acetoxyethoxy)-3-*tert*-butyl-4-diethylamino[1,1';4',1"]terphenyl-3"-carboxylate (obtained in Example 13b) are dissolved in 200 mL of a 2% solution of potassium carbonate in ethanol. The reaction medium is stirred for 2 hours at room temperature and then poured into saturated ammonium chloride solution and extracted with ethyl acetate. The residue obtained is purified by chromatography on silica gel (eluent: 7/3 heptane/ethyl acetate). 4 g of ethyl 4"-diethylamino-3"-ethyl-4'-(2-hydroxy-ethoxy)-[1,1';3',1"]terphenyl-4-carboxylate (yield = 66%) are obtained in the form of a white solid (m.p. = 97°C).

¹H NMR (DMSO, 400 MHz): 1.06 (t, *J*=7.0Hz, 6H); 1.28 (t, *J*=7.5Hz, 3H); 1.44 (t, *J*=7.1 Hz, 3H); 1.91 (bs, 1H); 2.80 (q, *J*=7.5Hz, 2H); 3.04 (q, *J*=7.0Hz, 4H); 3.90 (bs, 2H); 4.16 (m, 2H); 4.42 (q, *J*=7.1 Hz, 2H); 7.10 (d, *J*=8.5Hz, 1H); 7.18 (d, *J*=8.5Hz, 1H); 7.37 (dd, *J*=2.1hz, 8.2Hz, 1H); 7.47 (d, *J*=2.0Hz, 1H); 7.57 (dd, *J*=2.4Hz, 8.5Hz, 1H); 7.66 (m, 1H); 7.69 (d, *J*=8.4Hz, 2H); 8.12 (d, *J*=8.4Hz, 2H).

### Example 15 - Ethyl 3"-tert-butyl-4"-diethylamino-4'-(2-hydroxyethoxy)-[1,1';3',1"]-terphenyl-4-carboxylate

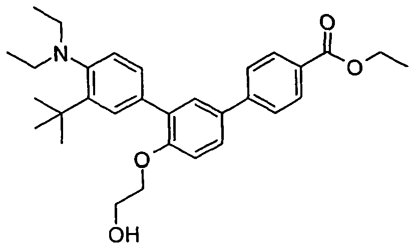

### a) Ethyl 3"-tert-butyl-4"-diethylamino-4'-(2-hydroxyethoxy)-[1,1';3',1"]terphenyl-4-carboxylate

In a manner similar to that of Example 14a, by reacting 490 mg of ethyl 4'-(2-acetoxyethoxy)-3"-*tert*-butyl-4"-diethylamino[1,1';3',1"]terphenyl-4-carboxylate (0.9 mmol) obtained in Example 6a with 10 mL of a 2% solution of potassium carbonate in ethanol, 400 mg of ethyl 3"-*tert*-butyl-4"-diethylamino-4'-(2-hydroxyethoxy)-[1,1';3',1"]terphenyl-4-carboxylate are obtained (yield = 82%) in the form of a white solid (m.p. = 127°C).

¹H NMR (DMSO, 400 MHz): 1.10 (t, *J*=7.0Hz, 6H); 1.41 (t, *J*=7.1Hz, 3H); 1.49 (s, 9H); 1.81 (bs, 1H); 2.90 (bs, 2H); 3.0 (bs, 2H); 3.87 (m, 2H); 4.13 (m, 2H); 4.40 (q, *J*=7.1 Hz, 2H); 7.08 (d, *J*=8.5Hz, 1H); 7.30 (d, *J*=8.5Hz, 1H); 7.35 (dd, *J*=2.1hz, 8.2Hz, 1H); 7.56 (dd, *J*=2.0Hz, 8.1Hz, 1H); 7.62 (dd, *J*=2.4Hz, 8.5Hz, 1H); 7.65 (m, 1H); 7.66 (d, *J*=8.4Hz, 2H); 8.09 (d, *J*=8.4Hz, 2H).

### Example 16 - Ethyl 4"-diethylamino-4'-(3-hydroxypropoxy)-3"-methyl[1,1';3',1"]-terphenyl-4-carboxylate

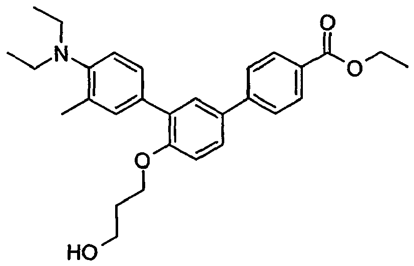

### a) (4-Bromo-2-methylphenyl)diethylamine

In a manner similar to that of Example 1b, by reacting 4.7 g (0.12 mol) of sodium hydride with 10 g (54 mmol) of 4-bromo-2-methylaniline, 8 g of (4-bromo-2-methylphenyl)diethylamine are obtained (yield = 62 %) in the form of a yellow oil.

### b) 4-Diethylamino-3-methylphenylboronic acid

In a manner similar to that of Example 1c, by reacting 8 g of (4-bromo-2-methylphenyl)diethylamine (33 mmol) with 16 mL of a 2.5 M solution of n-butyllithium and 11.5 mL (50 mmol) of triisopropyl borate, 7.6 g of 4-diethylamino-3-methylphenylboronic acid (yield = 100 %) are obtained in the form of a thick oil.

### c) Ethyl 4-[3-(tert-butyldimethylsilanyloxy)propoxy]-4'-diethylamino-3"-methyl[1,1;3',1"]-terphenyl-4-carboxylate

In a manner similar to that of Example 1d, by reacting 1 g (2 mmol) of ethyl 3'-bromo-4'-[3-(*tert*-butyldimethylsilanyloxy)propoxy]biphenyl-4-carboxylate (obtained in Example 6b) with 630 mg of 4-diethylamino-3-methylphenylboronic acid (3 mmol) in the presence of tetrakis(triphenylphosphine)palladium, 1.1 g of ethyl 4'-[3-(*tert*-butyldimethylsilanyloxy)-propoxy]-4"-diethylamino-3"-methyl[1,1';3',1"]terphenyl-4-carboxylate (yield = 95 %) are obtained in the form of a pale yellow oil.

### d) Ethyl 4"-diethylamino-4'-(3-hydroxypropoxy)-3"-methyl[1,1';3',1"]terphenyl-4-carboxylate

In a manner similar to that of Example 2b, by reacting 1.1 g of ethyl 4'-[3-(*tert-*butyldimethylsilanyloxy)propoxy]-4"-diethylamino-3"-methyl[1,1';3',1"]terphenyl-4-carboxylate (1.9 mmol) in 25 mL of tetrahydrofuran with 2.3 mL of 1M tetrabutylammonium fluoride, 130 mg of ethyl 4"-diethylamino-4'-(3-hydroxypropoxy)-3"-methyl[1,1';3',1"]terphenyl-4-carboxylate are obtained (yield = 15%).

¹H NMR (CDCl₃, 400 MHz): 1.06 (t, *J*=7.4Hz, 6H); 1.44 (t, *J*=7.6H, 3H); 2.05 (m, 2H); 2.37 (s, 3H); 3.05 (q, *J*=7.4Hz, 4H); 3.78 (q, *J*=5.6Hz, 2H); 4.19 (t, *J*=5.7Hz, 2H); 4.42 (q, *J*=7.6Hz, 2H); 7.09 (d, *J*=8.5Hz, 1H); 7.13 (d, *J*=8.1Hz, 1H); 7.35-7.39 (m, 2H); 7.57 (dd, J₁=2.4Hz, J₂=8.5Hz, 1H); 7.63 (d, *J*=2.4Hz, 1H); 7.68 (d, *J*=6.7Hz, 2H); 8.11 (d, *J*=6.7Hz, 2H).

### Example 17 - 4"-Diethylamino-4'-(3-hydroxypropoxy)-3"-methyl[1,1';3',1"]terphenyl-4-carboxylic acid

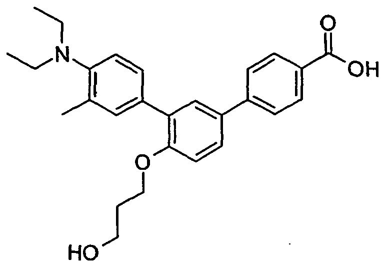

In a manner similar to that of Example 12a, by reacting 130 mg (0.3 mmol) of ethyl 4"-diethylamino-4'-(3-hydroxypropoxy)-3"-methyl[1,1';3',1"]terphenyl-4-carboxylate (Example 15d) with 3 mL of 1N sodium hydroxide solution. 60 mg of 4"-diethylamino-4'-(3-hydroxypropoxy)-3"-methyl[1,1';3',1"]terphenyl-4-carboxylic acid are obtained in the form of a white solid (yield = 46%, m.p. = 208°C).

**HPLC** Thermo Aquasil C₁₈, 3 microns, 2 x 150 mm, mobile phase: A (CH₃CN / 0.1 v/v HCO₂H); B (H₂O / 0.1v/v HCO₂H), Flow rate: 0.5 mL/minutes. Gradient: 0 min: 90% B, 0-20 min: 90-5% B, 20-30 min: 5% B; retention time: 8.95 min, purity: 92%, MS (ESI) m/z 434.3 (M+H)⁺.

### Example 18 - Ethyl 3"-tert-butyl-4'-(4-hydroxybutoxy)-4"-pyrrolidin-1-yl[1,1';3',1"]-terphenyl-4-carboxylate

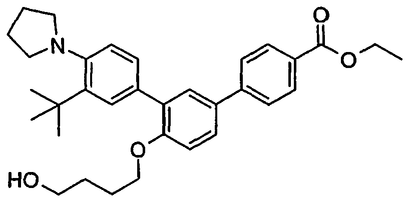

### a) 1-(4-Bromo-2-tert-butylphenyl)pyrrolidine

5.7 g (0.14 mol) of sodium hydride are suspended in 200 mL of tetrahydrofuran. 10 g (44 mmol) of 4-bromo-2-*tert*-butylaniline are added, along with 200 mL of dimethyl sulfoxide, added slowly. The mixture turns blue and, after 30 minutes, 13 mL (0.14 mol) of 1,4-dibromobutane are added and the reaction medium is stirred at room temperature for 13 hours. The reaction medium is then poured into saturated ammonium chloride solution and extracted with ethyl acetate, and the organic phase is then washed twice with water, dried and then concentrated to dryness. The residue is purified by chromatography on silica gel (eluent: 90/10 heptane/ethyl acetate). 6.4 g of 1-(4-bromo-2-*tert-*butylphenyl)pyrrolidine are obtained (yield = 52%) in the form of a thick yellow oil.

### b) 3-tert-Butyl-4-pyrrolidinophenylboronic acid

In a manner similar to that of Example 1c, by reacting 4.7 g (17 mmol) of 1-(4-bromo-2-tert-butylphenyl)pyrrolidine with 8 mL of a 2.5M solution of n-butyllithium and 6 mL (26 mmol) of triisopropyl borate, 2.8 g of 3-*tert*-butyl-4-pyrrolidinophenylboronic acid are obtained (yield = 66%).

### c) Ethyl 3"-tert-butyl-4'-hydroxy-4"-pyrrolidin-1-yl[1,1';3',1"]terphenyl-4-carboxylate

In a manner similar to that of Example 1d, by reacting 1 g of ethyl 3'-bromo-4'-hydroxybiphenyl-4-carboxylate (3 mmol) with 1.2 g of 3-*tert-*butyl-4-pyrrolidinophenylboronic acid (4.5 mmol) in the presence of 5 mL of 2M potassium carbonate (10 mmol) and 360 mg of tetrakis(triphenylphosphine)palladium (Pd(PPh₃)₄), 900 mg of ethyl 3"-*tert*-butyl-4'-hydroxy-4"-pyrrolidin-1-yl[1,1';3',1"]terphenyl-4-carboxylate are obtained (yield = 63 %) in the form of a yellow solid.

### d) Ethyl 3"-tert-butyl-4'-[4-(tert-butyldimethylsilanyloxy)butoxy]-4"-pyrrolidin-1-yl[1,1';3',1"]-terphenyl-4-carboxylate

In a manner similar to that of Example 2a, by reacting 123 mg of ethyl 3"-*tert*-butyl-4'-hydroxy-4"-pyrrolidin-1-yl[1,1';3',1"]terphenyl-4-carboxylate (0.27 mmol) with 110 mg (0.34 mmol) of caesium carbonate and 80 µL of 1-bromo-4-(*tert*-butyldimethylsilanyloxy)-butane (0.30 mmol), 170 mg of ethyl 3"-*tert*-butyl-4'-[4-(*tert*-butyldimethylsilanyloxy)-butoxy]-4"-pyrrolidin-1-yl[1,1';3',1"]terphenyl-4-carboxylate are obtained (yield = 97%) in the form of an oil.

### e) Methyl 3"-tert-butyl-4'-(4-hydroxybutoxy)-4"-pyrrolidin-1-yl[1,1';3',1"]terphenyl-4-carboxylate

In a manner similar to that of Example 2b, by reacting 170 mg (0.26 mmol) of ethyl 3"-*tert*-butyl-4'-[4-(*tert*-butyldimethylsilanyloxy)butoxy]-4"-pyrrolidin-1-yl[1,1';3',1"]terphenyl-4-carboxylate with 200 µL of a 1 M solution of tetrabutylammonium fluoride, 100 mg of ethyl 3"-*tert*-butyl-4'-(4-hydroxybutoxy)-4"-pyrrolidin-1-yl[1,1';3',1"]terphenyl-4-carboxylate are obtained (yield = 71%) in the form of a white solid.

¹H NMR (CDCl₃ - 400 MHz): 1.43 (t, *J*=7.5Hz, 3H); 1.47 (s, 9H); 1.64-1.71 (m, 2H); 1.85-1.96 (m, 2H); 2.10-2.25 (m, 4H); 3.03 (m, 4H); 3.64 (m, 2H); 4.09 (t, *J*=6.1Hz, 2H); 4.41 (t, *J*=7.5Hz, 2H); 7.07 (d, *J*=8.0Hz, 1H); 7.30 (s, 2H); 7.56-7.65 (m, 3H); 7.67 (d, *J*=8.2Hz, 2H); 8.10 (d, *J*=8.2Hz, 2H).

### Example 19 - 3"-tert-Butyl-4'-(4-hydroxybutoxy)-4"-pyrrolidin-1-yl[1,1';3',1"]-terphenyl-4-carboxylic acid

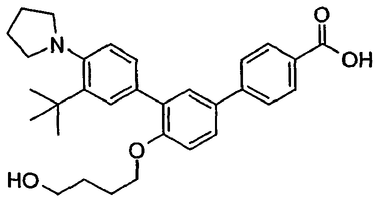

In a manner similar to that of Example 3, by reacting 100 mg of ethyl 3"-*tert*-butyl-4'-(4-hydroxybutoxy)-4"-pyrrolidin-1-yl[1,1';3',1"]terphenyl-4-carboxylate (0.18 mmol) with 0.3 mL of 1N sodium hydroxide solution. 50 mg of 3"-*tert*-butyl-4'-(4-hydroxybutoxy)-4"-pyrrolidin-1-yl[1,1';3',1"]terphenyl-4-carboxylic acid are obtained (yield = 56%) in the form of a white solid (m.p. = 206°C).

¹H NMR (DMSO, 400 MHz): 1.42 (s, 9H); 1.51 (m, 2H); 1.72 (m, 2H); 1.9 (s, 4H); 3.4 (m, 2H); 4 (m, 2H); 7.2 (d, 1H, *J*=8.6Hz); 7.4 (d, 1H, *J*=9.9Hz); 7.45 (d, *J*=8.2Hz); 7.55 (s); 7.63 (s, 1H); 7.67 (d, 1H, *J*=7.34Hz); 7.8 (d, 2H, *J*=8.45Hz); 7.97 (d, 2H, *J*=8.4Hz).

### Example 20 Ethyl 4'-(4-hydroxybutoxy)-4"-pyrrolidin-1-yl-3"-trifluoromethyl-[1,1';3',1"]terphenyl-4-carboxylate

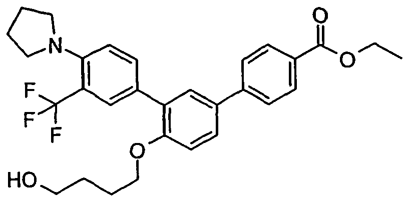

### a) 1-(4-Bromo-2-trifluoromethylphenyl)pyrrolidine

In a manner similar to that of Example 18a, by reacting 40 g of 4-bromo-2-trifluoromethylphenylamine (0.167 mol) with 16.6 g of sodium hydride (0.42 mol) and 49.7 mL of 1,4-dibromobutane (0.42 mol), 8.5 g of 1-(4-bromo-2-trifluoromethylphenyl)-pyrrolidine (yield = 17%) are obtained in the form of a yellow oil.

### b) 4-Pyrrolidino-3-trifluoromethylphenylboronic acid

In a manner similar to that of Example 1c, by reacting 2.3 g (7.9 mmol) of 1-(4-bromo-2-trifluoromethylphenyl)pyrrolidine with 3.8 mL of a 2.5M solution of n-butyllithium and 2.7 mL of triisopropyl borate (12 mmol), 2 g of 4-pyrrolidino-3-trifluoromethylphenyl-boronic acid are obtained (yield = 100%) in the form of a beige-coloured solid.

### c) Ethyl 3'-bromo-4'-(4-(tert-butyldimethylsilanyloxy)butoxy]biphenyl-4-carboxylate

In a manner similar to that of Example 2a, by reacting 2 g of ethyl 3'-bromo-4'-hydroxybiphenyl-4-carboxylate (6 mmol) with 2.4 g (7.5 mmol) of caesium carbonate and 1.78 mL of 1-bromo-4-(*tert*-butyldimethylsilanyloxy)butane (6.7 mmol), 2.8 g of ethyl 3'-bromo-4'-[4-(*tert*-butyldimethylsilanyloxy)butoxy]biphenyl-4-carboxylate are obtained (yield = 97%) in the form of an oil.

### d) Ethyl 4'-[4-(tert-butyldimethylsilanyloxy)butoxy]-4"-pyrrolidin-1-yl-3'-trifluoromethyl-[1,1',3,1"]terphenyl-4-carboxylate

In a manner similar to that of Example 1d, by reacting 500 mg of ethyl 3'-bromo-4'-[4-(*tert-*butyldimethylsilanyloxy)butoxy]biphenyl-4-carboxylate (1.2 mmol) with 450 mg of 4-pyrrolidino-3-trifluoromethylphenylboronic acid (1.7 mmol) in the presence of 1.5 mL of 2M potassium carbonate (3 mmol) and 40 mg of tetrakis(triphenylphosphine)palladium (Pd(PPh₃)₄), 400 mg of ethyl 4'-[4-(*tert*-butyldimethylsilanyloxy)butoxy]-4"-pyrrolidin-1-yl-3"-trifluoromethyl[1,1';3',1"]terphenyl-4-carboxylate are obtained (yield = 56%) in the form of a colourless oil.

### e) Ethyl 4'-(4-hydroxybutoxy)-4"-pyrrolidin-1-yl-3"-trifluoromethyl[1,1'; 3',1"]terphenyl-4-carboxylate

In a manner similar to that of Example 2b, by reacting 400 mg of ethyl 4'-[4-(*tert-*butyldimethylsilanyloxy)butoxy]-4"-pyrrolidin-1-yl-3"-trifluoromethyl[1,1';3',1"]terphenyl-4-carboxylate (0.6 mmol) with 0.75 mL of a 1N solution of tetrabutylammonium fluoride, 318 mg of ethyl 4'-(4-hydroxybutoxy)-4"-pyrrolidin-1-yl-3"-trifluoromethyl[1,1';3',1"]-terphenyl-4-carboxylate are obtained (yield = 97%) in the form of a colourless oil.

¹H NMR (CDCl₃ - 400 MHz): 1.43 (t, *J*=7.5Hz, 3H); 1.55 (m, 2H); 1.68-1.73 (m, 2H); 1.93 (m, 8H); 3.42 (m, 2H); 4.08 (t, *J*=6.2Hz, 2H); 4.41 (t, *J*=7.5Hz, 2H); 4.43 (bs, 1H); 7.13 (d, *J*=8.7Hz, 1H); 7.21 (d, *J*=8.7Hz, 1H); 7.67-7.73 (m, 3H); 7.85 (d, *J*=8.2Hz, 2H); 7.87 (s, 1H); 7.99 (d, *J*=8.2Hz, 2H).

### Example 21 - 4'-(4-Hydroxybutoxy)-4"-pyrrolidin-1-yl-3"-trifluoromethyl[1,1':3',1"]-terphenyl-4-carboxylic acid

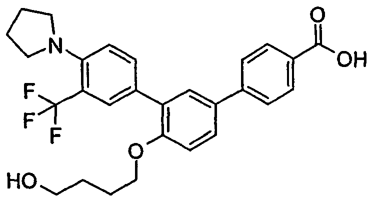

In a manner similar to that of Example 3, by reacting 318 mg of ethyl 4'-(4-hydroxybutoxy)-4"-pyrrolidin-1-yl-3"-trifluoromethyl[1,1';3',1"]terphenyl-4-carboxylate with 6 mL of 1 N sodium hydroxide solution, 75 mg of 4'-(4-hydroxybutoxy)-4"-pyrrolidin-1-yl-3"-trifluoromethyl[1,1';3',1"]terphenyl-4-carboxylic acid are obtained (yield = 25%) in the form of a white solid (m.p. = 237°C).

¹H NMR (DMSO, 400 MHz): 1.55 (m, 2H); 1.73 (m, 2H); 1.93 (m, 8H); 3.42 (m, 2H); 4.08 (t, *J*=6.2Hz, 2H); 4.43 (bs, 1H); 7.13 (d, *J*=8.7Hz, 1H); 7.21 (d, *J*=8.7Hz, 1H); 7.67-7.73 (m, 3H); 7.85 (d, *J*=8.2Hz, 2H); 7.87 (s, 1H); 7.99 (d, *J*=8.2Hz, 2H); 12.9 (bs, 1H).

### Example 22 - Ethyl 3"-tert butyl-4'-(3-hydroxypropoxyl-4"-pyrrolidin-1-yl[1,1';3',1"]-terphenyl-4-carboxylate

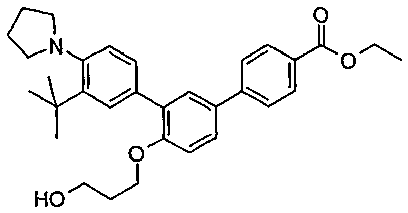

### a) Ethyl 3"-tert-butyl-4-[3-(tert-butyldimethylsilanyloxy)propoxy]-4'-pyrrolidin-1-yl-[1,1';3,1"]terphenyl-4-carboxylate

In a manner similar to that of Example 1d, by reacting 500 mg of ethyl 3'-bromo-4'-[3-(*tert-*butyldimethylsilanyloxy)propoxy]biphenyl-4-carboxylate obtained in Example 9b (1.2 mmol) with 380 mg of 3-*tert*-butyl-4-pyrrolidinophenylboronic acid (1.5 mmol) obtained in Example 17b in the presence of 1.3 mL of 2M potassium carbonate (2.6 mmol) and 35 mg of tetrakis(triphenylphosphine)palladium (Pd(PPh₃)₄), 550 mg of ethyl 3"-*tert*-butyl-4'-[3-(*tert*-butyldimethylsilanyloxy)propoxy]-4"-pyrrolidin-1-yl[1,1';3',1"]-terphenyl-4-carboxylate are obtained (yield = 89%) in the form of a yellow oil.

### b) Ethyl 3"-tert-butyl-4'-(3-hydroxypropoxy)-4"-pyrrolidin-1-yl[1,1';3',1"]terphenyl-4-carboxylate

In a manner similar to that of Example 2b, by reacting 550 mg of ethyl 3"-*tert*-butyl-4'-[3-(*tert*-butyldimethylsilanyloxy)propoxy]-4"-pyrrolidin-1-yl[1,1';3',1"]terphenyl-4-carboxylate (0.9 mmol) with 1.1 mL of a 1 N solution of tetrabutylammonium fluoride, 260 mg of ethyl 3"-*tert*-butyl-4'-(3-hydroxypropoxy)-4"-pyrrolidin-1-yl[1,1';3',1"]terphenyl-4-carboxylate are obtained (yield = 58%) in the form of a yellow oil.

¹H NMR (CDCl₃ - 400 MHz): 1.43 (t, *J*=7.5Hz, 3H); 1.47 (s, 9H); 2.05 (m, 2H); 2.10-2.25 (m, 4H); 3.03 (m, 4H); 3.81 (m, 2H); 4.17 (t, *J*=5.7Hz, 2H); 4.41 (t, *J*=7.5Hz, 2H); 7.07 (d, *J*=8.0Hz, 1H); 7.30 (s, 2H); 7.56-7.65 (m, 3H); 7.67 (d, *J*=8.2Hz, 2H); 8.10 (d, *J*=8.2Hz, 2H).

### Example 23 - 3"-tert-Butyl-4'-(3-hydroxypropoxy)-4"-pyrrolidin-1-yl[1,1';3',1"]-terphenyl-4-carboxylic acid

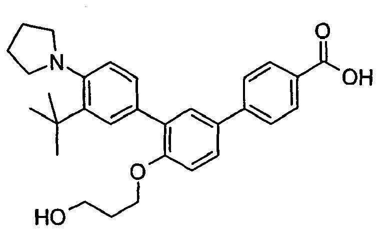

In a manner similar to that of Example 3, by reacting 260 mg of ethyl 3"-*tert*-butyl-4'-(3-hydroxypropoxy)-4"-pyrrolidin-1-yl[1,1';3',1"]terphenyl-4-carboxylate (0.5 mmol) with 5 mL of 1 N sodium hydroxide solution, 120 mg of 3"-*tert*-butyl-4'-(3-hydroxypropoxy)-4"-pyrrolidin-1-yl[1,1';3',1"]terphenyl-4-carboxylic acid are obtained (yield = 48%) in the form of a white solid (m.p. = 230°C).

**HPLC** Waters Atlantis C₁₈, 5 microns, 2 x 150 mm, mobile phase: A (CH₃CN / 0.1 v/v HCO₂H); B (H₂O / 0.1 v/v HCO₂H) Flow rate: 0.5 mL/minutes. Gradient: 0 min: 90% B, 0-20 min: 90-5% B, 20-30 min: 5% B; retention time: 13.2 min, purity: 97.8%, MS (ESI) m/z 474.3 (M+H)⁺.

### Example 24 - Ethyl 3"-tert-butyl-4'-(2-hydroxyethoxy)-4"-pyrrolidin-1-yl[1,1';3',1"]-terphenyl-4-carboxylate

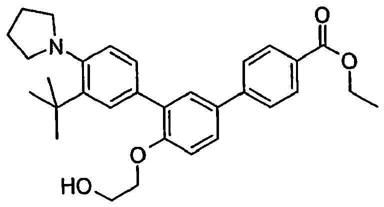

### a) Ethyl 4'-(2-acetoxyethoxy)-3"-tert-butyl-4"-pyrrolidin-1-yl[1,1';3', 1"]terphenyl-4-carboxylate

In a manner similar to that of Example 1d, by reacting 800 mg of ethyl 3'-bromo-4'-(2-acetoxyethoxy)biphenyl-4-carboxylate obtained in Example 13a (2 mmol) with 730 mg of 3-*tert*-butyl-4-pyrrolidinophenylboronic acid (2.9 mmol) obtained in Example 18b in the presence of 2.6 mL of 2M potassium carbonate (5.2 mmol) and 70 mg of tetrakis(triphenylphosphine)palladium (Pd(PPh₃)₄), 500 mg of ethyl 4'-(2-acetoxyethoxy)-3"-*tert*-butyl-4"-pyrrolidin-1-yl[1,1';3',1"]terphenyl-4-carboxylate are obtained (yield = 47%) in the form of a yellow oil.

### b) Ethyl 3"-tert-butyl-4'-(2-hydroxyethoxy)-4"-pyrrolidin-1-yl[1,1';3',1"]terphenyl-4-carboxylate

In a manner similar to that of Example 14a, by reacting 110 mg of ethyl 4'-(2-acetoxyethoxy)-3"-*tert*-butyl-4"-pyrrolidin-1-yl[1,1';3',1"]terphenyl-4-carboxylate

(0.2 mmol) with 2 mL of a 1% solution of potassium carbonate in ethanol, 40 mg of ethyl 3"-*tert-*butyl-4'-(2-hydroxyethoxy)-4"-pyrrolidin-1-yl[1,1';3',1"]terphenyl-4-carboxylate are obtained (yield = 39%) in the form of a white solid (m.p. = 193°C).

¹H NMR (DMSO, 400 MHz): 1.40 (t, 3H); 1.46 (m, 4H); 1.54 (s, 9H); 1.9 (bs, 4H); 3.0 (bs, 3H); 3.9 (bs, 2H); 4.15 (m, 2H); 4.40 (m, 2H); 7.1 (d, *J*=8.6Hz, 1H); 7.4 (d, *J*=9.9Hz, 1H); 7.45 (d, *J*=8.2Hz, 1H); 7.55 (s, 1H); 7.63 (s, 1H); 7.67 (d, *J*=7.34Hz, 1H); 7.80 (d, *J*=8.45Hz, 2H); 7.97 (d, *J*=8.4Hz, 2H).

### Example 25 - 3"-tert Butyl-4'-(2-hydroxyethoxy)-4"-pyrrolidin-1-yl[1,1':3',1"]-terphenyl-4-carboxylic acid

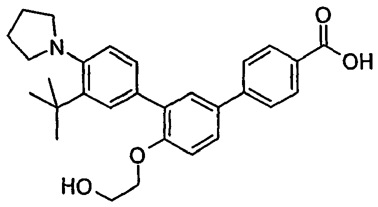

In a manner similar to that of Example 6b, by reacting 500 mg (0.9 mmol) of ethyl 4'-(2-acetoxyethoxy)-3"-*tert*-butyl-4"-pyrrolidin-1-yl[1,1';3',1"]terphenyl-4-carboxylate with 300 mg (8 mmol) of sodium hydroxide, 242 mg of 3"-*tert*-butyl-4'-(2-hydroxyethoxy)-4"-pyrrolidin-1-yl[1,1';3',1"]terphenyl-4-carboxylic acid are obtained (yield = 55 %) in the form of a white solid (m.p. = 223°C).

¹H NMR (DMSO, 400 MHz): 1.43 (s, 9H); 1.90 (m, 4H); 3.0 (m, 4H); 3.73 (d, *J*=4.7Hz, 2H); 4.1 (m, 2H); 4.7 (s, 1H); 7.2 (d, 1H, *J*=8.6Hz); 7.48 (m, 2H); 7.59 (d, *J*=1.6Hz, 1H); 7.64 (d, *J*=1.1Hz. 1H); 7.68 (dd, *J*=2Hz, 7.8Hz, 1H); 7.82 (d, *J*=8.3Hz, 2H); 7.99 (d, *J*=8.4Hz, 2H).

### Example 26 - 4'-(3-Hydroxypropoxy)-4"-pyrrolidin-1-yl(1,1';3',1"]terphenyl-4-carboxylic acid

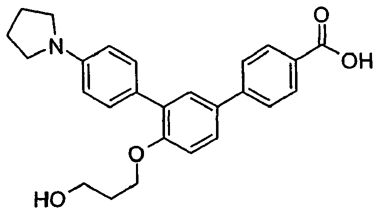

### a) 4-Pyrrolidinophenylboronic acid

In a manner similar to that of Example 1 c, by reacting 8.6 g (38 mmol) of 4-bromophenyl-1-pyrrolidine with 18 mL of a 2.5M solution of n-butyllithium and 13 mL of triisopropyl borate (57 mmol), 5 g of 4-pyrrolidinophenylboronic acid are obtained (yield = 69%) in the form of a beige-coloured solid.

### b) Ethyl 4'-[3-(tert-butyldimethylsilanyloxy)propoxy]-4"-pyrrolidin-1-yl[1,1';3;1"]terphenyl 4-carboxylate

In a manner similar to that of Example 1d, by reacting 520 mg of ethyl 3'-bromo-4'-[3-(*tert-*butyldimethylsilanyloxy)propoxy]biphenyl-4-carboxylate obtained in Example 8b (1 mmol) with 290 mg of 4-pyrrolidinophenylboronic acid (1.5 mmol) in the presence of 1.3 mL of 2M potassium carbonate (2.6 mmol) and 35 mg of tetrakis(triphenylphosphine)palladium (Pd(PPh₃)₄), 170 mg of ethyl 4'-[3-(*tert*-butyldimethylsilanyloxy)propoxy]-4"-pyrrolidin-1-yl[1,1';3',1"]terphenyl-4-carboxylate are obtained (yield = 30%) in the form of a yellow oil.

### c) Ethyl 4'-(3-hydroxypropoxy)-4"-pyrrolidin-1-yl[1,1';3',1"]terphenyl-4-carboxylate

In a manner similar to that of Example 2b, by reacting 170 mg of ethyl 4'-[3-(*tert-*butyldimethylsilanyloxy)propoxy]-4"-pyrrolidin-1-yl[1,1';3',1"]terphenyl-4-carboxylate (0.3 mmol) with 0.4 mL of a 1 N solution of tetrabutylammonium fluoride, 70 mg of ethyl 4'-(3-hydroxypropoxy)-4"-pyrrolidin-1-yl[1,1';3',1"]terphenyl-4-carboxylate are obtained (yield = 52%) in the form of a yellow oil.

### d) 4'-(3-Hydroxypropoxy)-4'-pyrrolidin-1-yl[1,1;3;1"]terphenyl-4-carboxylic acid

In a manner similar to that of Example 3, by reacting 70 mg of ethyl 4'-(3-hydroxypropoxy)-4"-pyrrolidin-1-yl[1,1';3',1"]terphenyl-4-carboxylate (0.16 mmol) with 1.6 mL of 1N sodium hydroxide solution, 10 mg of 4'-(3-hydroxypropoxy)-4"-pyrrolidin-1-yl[1,1';3',1"]terphenyl-4-carboxylic acid are obtained (yield = 15%) in the form of a white solid (m.p. = 195°C).

**HPLC** Thermo AquasilC_{18,} 3 microns, 2 x 150 mm, mobile phase: A (CH₃CN / 0.1 v/v HCO₂H) B (H₂O /0.1v/v HCO₂H), Flow rate: 0.5 mUminutes. Gradient: 0 min: 90% B, 0-20 min: 90-5% B, 20-30 min: 5% B; retention time: 14.77 min, purity: 93%, MS (ESI) m/z 418.2 (M+H)⁺.

### Example 27 3"-tert-Butyl-4"-diethylamino-4'-hydroxy[1,1';3',1"]terphenyl-4-carboxylic acid

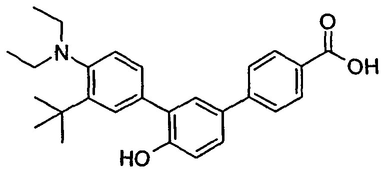

In a manner similar to that of Example 3, by reacting 300 mg of ethyl 3"-*tert*-butyl-4"-diethylamino-4'-hydroxy[1,1';3',1"]terphenyl-4-carboxylate obtained in Example 1d (0.67 mmol) with 7 mL of 1N sodium hydroxide solution, 236 mg of 3"-*tert*-butyl-4"-diethylamino-4'-hydroxy[1,1';3',1"]terphenyl-4-carboxylic acid are obtained (yield = 84%) in the form of a white solid (m.p. = 190°C).

¹H NMR (DMSO, 400 MHz): 1.06 (t, *J*=7.4Hz, 6H); 1.46 (s, 9H); 2.84 (bs, 2H); 2.94 (bs, 2H); 7.05 (d, *J*=8.5Hz, 1H); 7.31-7.47 (m, 2H); 7.54-7.62 (m, 3H); 7.78 (d, *J*=6.7Hz, 2H); 7.97 (d, *J*=6.7Hz, 2H); 9.82 (s, 1H); 12.90 (bs, 1H).

### Example 28 -Ethyl 4"-diethylamino-4'-hydroxy-3"-trifluoromethyl[1,1';3',1"]-terphenyl-4-carboxylate

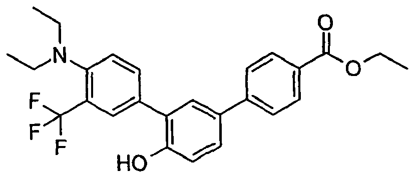

### a) (4-Bromo-2-trifluoromethylphenyl)diethylamine

In a manner similar to that of Example 1 b, by reacting 10 g of 4-bromo-2-trifluoromethylphenylamine (41 mmol) with 5 g (125 mmol) of 60% sodium hydride and 10 mL (125 mmol) of ethyl iodide, 7 g of (4-bromo-2-trifluoromethylphenyl)diethylamine are obtained (yield = 60%) in the form of a yellow oil.

### b) 4-Diethylamino-3-trifluoromethylphenylboronic acid

In a manner similar to that of Example 1c, by reacting 5 g of (4-bromo-2-trifluoromethylphenyl)diethylamine with 8.1 mL of a 2.5 M solution of n-butyllithium and 5.8 mL of triisopropyl borate, 4.3 g of 4-diethylamino-3-trifluoromethylphenylboronic acid are obtained (yield = 100%) in the form of a thick orange oil.

### c) Ethyl 4"-diethylamino-4'-hydroxy-3"-trifluoromethyl[1,1';3',1"]terphenyl-4-carboxylate

In a manner similar to that of Example 1d, by reacting 3.5 g of ethyl 3'-bromo-4'-hydroxybiphenyl-4-carboxylate (10.9 mmol) with 4.3 g of 4-diethylamino-3-trifluoromethyl-phenylboronic acid (16.4 mmol) in the presence of 14.3 mL of 2M potassium carbonate solution (28.5 mmol) and 380 mg of tetrakis(triphenylphosphine)palladium (0.3 mmol), 1.4 g of ethyl 4"-diethylamino-4'-hydroxy-3"-trifluoromethyl[1,1';3',1"]terphenyl-4-carboxylate are obtained (yield = 28%) in the form of a white solid (m.p. = 170°C).

¹H NMR (DMSO, 400 MHz): 0.97 (t, *J*=7.1 Hz, 6H); 1.44 (t, *J*=7.6H, 3H); 2.98 (q, *J*=7.1Hz, 4H); 5.49 (s, 1H); 7.09 (d, *J*=8.4 Hz, 1H); 7.60 (m, 2H); 7.69 (d, *J*=2.3Hz, 1H); 7.81 (d, *J*=8.4Hz, 2H); 7.93 (m, 2H); 7.97 d, *J*=8.4Hz, 2H).

### Example 29 - 4"-Diethylamino-4'-hydroxy-3"-trifluoromethyl[1,1';3'.1"]terphenyl-4-carboxylic acid

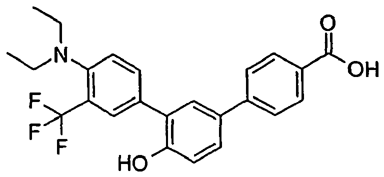

In a manner similar to that of Example 3, by reacting 200 mg of ethyl 4"-diethylamino-4'-hydroxy-3"-trifluoromethyl[1,1';3',1"]terphenyl-4-carboxylate (0.44 mmol) with 4 mL of 1N sodium hydroxide solution, 100 mg of 4"-diethylamino-4'-hydroxy-3"-trifluoromethyl-[1,1';3',1"]terphenyl-4-carboxylic acid are obtained (yield = 50%) in the form of a white solid (m.p. = 195°C).

¹H NMR (DMSO, 400 MHz): 0.97 (t, *J*=7.1 Hz, 6H); 2.98 (q, *J*=7.1 Hz, 4H); 7.09 (d, *J*=8.4 Hz, 1H); 7.60 (m, 2H); 7.69 (d, *J*=2.3Hz, 1H); 7.81 (d, *J*=8.4Hz, 2H); 7.93 (m, 2H); 7.97 d, *J*=8.4Hz, 2H); 10.05 (s, 1H); 12.90 (bs, 1H).

### Example 30 - 3"-tert-Butyl-4"-diethylamino-4'-(4-isopropylaminobutoxy)-[1,1';3',1"]-terphenyl-4-carboxylic acid

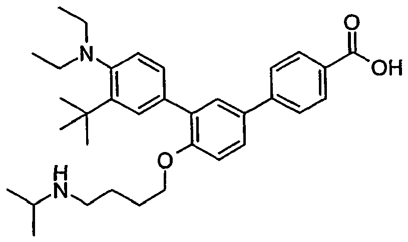

*a) Ethyl 3"-tert-butyl-4"-diethylamino-4'-(4-oxobutoxy)-[1,1';3,1"]terphenyl-4-carboxylate* 20 mL of dichloromethane placed in a three-necked flask are cooled to -78°C and 0.3 mL of oxalyl chloride are added, followed by addition of 0.54 mL of dimethyl sulfoxide diluted in 4 mL of dichloromethane. The reaction medium is stirred at -78°C for 30 minutes. 900 mg of ethyl 3"-*tert*-butyl-4"-diethylamino-4'-(4-hydroxybutoxy)-[1,1';3',1"]terphenyl-4-carboxylate obtained in Example 2b (1.7 mmol), dissolved in 55 mL of dichloromethane in the presence of 1 equivalent of triethylamine are added. The reaction medium is stirred at -78°C for 1 hour and then at room temperature for 12 hours.

The reaction is stopped by adding 100 mL of saturated ammonium chloride solution and then extracted with dichloromethane. The organic phase is washed twice with water, dried over magnesium sulfate, filtered and concentrated. The residue obtained is chromatographed on silica gel (70/30 heptane/ethyl acetate). 720 mg of ethyl 3"-*tert-*butyl-4"-diethylamino-4'-(4-oxobutoxy)-[1,1';3',1"]terphenyl-4-carboxylate are obtained (yield = 78%) in the form of a yellow oil.

### b) Ethyl 3'=tert-butyl-4"-diethylamino-4'-(4-isopropylaminobutoxy)-[1,1';3',1"]terphenyl-4-carboxylate

720 mg of aldehyde ethyl 3"-*tert*-butyl-4"-diethylamino-4'-(4-oxobutoxy)-[1,1';3',1"]-terphenyl-4-carboxylate (1.3 mmol) are dissolved in 15 mL of methanol under a nitrogen atmosphere. 0.6 mL of isopropylamine are then added. The reaction medium is stirred at room temperature for 1 hour 15 minutes. 94 mg of sodium cyanoborohydride are then added to the reaction medium with stirring. The medium is stirred for 3 days. The reaction is stopped by adding 5 mL of water and then extracted with ethyl acetate. The organic phases are combined and dried over magnesium sulfate. The solvents are evaporated off and the residue is precipitated from 8 mL of heptane and filtered through a sinter funnel. 178 mg of ethyl 3"-*tert*-butyl-4"-diethylamino-4'-(4-isopropylaminobutoxy)-[1,1';3',1"]terphenyl-4-carboxylate are obtained (yield = 24%) in the form of a white solid.

### c) 3"-tert-Butyl-4"-diethylamino-4'-(4-isopropylaminobutoxy)-[1,1';3;1"]terphenyl-4-carboxylic acid

In a manner similar to that of Example 3, by reacting 190 mg of ethyl 3"-*tert*-butyl-4"-diethylamino-4'-(4-isopropylaminobutoxy)-[1,1';3',1"]terphenyl-4-carboxylate (0.3 mmol) with 0.5 mL of 1 N sodium hydroxide solution, 45 mg of 3"-*tert*-butyl-4"-diethylamino-4'-(4-isopropylaminobutoxy)-[1,1';3',1"]terphenyl-4-carboxylic acid are obtained (yield = 25%) in the form of a white solid.

¹H NMR(CD₃OD, 400 MHz): 1.11 (t, 6H); 1.26 (d, 6H); 1.52 (s, 9H); 1.84 (m, 2H);1.90 (m, 2H); 2.98 (s, 4H); 3.2 (m, 1H); 3.3 (m, 2H); 4.13 (t, 2H); 7.19 (d, *J*=8.54Hz, 1H); 7.36 (s, 1H); 7.42 (s, 1H); 7.61 (s, 1H); 7.65 (s, 2H); 7.72 (d, *J*=8.36Hz, 2H); 8.08 (d, *J*=8.35Hz, 2H).

### Example 31 - 3"-tert-Butyl-4'-(4-isopropylaminobutoxy)-4"-pyrrolidin-1-yl[1,1';3',1"]-terphenyl-4-carboxylic acid

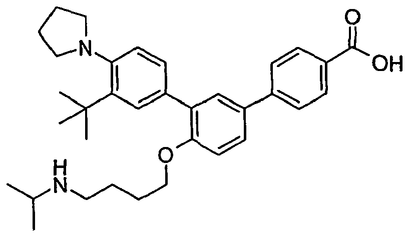

### a) Ethyl 3'-tert-butyl-4'-(4-oxobutoxy)-4"-pyrrolidin-1-yl[1,1';3;1"]terphenyl-4-carboxylate

In a manner similar to that of Example 30a, by reacting 900 mg of ethyl 3"-*tert*-butyl-4'-(4-hydroxybutoxy)-4"-pyrrolidin-1-yl[1,1';3',1"]terphenyl-4-carboxylate obtained in Example 18e with 0.3 mL of oxalyl chloride, 0.54 mL of DMSO and 0.25 mL of triethylamine, 700 mg of ethyl 3"-*tert*-butyl-4'-(4-oxobutoxy)-4"-pyrrolidin-1-yl[1,1';3',1"]terphenyl-4-carboxylate are obtained (yield = 78%) in the form of a yellow oil.

### b) Ethyl 3"-tert-butyl-4'-(4-isopropylaminobutoxy)-4"-pyrrolidin-1-yl[1,1';3',1"]terphenyl-4-carboxylate

In a manner similar to that of Example 30b, by reacting 700 g of ethyl 3"-*tert*-butyl-4'-(4-oxobutoxy)-4"-pyrrolidin-1-yl[1,1';3',1"]terphenyl-4-carboxylate with 0.6 mL of isopropylamine and 94 mg of sodium cyanoborohydride, 228 mg of ethyl 3"-*tert*-butyl-4'-(4-isopropylaminobutoxy)-4"-pyrrolidin-1-yl[1,1';3',1"]terphenyl-4-carboxylate are obtained (yield = 24%) in the form of a white solid.

### c) 3"-tert-Butyl-4'-(4-isopropylaminobutoxy)-4"-pyrrolidin-1-yl[1,1';3',1"]terphenyl-4-carboxylic acid

In a manner similar to that of Example 3, by reacting 190 mg of ethyl 3"-*tert*-butyl-4'-(4-isopropylaminobutoxy)-4"-pyrrolidin-1-yl[1,1';3',1"]terphenyl-4-carboxylate (0.3 mmol) with 2 mL of 1 N sodium hydroxide solution, 45 mg of 3"-*tert*-butyl-4"-diethylamino-4'-(4-isopropylaminobutoxy)-[1,1';3',1"]terphenyl-4-carboxylic acid are obtained (yield = 25%) in the form of a white solid.

**HPLC** Waters Atlantis C₁₈, 5 microns, 2 x 150 mm, mobile phase: A (CH₃CN / 0.05 v/v CF₃CO₂H); B (H₂O / 0.05v/v CF₃CO₂H), Flow rate: 1 mUminutes. Gradient: 0 min: 90% B, 0-20 min: 90-10% B, 20-30 min: 10% B; retention time: 17.5 min, purity: 98%, MS (ESI) m/z 541.3 (M+H)⁺.

### Example 32 - Ethyl 3"-tert-butyl-4"-diethylamino-4'-(3-hydroxypropyl)[1,1';3',1"]-terphenyl-4-carboxylate

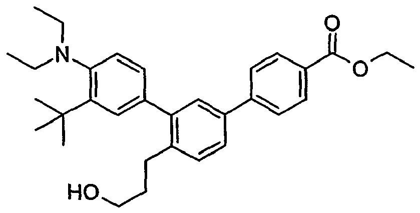

### a) Ethyl 3"-tert-butyl-4"-diethylamino-4'-trifluoromethanesulfonyloxy[1,1';3',1"]terphenyl-4-carboxylate

1.1 g of ethyl 3"-*tert*-butyl-4"-diethylamino-4'-hydroxy[1,1';3',1"]terphenyl-4-carboxylate obtained in Example 1d (2.5 mmol) are dissolved at room temperature in 22.4 mL of dichloromethane and the temperature of the reaction medium is then lowered to 0°C; 112 mg of dimethylaminopyridine are added, followed by addition of 0.88 mL of triethylamine (6 mmol) and dropwise addition of 0.5 mL of triflic anhydride (3 mmol). The temperature is raised to room temperature and the reaction medium is stirred for 20 minutes. The reaction is stopped by adding 30 mL of water and then extracted with 30 mL of dichloromethane. The organic phases are washed with 60 mL of water and then dried over sodium sulfate. The solvents are evaporated off and the residue is then purified by chromatography on silica gel (eluent: heptane). 1.2 g of ethyl 3"-*tert*-butyl-4"-diethylamino-4'-trifluoromethanesulfonyloxy[1,1';3',1 "]terphenyl-4-carboxylate are obtained (yield = 79%).

### b) Ethyl 3"-tert-butyl-4"-diethylamino-4'-vinyl[1,1';3',1"]terphenyl-4-carboxylate

1.2 g of ethyl 3"-*tert*-butyl-4"-diethylamino-4'-trifluoromethanesulfonyloxy[1,1';3',1"]-terphenyl-4-carboxylate (2 mmol) are dissolved at room temperature in 23 mL of dimethylformamide, and 250 mg of LiCl (6 mmol) and 0.74 mL of allyltributyltin (2.4 mmol) are then added. The reaction medium is heated and, at 40°C, 70 mg of dichlorobis(triphenylphosphine)palladium (0.1 mmol) are added and the reaction medium brought to 120°C and stirred for 20 minutes. The reaction is stopped by adding 30 mL of

water and then extracted with 30 mL of ethyl acetate. The organic phases are washed with 80 mL of water and then dried over magnesium sulfate. The solvents are evaporated off and the residue is then purified by chromatography on silica gel (eluent: heptane). 940 mg of ethyl 3"-*tert*-butyl-4"-diethylamino-4'-vinyl[1,1 ;3',1"]terphenyl-4-carboxylate are obtained (yield = 100%).

### c) Ethyl 3'-tert-butyl-4"-diethylamino-4'-(3-hydroxypropyl)-[1,1';3',1"]terphenyl-4-carboxylate

8 g of ethyl 3"-*tert*-butyl-4"-diethylamino-4'-vinyl[1,1';3',1"]terphenyl-4-carboxylate (17 mmol) are dissolved in 400 mL of THF with stirring and, under cold conditions (ice bath), 6.2 g of 9 borabicyclo[3.3.1]nonane (51 mmol) are added and the ice bath is removed to raise the temperature of the reaction medium to room temperature, at which point the reaction medium is stirred for 1 hour 30 minutes. The temperature of the reaction medium is again reduced to 0°C, 52.8 mL of NaOH (53 mmol) are added portionwise, and the medium is stirred for 10 minutes at 0°C; 37.3 mL of H₂O₂ (426 mmol) are then added dropwise and the reaction medium is warmed to room temperature and then stirred for 2 hours 30 minutes. The reaction is stopped by adding 500 mL of ice-water and then extracted with 500 mL of ethyl acetate. The organic phases are washed with 1 L of water and then dried over magnesium sulfate. The solvents are evaporated off and the residue is then purified by chromatography on silica gel (eluent: 7/3 heptane/ethyl acetate). 6.4 g of ethyl 3"-*tert*-butyl-4"-diethylamino-4'-(3-hydroxypropyl)-[1,1';3',1"]terphenyl-4-carboxylate are obtained (yield = 75%).

¹H NMR (CDCl₃, 400 MHz): 1.13 (t, 6H); 1.44 (t, *J*=7.5Hz, 3H); 1.50 (s, 9H); 1.75-1.78 (m, 2H), 2.76 (t, 2H); 2.88 (bs, 2H); 2.97 (bs, 2H); 3.51-3.56 (m, 2H); 4.41 (q, *J*=7.5Hz, 2H); 7.19 (m, 1H); 7.32 (d, *J*=8Hz, 1H); 7.38-7.42 (m, 2H); 7.55-7.61 (m, 2H); 7.72 (d, *J*=8.4Hz, 2H); 8.11 (d, *J*=8.4Hz, 2H). 1.09 (t, 6H); 1.5 (s, 9H); 1.7 (m, 2H), 2.75 (t, 2H); 2.90-2.99 (m, 4H); 3.5 (t, 2H); 7.19 (d, 1H, *J*=7.0Hz); 7.31 (d, 1H, *J*=8Hz); 7.42 (d, 2H, *J*=8Hz); 7.56 (s, 2H); 7.76 (d, 2H, *J*=8.4Hz); 8.17 (d, 2H, *J*=8.4Hz).

### Example 33 - 3"-tert-Butyl-4"-diethylamino-4'-(3-hydroxypropyl)-[1,1';3',1"]-terphenyl-4-carboxylic acid

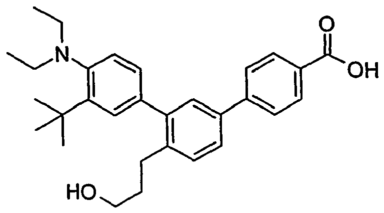

In a manner similar to that of Example 3, by reacting 70 mg of ethyl 3"-*tert*-butyl-4"-diethylamino-4'-(3-hydroxypropyl)-[1,1';3',1"]terphenyl-4-carboxylate (0.14 mmol) with 0.2 mL of 1 N sodium hydroxide solution, 64 mg of 3"-*tert*-butyl-4"-diethylamino-4'-(3-hydroxypropyl)-[1,1';3',1"]terphenyl-4-carboxylic acid are obtained (yield = 94%) in the form of a white solid (m.p. = 95°C).

¹H NMR (CDCl₃, 400 MHz): 1.09 (t, 6H); 1.5 (s, 9H); 1.7 (m, 2H), 2.75 (t, 2H); 2.90-2.99 (m, 4H); 3.5 (t, 2H); 7.19 (d, 1H, *J*=7.0Hz); 7.31 (d, 1H, *J*=8Hz); 7.42 (d, 2H, *J*=8Hz); 7.56 (s, 2H); 7.76 (d, 2H, *J*=8.4Hz); 8.17 (d, 2H, *J*=8.4Hz).

### Example 34 Ethyl 3"-tert-butyl-4"-diethylamino-4'-(2,3-dihydroxypropyl)-[1,1';3',1"]terphenyl-4-carboxylate

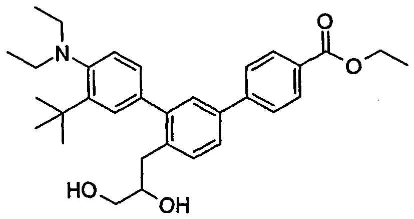

150 mg of ethyl 3"-*tert*-butyl-4"-diethylamino-4'-vinyl[1,1';3',1"]terphenyl-4-carboxylate obtained in Example 32b are dissolved in 10 mL of dichloromethane under a nitrogen atmosphere. 45 mg of N-methylmorpholine are added to the reaction medium, and 0.5 mL of a commercial 2.5% solution of osmium tetroxide in water is then added dropwise and the reaction medium is stirred at room temperature for 3 hours. The reaction is then stopped by adding 10 mL of water and extracted with dichloromethane. The organic phase is washed with water, dried over magnesium sulfate and filtered

through a sinter funnel. The solvent is evaporated off and the residue obtained is purified by chromatography on silica (eluent: 7/3 heptane/ethyl acetate). 20 mg of ethyl *3"-tert-*butyl-4"-diethylamino-4'-(2,3-dihydroxypropyl)-[1,1';3',1"]terphenyl-4-carboxylate are obtained in the form of a white solid.

¹H NMR (CDCl₃, 400 MHz): 1.12 (t, 6H); 1.43 (t, *J*=7.5Hz, 3H); 1.57 (s, 9H); 2.87 (t, 2H); 2.9 (bs, 2H); 2.97 (bs, 2H); 3.33-3.52 (m, 2H); 3.77 (m, 1H); 4.42 (q, *J*=7.5Hz, 2H); 7.18 (d, 1H, *J*=8.0Hz); 7.31 (d, *J*=8.0Hz, 1H); 7.39 (d, *J*=2Hz, 1H); 7.47 (d, *J*=7.8Hz); 7.57-7.60 (m, 2H); 7.71 (d, *J*=8.4Hz, 2H); 8.12 (d, *J*=8.4Hz, 2H).

### Example 35 - 3"-tert-Butyl-4"-diethylamino-4'-(2,3-dihydroxypropyl)-[1,1';3',1"]-terphenyl-4-carboxylic acid

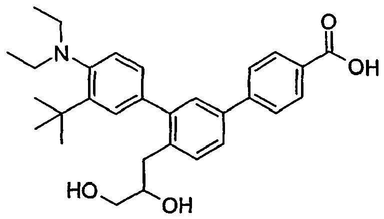

In a manner similar to that of Example 3, by reacting 70 mg of ethyl 3"-*tert*-butyl-4"-diethylamino-4'-(2,3-dihydroxypropyl)-[1,1';3',1"]terphenyl-4-carboxylate (0.14 mmol) with 0.2 mL of 1N sodium hydroxide solution, 58 mg of 3"-*tert*-butyl-4"-diethylamino-4'-(3-hydroxypropyl)-[1,1';3',1"]terphenyl-4-carboxylic acid are obtained (yield = 86%) in the form of a white solid (m.p. = 130°C).

¹H NMR (CDCl₃, 400 MHz): 1.09 (t, 6H); 1.49 (s, 9H); 2.83 (t, 2H); 2.92 (m, 4H); 3.33-3.52 (m, 2H); 3.77 (m, 1H); 7.18 (d, *J*=7.9Hz, 1H); 7.31 (d, *J*=8Hz, 1H); 7.42 (d, *J*=8Hz, 2H); 7.56 (s, 2H); 7.76 (d, *J*=8.4Hz, 2H); 8.17 (d, *J*=8.4Hz, 2H).

### Example 36 Ethyl 4"-(acetylethylamino)-3"-tert-butyl-4'-(3-hydroxypropyl)-[1,1':3'.1"]terphenyl-4-carboxylate

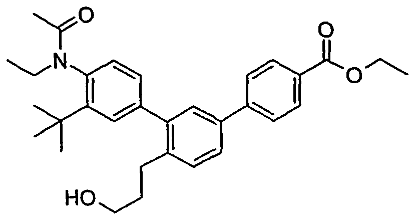

### a) Ethyl 4'-(acetylethylamino)-3"-tert-butyl-4'-trifluoromethanesulfonyloxy[1,1';3',1"]-terphenyl-4-carboxylate

In a manner similar to that of Example 32a, by reacting 7 g (58.5 mmol) of ethyl 4"-(acetylethylamino)-3"-*tert*-butyl-4'-hydroxy[1,1';3',1"]terphenyl-4-carboxylate obtained in Example 4d with 3.85 g (38 mmol, 5.3 mL) of triethylamine and 0.70 g (5.7 mmol) of 4-dimethylaminopyridine and 5.16 g (18 mmol, 3.1 mL) of triflic anhydride, 2.56 g of ethyl 4"-(acetylethylamino)-3"-*tert*-butyl-4'-trifluoromethanesulfonyloxy[1,1';3',1"]terphenyl-4-carboxylate are obtained (yield = 28%) in the form of a yellow oil.

### b) Ethyl 4'-(acetylethylamino)-4'-allyl-3"-tert-butyl[1,1';3;1"]terphenyl-4-carboxylate.

In a manner similar to that of Example 32b, by reacting 2.5 g (4.3 mmol) of ethyl 4"-(acetylethylamino)-3"-*tert*-butyl-4'-trifluoromethanesulfonyloxy[1,1';3',1"]terpheny)-4-carboxylate with 540 mg (12.7 mmol) of lithium chloride and 1.66 g (5.0 mmol) of allyltributyltin and 148 mg (0.2 mmol) of dichlorobis(triphenylphosphine)palladium, 1.67 g of ethyl 4"-(acetylethylamino)-4'-allyl-3"-*tert*-butyl[1,1';3',1"]terphenyl-4-carboxylate are obtained (yield = 80%) in the form of a yellow oil.

### c) Ethyl 4'-(acetylethylamino)-3"-tert-butyl-4'-(3-hydroxypropyl)-[1,1';3;1"]terphenyl-4-carboxylate

In a manner similar to that of Example 32c, by reacting 0.2 g (0.41 mmol) of ethyl 4"-(acetylethylamino)-4'-allyl-3"-*tert*-butyl[1,1'; 3',1"]terphenyl-4-carboxylate with 150 mg (1.24 mmol) of 9-borabicyclo[3.3.1]nonane, followed by addition of 1.25 mL (1.28 mmol) of 1 N sodium hydroxide solution and 1 g (10.3 mmol) of hydrogen peroxide, 205 mg of ethyl 4"-(acetylethylamino)-3"-*tert*-butyl-4'-(3-hydroxypropyl)-[1,1';3',1"]terphenyl-4-carboxylate are obtained (yield = 100%) in the form of a colourless oil.

¹H NMR (CDCl₃, 400 MHz): 1.28 (t, *J*=7.2 Hz, 3H); 1.43 (s, 9H); 1.46 (t, 3H); 1.78 (m, 2H); 1.88 (s, 3H); 2.76 (t, *J*=7.2 Hz, 2H); 2.94 (m, 1H); 3.57 (t, *J*=6.4 Hz, 2H); 4.43 (t, *J*=7.2Hz, 2H); 4.45 (m, 1H); 7.07 (d, *J*=8.0Hz, 1H); 7.23 (dd, J₁=2Hz, J₂=8.0 Hz, 1H); 7.45 (d, *J*=8.0 Hz, 1H); 7.52 (d, *J*=2.0 Hz, 1H); 7.56 (d, *J*=2.0 Hz, 1H); 7.62 (dd, J₁=2.0 Hz, J₂=8.0 Hz, 1H); 7.73 (d, *J*=8.4 Hz, 2H); 8.12 (d, *J*=8.4 Hz, 2H).

### Example 37 - 4"-(Acetylethylamino)-3"-tert-butyl-4'-(3-hydroxypropyl)-[1,1';3',1"]-terphenyl-4-carboxylic acid

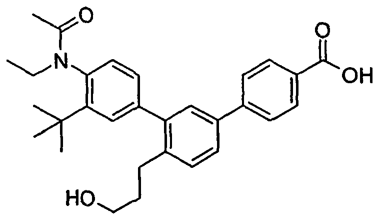

In a manner similar to that of Example 3, by reacting 200 mg of ethyl 4"-(acetylethylamino)-3"-*tert*-butyl-4'-(3-hydroxypropyl)-[1,1';3',1"]terphenyl-4-carboxylate (0.4 mmol) with 1 mL of 1 N sodium hydroxide solution, 45 mg of 4"-(acetylethylamino)-3"-*tert*-butyl-4'-(3-hydroxypropyl)-[1,1';3',1"]terphenyl-4-carboxylic acid are obtained (yield = 23%) in the form of a white solid (m.p. = 209°C).

¹H NMR (CDCl₃, 400 MHz): 1.21 (t, *J*=7.2 Hz, 3H); 1.41 (s, 9H); 1.78 (m, 2H); 1.88 (s, 3H); 2.75 (t, *J*=7.2 Hz, 2H); 2.94 (m, 1H); 3.56 (t, *J*=6.4 Hz, 2H); 4.45 (m, 1H); 7.07 (d, *J*=8.0Hz, 1H); 7.23 (dd, J₁=2Hz, J₂=8.0 Hz, 1H); 7.45 (d, *J*=8.0 Hz, 1H); 7.52 (d, *J*=2.0 Hz, 1H); 7.56 (d, *J*=2.0 Hz, 1H); 7.62 (dd, J₁=2.0 Hz, J₂=8.0 Hz, 1H); 7.73 (d, *J*=8.4 Hz, 2H); 8.18 (d, *J*=8.4 Hz, 2H).

### Example 38 Ethyl -3"-tert-butyl-4'-(3-cyclopropylaminopropyl)-4"-diethylamino [1,1';3',1"]terphenyl-4-carboxylate

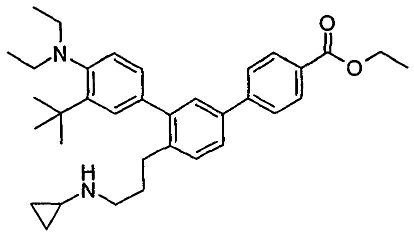

### a) Ethyl 4'-(3-bromopropyl)-3"-tert-butyl 4"-diethylamino[1,1';3;1"]terphenyl-4-carboxylate

6.4 g of ethyl 3"-*tert*-butyl-4"-diethylamino-4'-(3-hydroxypropyl)-[1,1';3',1"]terphenyl-4-carboxylate obtained in Example 32c (13 mmol) are dissolved in 128 mL of diethyl ether. The dissolution is performed under cold conditions (0°C), and 11.4mL of trioctylphosphine are then added (26 mmol) followed by addition of a solution of 8.5 g of carbon tetrabromide (26 mmol) dissolved in 10 volumes of Et₂O added dropwise. The reaction medium is stirred at 0°C for 30 minutes and then for 1 hour 30 minutes at room temperature. The reaction is stopped by adding 100 mL of water and then extracted with 100 mL of ethyl acetate. The organic phases are washed with 400 mL of water and then dried over magnesium sulfate. The solvents are evaporated off and the residue is then purified by chromatography on silica gel (eluent: 9/1 heptane/ethyl acetate). 6 g of ethyl 4'-(3-bromopropyl)-3"-*tert*-butyl-4"-diethylamino[1,1';3',1"]terphenyl-4-carboxylate are obtained (yield = 83%) in the form of a thick brown oil.

### b) Ethyl 3"-tert-butyl-4'-(3-cyclopropylaminopropyl)-4'-diethylamino[1,1'; 3;1"]terphenyl-4-carboxylate

600 mg of ethyl 4'-(3-bromopropyl)-3"-*tert*-butyl-4"-diethylamino[1,1';3',1"]terphenyl-4-carboxylate (1.1 mmol) are dissolved in 12 mL of ethanol at room temperature, and 0.76 mL of cyclopropylamine (11 mmol) are then added. The medium is brought to reflux, and stirred for 24 hours. After concentrating the reaction mixture, the residue is purified by chromatography on silica gel.

(eluent: 95/5 dichloromethane/methanol). 300 mg of ethyl 3"-*tert*-butyl-4'-(3-cyclopropylaminopropyl)-4"-diethylamino[1,1';3',1"]terphenyl-4-carboxylate are obtained (yield = 53%).

¹H NMR (CDCl₃, 400 MHz): 0.66 (d, *J*=6.7Hz, 2H); 0.95 (d, *J*=7.3Hz, 2H); 1.12 (t, *J*=7.2Hz, 6H); 1.43 (t, *J*=7.5Hz, 3H); 1.49 (s, 9H); 2.01-2.08 (m, 2H); 2.30-2.34 (m, 1H); 2.76 (t, *J*=7.7Hz, 2H); 2.83 (t, *J*=7.7Hz, 2H); 2.90 (bs, 2H); 2.98 (bs, 2H); 4.42 (q,

*J*=7.5Hz, 2H); 7.16 (m, 1H); 7.28-7.34 (m, 2H); 7.43 (d, *J*=8.0Hz, 1H); 7.52-7.56 (m, 2H); 7.68 (dd, J₁=1.9Hz, J₂=6.8Hz, 2H); 8.10 (d, J₁=1.9Hz, J₂=6.8Hz, 2H).

### Example 39 - 3"-tert-Butyl-4'-(3-cyclopropylaminopropyl)-4"-diethylamino-[1,1';3',1"]terphenyl-4-carboxylic acid

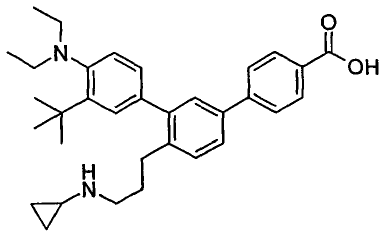

In a manner similar to that of Example 3, by reacting 300 mg of ethyl 3"-*tert*-butyl-4'-(3-cyclopropylaminopropyl)-4"-diethylamino[1,1';3',1"]terphenyl-4-carboxylate (0.57 mmol) with 2 mL of 1 N sodium hydroxide solution, 50 mg of 3"-*tert*-butyl-4'-(3-cyclopropylaminopropyl)-4"-diethylamino[1,1';3',1"]terphenyl-4-carboxylic acid are obtained (yield = 18%) in the form of a white solid (m.p. = 189°C).

¹H NMR (CD₃OD, 400 MHz): 0.62 (m, 2H); 0.72 (m, 2H); 1.14 (t, *J*=7.17 Hz, 6H); 1.52 (s, 9H); 1.83 (m, 2H); 2.42 (m, 1H); 2.81 (m, 4H); 2.92 (m, 2H); 3.05 (m, 2H); 7.22 (dd, J₁ =2.01 Hz, J₂=8.04Hz, 1 H); 7.24 - 7.44 (m, 3H); 7.49 (s, 1H); 7.61-7.67 (m, 3H); 8.03 (d, *J*=8.39 Hz, 2H).

### Example 40 - Ethyl 3"-tert-butyl-4'-(3-cyclopentylaminopropyl)-4"-diethylamino-[1,1';3',1"]terphenyl-4-carboxylate

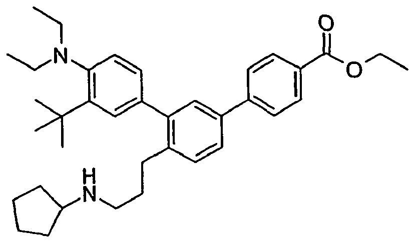

In a manner similar to that of Example 38b, by reacting 600 mg of ethyl 4'-(3-bromopropyl)-3"-*tert*-butyl-4"-diethylamino[1,1';3',1"]terphenyl-4-carboxylate (1.1 mmol) with 1.1 mL of cyclopentylamine (11 mmol), 560 mg of ethyl 3"-*tert*-butyl-4'-(3-cyclopentylaminopropyl)-4"-diethylamino[1,1';3',1"]terphenyl-4-carboxylate are obtained (yield = 93%) in the form of an orange-coloured oil.

¹H NMR (CDCl₃, 400 MHz): 1.14 (t, *J*=8.2Hz, 6H); 1.46 (t, *J*=7.5Hz, 3H); 1.49 (s, 9H); 1.52-1.54 m, 2H); 1.75 -1.81 (m, 2H); 1.91-1.98 (m, 2H); 2.07-2.13 (m, 2H); 2.73 (t, *J*=8.2Hz, 4H); 2.94 (bs, 2H); 3.0 (bs, 2H); 3.20 (m, 1H); 4.41 (q, *J*=7.5Hz, 2H); 5.2 (bs, 1H); 7.15 (m, 1H); 7.29-7.32 (m, 2H); 7.44 (d, *J*=8.0Hz, 1H); 7.51-7.55 (m, 2H); 7.67 (dd, J₁=1.8Hz, J₂=6.7Hz, 2H); 8.09 (d, J₁=1.8Hz, J₂=6.7Hz, 2H).

### Example 41 - 3"-tert-Butyl-4'-(3-cyclopentylaminopropyl)-4"-diethylamino-[1,1';3',1"]terphenyl-4-carboxylic acid

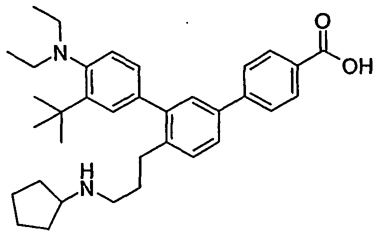

In a manner similar to that of Example 3, by reacting 300 mg of ethyl 3"-*tert*-butyl-4'-(3-cyclopentylaminopropyl)-4"-diethylamino[1,1';3',1"]terphenyl-4-carboxylate (0.57 mmol) with 2 mL of 1 N sodium hydroxide solution, 300 mg of 3"-*tert*-butyl-4'-(3-cyclopentylaminopropyl)-4"-diethylamino[1,1';3',1"]terphenyl-4-carboxylic acid are obtained (yield = 56%) in the form of a white solid (m.p. = 255°C).

¹H NMR (CD₃OD, 400 MHz): 1.14 (t, *J*=7.18Hz, 6H); 1.52 (s, 9H); 1.45 -1.95 (m, 8H); 2.03 (m, 2H); 2.82 (t, *J*=8.06Hz, 4H); 2.94 (m, 2H); 3.05 (m, 2H); 7.24 (d, *J*=2.08Hz, 2H); 7.40 (m, 2H); 7.49 (d, *J*=1.94Hz, 1H);7.62 (m, 3 H); 8.0 (d, *J*=8.4Hz, 2H).

### Example 42 - Ethyl 3"-tert-butyl-4'-(3-cyclohexylaminopropyl)-4"-diethylamino-[1,1';3';1"]terphenyl-4-carboxylate

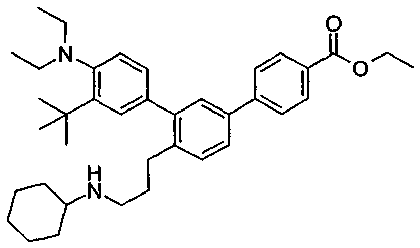

In a manner similar to that of Example 38b, by reacting 600 mg of ethyl 4'-(3-bromopropyl)-3"-*tert*-butyl-4"-diethylamino[1,1';3',1"]terphenyl-4-carboxylate (1.1 mmol) with 1.25 mL of cyclohexylamine (11 mmol), 620 mg of ethyl 3"-*tert*-butyl-4'-(3-cyclohexylaminopropyl)-4"-diethylamino[1,1';3',1"]terphenyl-4-carboxylate are obtained (yield = 100%) in the form of an orange-coloured oil.

¹H NMR (CDCl₃, 400 MHz): 1.12 (t, *J*=7.8Hz, 6H); 1.46 (t, *J*=7.5Hz, 3H); 1.49 (s, 9H); 1.52-1.54 m, 2H); 1.71 -1.81 (m, 4H); 1.91-1.98 (m, 2H); 2.00-2.05 (m, 2H); 2.71 (m, 4H); 2.90 (bs, 2H); 2.90 m, 1H); 2.97 (bs, 2H); 3.20 (m, 1H); 4.2 (bs, 1H); 4.41 (q, *J*=7.5Hz, 2H); 7.15 (m, 1H); 7.28-7.33 (m, 2H); 7.45 (d, *J*=7.8Hz, 1H); 7.52-7.56 (m, 2H); 7.67 (dd, J₁=1.8Hz, J₂=6.7Hz, 2H); 8.10 (dd, J₁=1.8Hz, J₂=6.7Hz, 2H).

### Example 43 - 3"-tert-Butyl-4'-(3-cyclohexylaminopropyl)-4"-diethylamino[1,1';3',1"]-terphenyl-4-carboxylic acid

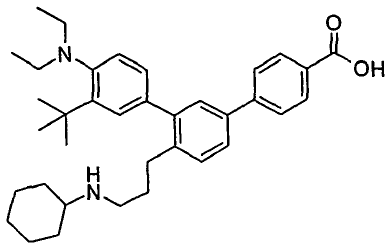

In a manner similar to that of Example 3, by reacting 600 mg of ethyl 3"-*tert*-butyl-4'-(3-cyclohexylaminopropyl)-4"-diethylamino[1,1';3',1"]terphenyl-4-carboxylate (1.2 mmol) with 3 mL of 1N sodium hydroxide solution, 400 mg of 3"-*tert*-butyl-4'-(3-cyclohexylamino-propyl)-4"-diethylamino[1,1';3',1"]terphenyl-4-carboxylic acid are obtained (yield = 56%) in the form of a white solid (m.p. = 253°C).

¹H NMR (CD₃OD, 400 MHz): 1.13 (t, *J*=7.13Hz, 6H); 1.52 (s, 9H); 1.58 - 1.93 (m, 12H); 2.45 (m, 1H); 2.52 (t, *J*=7.66Hz, 2H); 2.73 (t, *J*=7.67Hz, 2H); 2.52 (m, 2H); 3.05 (m, 2H); 7.22 (dd, *J*₁=2.02Hz, J₂=7.98Hz, 1H); 7.39 (m, 3H); 7.47 (d, *J*=1.90Hz, 1H); 7.61 (m, 3H); 8.02 (d, *J*=8.28Hz, 2H).

### Example 44 Ethyl 3"-tert-butyl-4'-(3-tert-butylaminopropyl-4"-diethylamino-[1,1':3'.1"]terphenyl-4-carboxylate

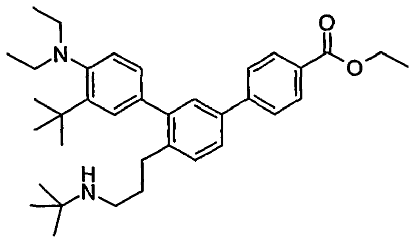

In a manner similar to that of Example 38b, by reacting 600 mg of ethyl 4'-(3-bromopropyl)-3"-*tert*-butyl-4"-diethylamino[1,1';3',1"]terphenyl-4-carboxylate (1.1 mmol) with 1.06 mL of *tert*-butylamine (11 mmol), 500 mg of ethyl 3"-*tert*-butyl-4'-(3-*tert-*butylaminopropyl)-4"-diethylamino[1,1';3',1"]terphenyl-4-carboxylate are obtained (yield = 92%) in the form of an orange-coloured oil.

¹H NMR (CDCl₃, 400 MHz): 1.12 (t, *J*=7.2Hz, 6H); 1.43 (t, *J*=7.5Hz, 3H); 1.44 (s, 9H); 1.48 (s, 9H); 2.37-2.41 (m, 2H); 2.68-2.74 (m, 4H); 2.85 (bs, 2H); 2.95 (bs, 2H); 4.40 (q, *J*=7.5Hz, 2H); 7.12 (m, 1H); 7.28-7.31 (m, 2H); 7.44 (d, *J*=8.0Hz, 1H); 7.50-7.53 (m, 2H); 7.63 (d, *J*=6.8Hz, 2H); 8.07 (d, *J*=6.8Hz, 2H); 8.88 (bs, 1H).

### Example 45- 3"-tert-Butyl-4'-(3-tert-butylaminopropyl)-4"-diethylamino[1,1':3',1"]-terphenyl-4-carboxylic acid

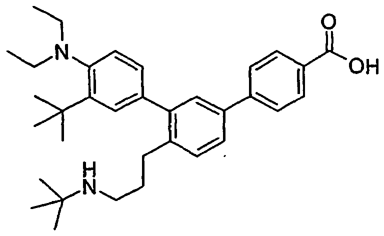

In a manner similar to that of Example 3, by reacting 480 mg of ethyl 3"-*tert*-butyl-4'-(3-*tert*-butylaminopropyl)-4"-diethylamino[1,1';3',1"]terphenyl-4-carboxylate (0.8 mmol) with 3 mL of 1N sodium hydroxide solution, 250 mg of 3"*-tert*-butyl-4'-(3-*tert-*butylaminopropyl)-4"-diethylamino[1,1';3',1"]terphenyl-4-carboxylic acid are obtained (yield = 55%) in the form of a white solid (m.p. = 273°C).

¹H NMR (CDCl₃, 400 MHz): 1.10 (t, *J*=7.2Hz, 6H); 1.41 (s, 9H); 1.46 (s.9H); 2.31 (m, 2H); 2.64 (m, 2H); 2.72 (m, 2H); 2.89 (m, 2H); 2.97 (m, 2H); 7.11 (m, 2H); 7.21 (d, *J*=8.0Hz, 1H); 7.28 (m, 2H); 7.42-7.47 (m, 3H); 7.94 (d, *J*=8.0Hz, 2H); 9.28 (bs.1H).

### Example 46 Ethyl 4"-(acetylethylamino)-3"-tert-butyl-4'-(3-cyclopropylamino-propyl]-[1,1';3',1]terphenyl-4-carboxylate

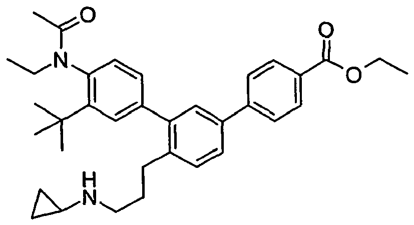

### a) Ethyl 4"-(acetylethylamino)-4'-(3-bromopropyl)-3"-tert-butyl[1,1';3',1"]terphenyl-4-carboxylate

In a manner similar to that of Example 38a, by reacting 450 mg of ethyl 4"-(acetylethylamino)-3"-*tert*-butyl-4'-(3-hydroxypropyl)-[1,1';3',1"]terphenyl-4-carboxylate (0.9 mmol) with 665 mg (1.8 mmol, 0.80 mL) of trioctylphosphine and 600 mg (1.80 mmol) of carbon tetrabromide, 500 mg of ethyl 4"-(acetylethylamino)-4'-(3-bromopropyl)-3"-*tert*-butyl[1,1';3',1"]terphenyl-4-carboxylate are obtained (yield = 98%) in the form of an orange oil.

### b) Ethyl 4"-(acetylethylamino)-3"-tert-butyl-4'-(3-cyclopropylaminopropyl)-[1,1;3',1"]-terphenyl-4-carboxylate

In a manner similar to that of Example 38b, by reacting 490 mg of ethyl 4"-(acetylethylamino)-4'-(3-bromopropyl)-3"-*tert*-butyl[1,1';3',1"]terphenyl-4-carboxylate (0.87 mmol) with 0.6 mL of cyclopropylamine (9 mmol). 270 mg of ethyl 4"-(acetylethylamino)-3"-*tert*-butyl-4'-(3-cyclopropylaminopropyl)-[1,1';3',1"]terphenyl-4-carboxylate are obtained (yield = 57%) in the form of an orange-coloured oil.

¹H NMR (CDCl₃, 400 MHz): 0.73 (m, 4H); 1.27 (t, J=6.8Hz, 3H); 1.42 (s, 9H); 1.44 (t, 3H); 1.87 (s, 3H); 1.89 (m, 2H); 2.48 (m, 1H); 2.73 (t, *J*=8.0Hz, 2H); 2.86 (t, *J*=8.0Hz, 2H); 2.97 (m, 1H); 3.89 (m, 2H); 4.42 (q, *J*=7.4Hz, 2H); 7.12 (d, *J*=8.0Hz, 1H); 7.25 (m, 1H); 7.50-7.53 (m, 3H); 7.58-7.62 (m, 1H); 7.68 (d, *J*=8.0Hz, 2H); 8.11 (d, *J*=8.0Hz, 2H).

### Example 47 4"-(Acetylethylamino)-3"-tert-butyl-4'-(3-cyclopropylaminopropyl)-[1,1';3',1"]terphenyl-4-carboxylic acid

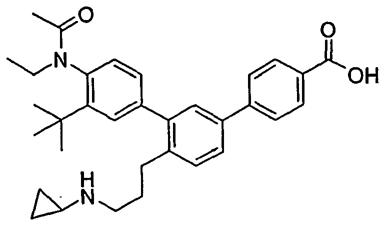

In a manner similar to that of Example 3, by reacting 260 mg of ethyl 4"-(acetylethylamino)-3"-*tert*-butyl-4'-(3-cyclopropylaminopropyl)-[1,1';3',1"]terphenyl-4-carboxylate (0.48 mmol) with 2 mL of 1N sodium hydroxide solution, 100 mg of 4"-(acetylethylamino)-3"-*tert*-butyl-4'-(3-cyclopropylaminopropyl)-[1,1';3',1"]terphenyl-4-carboxylic acid are obtained (yield = 41%) in the form of a white solid (m.p. = 190°C).

¹H NMR (CDCl₃, 400 MHz): 0.73 (m, 4H); 1.26 (t, *J*=6.8Hz, 3H); 1.42 (s, 9H); 1.87 (s, 3H); 1.89 (m, 2H); 2.48 (m, 1H); 2.73 (t, *J*=8.0Hz, 2H); 2.86 (t, *J*=8.0Hz, 2H); 2.97 (m, 1H); 4.34 (m, 1H); 7.12 (d, *J*=8.0Hz, 1H); 2.58 (dd, J₁=2.0Hz, J₂=8.0Hz, 1H); 7.35 (d, *J*=8.0Hz, 1H); 7.49 (d, *J*=2.0Hz, 1H); 7.51 (dd, J₁=2.0Hz, J₂=8.0Hz, 1H); 7.58-7.62 (m, 3H); 8.00 (d, *J*=8.0Hz, 2H).

### Example 48 - Ethyl 3"-tert-butyl-4"-diethylamino-4'-(3-isopropylaminopropyl)-[1,1';3',1"]terphenyl-4-carboxylate

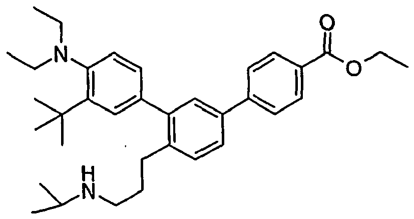

In a manner similar to that of Example 38b, by reacting 600 mg of ethyl 4'-(3-bromopropyl)-3"-*tert*-butyl-4"-diethylamino[1,1';3',1"]terphenyl-4-carboxylate (1.1 mmol) with 0.93 mL of isopropylamine (11 mmol), 353 mg of ethyl 3"-*tert*-butyl-4'-(3-isopropy)aminopropyl)-4"-diethylamino[1,1';3',1"]terphenyl-4-carboxylate are obtained (yield = 57%) in the form of a white solid.

¹H NMR (CDCl₃, 400 MHz): 1.16 (t, *J*=7.2Hz, 6H); 1.37 (d, *J*=6.8Hz, 6H); 1.42 (t, 3H); 1.48 (s, 9H); 2.20-2.25 (m, 2H); 2.75-2.79 (m, 4H); 2.93 (bs, 2H); 3.00 (bs, 2H); 3.28 m, 1H); 4.41 (q, *J*=7.5Hz, 2H); 7.15 (d, *J*=2.0Hz, 1H); 7.30 (dd, J₁=2.1Hz, J₂=8.3Hz, 2H); 7.44-7.55 (m, 3H); 7.65 (d, *J*=8.0Hz, 2H); 8.09 (d, *J*=8.0Hz, 2H); 8.98 (bs, 1H).

### Example 49 - 3"-tert-Butyl-4'-(3-isopropylaminopropyl)-4"-diethylamino[1,1';3',1"]-terphenyl-4-carboxylic acid

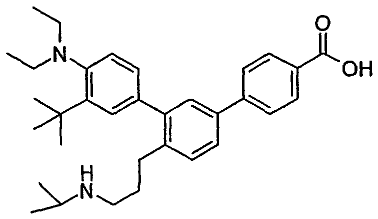

In a manner similar to that of Example 3, by reacting 350 mg of ethyl 3"-*tert*-butyl-4'-(3-isopropylaminopropyl)-4"-diethylamino[1,1';3',1"]terphenyl-4-carboxylate (0.67 mmol) with 2 mL of 1 N sodium hydroxide solution, 200 mg of 3"-*tert*-butyl-4'-(3-isopropylaminopropyl)-4"-diethylamino[1,1;3',1"]terphenyl-4-carboxylic acid are obtained (yield = 59%) in the form of a white solid (m.p. = 253°C).

¹H NMR(CD₃OD, 400 MHz): 1.23 (t, *J*=7.2Hz, 6H); 1.37 (d, *J*=6.8Hz, 6H); 1.62 (s, 9H); 1.97 (m, 2H); 2.74 (m, 1H); 2.89-2.96 (m, 4H); 3.02 (m, 2H); 3.14 (m, 2H); 7.35 (dd, J₁=2.0Hz, 1H); 7.49-7.51 (m, 2H); 7.55 (d, *J*=8.0Hz, 1H); 7.62 (d, *J*=2.0Hz, 1H); 7.75 (dd, J₁=2.0Hz, J₂=8.0Hz, 1H); 7.78 (d, *J*=8.0Hz, 2H); 8.13 (d, *J*=8.0Hz, 2H).

### Example 50 - Ethyl 3"-tert-butyl-4'-(3-aminopropyl)-4"-diethylamino[1,1';3',1"]-terphenyl-4-carboxylate

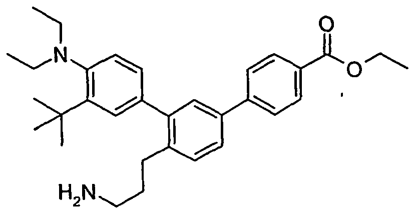

### a) Ethyl 3"-tert-butyl-4"-diethylamino-4'-[3-(1,3-dioxo-1,3-dihydroisoindol-2-yl)propyl]-[1,1',3',1"]terphenyl-4-carboxylate

In a 50 mL three-necked flask under a nitrogen atmosphere, equipped with a magnetic stirrer, 1.0 g (1.82 mmol) of ethyl 4'-(3-bromopropyl)-3"-*tert*-butyl-4"-diethylamino-[1,1';3',1"]terphenyl-4-carboxylate, 294 mg (2 mmol) of isoindole-1,3-dione and 276 mg of potassium carbonate (2 mmol) are placed in 20 mL of dimethylformamide. The reaction medium is heated at 100°C for 3 hours and is then poured into water beforehand and acidified slightly with a 1 mol/L solution of hydrochloric acid, and extracted twice with ethyl acetate. The organic phases obtained are combined and washed with water, dried over anhydrous magnesium sulfate, filtered and evaporated to give a brownish oil. This oil is purified by chromatography on silica (eluent: 80/20 heptane/ethyl acetate) to give 950 mg of ethyl 3"-*tert*-butyl-4"-diethylamino-4'-[3-(1,3-dioxo-1,3-dihydroisoindol-2-yl)propyl]-[1,1';3',1"]terphenyl-4-carboxylate (yield = 86%) in the form of a colourless oil.

### b) Ethyl 3"-tert-butyl-4'-(3-aminopropyl)-4"-diethylamino[1,1';3',1"]terphenyl-4-carboxylate In a 50 mL three-necked flask under a nitrogen atmosphere, equipped with a magnetic

stirrer, 900 mg (1.46 mmol) of ethyl 3"-*tert*-butyl-4"-diethylamino-4'-[3-(1,3-dioxo-1,3-dihydroisoindol-2-yl)propyl]-[1,1';3',1"]terphenyl-4-carboxylate and 292 mg (5.8 mmol) of hydrazine hydrate are placed in 20 mL of ethanol. The reaction medium is heated to the reflux point of the ethanol for 20 hours and then filtered; the filtrate obtained is evaporated and then purified directly on a column of silica (eluent: 94/6 dichloromethane/methanol) to give, after evaporation of the purest fractions, 545 mg of ethyl 3"-*tert*-butyl-4'-(3-aminopropyl)-4"-diethylamino[1,1';3',1"]terphenyl-4-carboxylate (yield = 77%) in the form of a colourless oil.

¹H NMR (CDCl₃, 400 MHz): 1.12 (t, *J*=7.2Hz, 6H); 1.43 (t, *J*=7.5Hz, 3H); 1.50 (s, 9H); 1.63-1.69 (m, 2H); 2.61 (t, *J*=6.9Hz, 2H); 2.70 (t, *J*=6.9 Hz, 2H); 2.90 (bs, 2H); 2.95 (bs, 2H); 4.42 (q, *J*=7.5Hz, 2H); 7.18 (m, 1H); 7.28-7.32 (m, 1H); 7.38 (d, *J*=2.0Hz, 1H); 7.40 (d, *J*=8.3 Hz, 1H); 7.55-7.59 (m, 2H); 7.71 (d, *J*=6.8Hz, 2H); 8.11 (d, *J*=6.8Hz, 2H).

### Example 51 - 3"-tert-Butyl-4'-(3-aminopropyl)-4"-diethylamino[1,1';3',1"]terphenyl-4-carboxylic acid

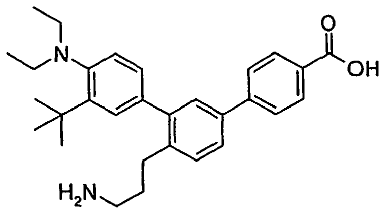

In a manner similar to that of Example 3, by reacting 545 mg of ethyl 3"-*tert*-butyl-4'-(3-aminopropyl)-4"-diethylamino[1,1';3',1"]terphenyl-4-carboxylate (1.1 mmol) with 4 mL of 1 N sodium hydroxide solution, 282 mg of 3"-*tert*-butyl-4'-(3-aminopropyl)-4"-diethylamino [1,1';3',1"]terphenyl-4-carboxylic acid are obtained (yield = 55%) in the form of a white solid (m.p. = 272°C).

¹H NMR (CDCl₃ + εCD₃COOD, 400 MHz): 1.11 (t, *J*=7.2Hz, 6H); 1.47 (s, 9H); 1.91 (m, 2H); 2.72 (t, *J*=7.2Hz, 2H); 2.84 (t, *J*=7.6Hz, 2H); 2.92 (m, 2H); 3.00 (m, 2H); 7.14 (dd, J₁=2.0Hz, J₂=8.0Hz, 1H); 7.31 (m, 2H); 7.38 (d, *J*=7.6Hz, 1H); 7.52-7.56 (m, 2H); 7.70 (d, *J*=8.4Hz, 2H); 8.12 (d, *J*=8.4Hz, 2H).

### Example 52 - [3"-tert-Butyl-4-carboxy-4'-(3-hydroxypropyl)-[1,1';3',1"]terphenyl-4"-yl]diethylamine hydrochloride

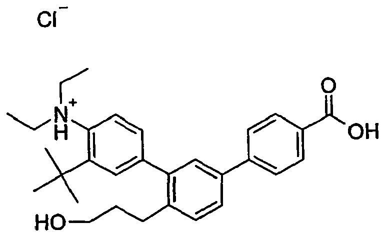

0.7 g of 3"-*tert*-butyl-4"-diethylamino-4'-(3-hydroxypropyl)-[1,1';3',1"]terphenyl-4-carboxylic acid (1.5 mmol, 1 eq) obtained in Example 33 are dissolved at room temperature in 7 mL of Et₂O (10 vol) and 7 ml of ethanol, and 0.14 mL of HCl (1.6 mmol, 1.1 eq) is then added.

The reaction mixture is then stirred for 5 hours.

Crystallization is performed in THF.

0.3 g of [3"-*tert*-butyl-4-carboxy-4'-(3-hydroxypropyl)-[1,1';3',1"]terphenyl-4"-yl]diethylamine hydrochloride is obtained (yield = 41%), (m.p. =164-166°C).

### ¹H NMR (Methanol, 400 MHz)

1.36 (m, 6H); 1.61 (s, 9H); 1.72 (m, 2H); 1.68 (m, 1H); 2.71 (m, 2H); 3.47 (t, 6.3 Hz, 2H); 3.74 (m, 1H); 3.90 (m, 2H); 3.99 (m, 2H); 7.50 (d, 8.04 Hz, 1H); 7.53 (d, 1.30 Hz, 1H); 7.58 (d, 7.8 Hz, 1H); 7.70 (m, 5.52 Hz, 2H); 7.76 (d, 8.2 Hz, 2H); 7.81 (d, 1H); 8.10 (d, 8.2 Hz, 2H).

### Example 53 - 3"-tert-Butyl-4'-(2-hydroxyethoxy)-4"-(2-oxopyrrolidin-1-yl)-[1,1';3',1"]terphenyl-4-carboxylic acid

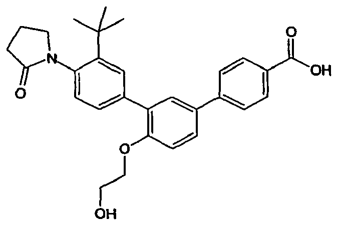

### a) N-(2-tert-Butyl-4-bromophenyl)-4-chlorobutanamide:

20 g (0.0877 mol; 1 eq.) of 2-tert-butyl-4-bromoaniline (prepared according to Example 1 a)) are dissolved in 100 ml of dichloromethane at about 0°C.

13 ml (0.0921 mol; 1.05 eq.) of triethylamine are added, followed, after 15 minutes, by addition of 10.5 ml (0.0921 mol; 1.05 eq.) of 4-chlorobutanoyl chloride.

At the end of the addition, the reaction medium is returned to room temperature and stirred for 1 hour 30 minutes. 70 ml of H₂O are added and the reaction medium is then allowed to settle. The aqueous phase is re-extracted with dichloromethane, and the organic phases are collected and washed successively with aqueous 1M NaHCO₃ and then with H₂O.

The resulting organic phase is dried over sodium sulfate, filtered and concentrated on a rotavapor.

An orange crystalline powder (m = 31 g) is obtained, which, after recristallization from heptane/ethyl acetate medium, gives 24 g of N-(2-tert-butyl-4-bromophenyl)-4-chlorobutanamide (yield = 82%).

### b) 1-(-2-tert-Butyl-4-bromophenyl)pyrrolidin-2-one:

24 g (0.0721 mol; 1 eq.) of N-(2-tert-butyl-4-bromophenyl)-4-chlorobutanamide (obtained from step a)) are suspended in 170 ml of absolute ethanol.

This suspension is cooled to about 0°C, and 60 ml (0.155 mol; 2.2 eq.) of sodium ethoxide as a 21% w/w solution in ethanol are added slowly. The reaction medium is then brownish-coloured; it is stirred for 15 hours at room temperature. 200 ml of H₂O are added and the mixture is extracted with heptane/ethyl acetate and washed with H₂O until the aqueous phase is neutral. The organic phase is concentrated on a rotavapor: 20 g of an orange crystalline powder are isolated and recrystallized from 200 ml of diisopropyl ether. Finally, 16 g of 1-(-2-tert-butyl-4-bromophenyl)pyrrolidin-2-one are obtained (yield = 75%).

c) *1-[2-tert-Butyl-4-(4,4,5,5-tetramethyl[1,3,2]dioxaborolan-2-yl)phenyl]pyrrolidin-2-one:* 6.5 g (0.022 mol; 1 eq.) of 1-(-2-tert-butyl-4-bromophenyl)pyrrolidin-2-one (obtained in step b)), 5.85 g (0.023 mol; 1.05 eq.) of bis(pinacolato)diborane and 6.46 g (0.066 mol; 3 eq.) of potassium acetate are suspended in 50 ml of dimethylformamide. After bubbling nitrogen into the reaction medium for 15 minutes, 540 mg (0.66 mmol; 0.03 eq.) of catalyst (PdCl₂(dppf)) are added and the medium is then heated at 90°C until the reaction is complete.

The reaction medium cooled to room temperature and filtered through a sinter funnel packed with Celite; the filter cake is rinsed thoroughly with ethyl acetate, and H₂O is added to the filtrate, which is then allowed to settle. The organic phase obtained is then concentrated on a rotavapor to give a residue, which is purified by chromatography on silica.

5.7 g of 1-[2-tert-butyl-4-(4,4,5,5-tetramethyl[1,3,2]dioxaborolan-2-yl)phenyl]pyrrolidin-2-one are isolated in the form of a white powder (yield = 75%).

### d) Ethyl [3'-bromo-4'-(2-hydroxyethoxy)]biphenyl-4-benzoate:

70 g (0.218 mol; 1 eq.) of ethyl [3'-bromo-4'-hydroxy]biphenyl-4-benzoate are suspended with 45.2 g (0.327 mol; 1.5 eq.) of potassium carbonate, in 700 ml of methyl ethyl ketone. 39 ml (0.545 mol; 2.5 eq.) of 2-bromoethanol are added in a single portion, and this suspension is heated to reflux. The reaction medium is maintained under these conditions for 14 hours and then cooled to room temperature.

The reaction medium is filtered and the filtrate is concentrated. The residue is added to ethyl acetate, washed with H₂O and once again concentrated. 90 g of a powder are obtained, which product is recrystallized from heptane/ethyl acetate to give after drying 65 g of ethyl [3'-bromo-4'-(2-hydroxyethoxy)]biphenyl-4-benzoate, in the form of a white crystalline powder (yield = 82%).

### e) [3'-Bromo-4'-(2-hydroxyethoxy)]biphenyl-4-carboxylic acid:

63 g (0.173 mol; 1 eq.) of ethyl [3'-bromo-4'-(2-hydroxyethoxy)]biphenyl-4-benzoate (obtained in step d)) are dissolved in 300 ml of tetrahydrofuran. 10.9 g (0.259 mol; 1.5 eq.) of lithium hydroxide monohydrate as a solution in 70 ml of H₂O are added at room temperature.

The reaction medium is refluxed for about 1 hour 30 minutes.

The reaction medium is then cooled to room temperature and dilute hydrochloric acid solution is added (330 ml; ~1M).

100 ml of H₂O are added and this suspension is cooled to about 0°C; it is maintained at this temperature for about 15 minutes then filtered. After drying, 59 g of a white powder are obtained.

This product is slurried in 240 ml of acetone for two hours at room temperature and then filtered and oven-dried. 55 g of [3'-bromo-4'-(2-hydroxyethoxy)]biphenyl-4-carboxylic acid are thus isolated (yield = 95%).

### f) 3"-tert-Butyl-4'-(2-hydroxyethoxy)-4'-(2-oxopyrrolidin-9 yl)-[1,1',3';1"]terphenyl-4-carboxylic acid:

1.3 g (3.78 mmol; 1 eq.) of 1-[2-tert-butyl-4-(4,4,5,5-tetramethyl[1,3,2]dioxaborolan-2-yl)phenyl]pyrrolidin-2-one (obtained in step c)) and 1.68 g (4.98 mmol; 1.3 eq.) of [3'-bromo-4'-(2-hydroxyethoxy)]biphenyl-4-carboxylic acid (obtained in step e)) are dissolved in 8 ml of dimethylformamide. 7.5 ml (0.015 mol; 3.97 eq.) of aqueous 2M potassium carbonate solution and 35 mg (0.10 mmol; 0.026 eq.) of 2-dicyclohexylphosphinobiphenyl are then added.

A stream of nitrogen is bubbled through the medium for about 10 minutes, and 11 mg (0.08 mmol; 0.014 eq.) of palladium acetate are introduced. The mixture is then heated to about 90°C and maintained under these conditions for 4-6 hours.

The reaction medium is then cooled to room temperature, filtered through a sinter funnel packed with Celite and rinsed with a minimum amount of dimethylformamide, and dilute hydrochloric acid solution (-2M) is added to the filtrate.

After stirring for 4 hours, the precipitate formed is filtered off, rinsed to neutrality with H₂O, filtered off by suction and oven-dried. 1.05 g of 3"-tert-butyl-4'-(2-hydroxyethoxy)-4"-(2-oxopyrrolidin-1-yl)-[1,1',3',1"]terphenyl-4-carboxylic acid are thus isolated (yield = 57%).

### (Example 54-3"-tert-Butyl-4"-ethylamino-4'-(3-hydroxypropoxy)-[1,1';3',1"]ter-phenyl-4- carboxylic acid

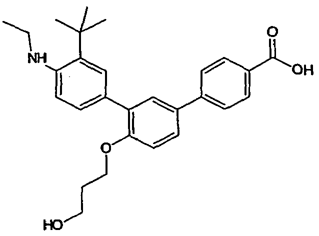

### a) N-(2-tert-Butyl-4-bromophenyl)acetamide:

18 g (0.079 mol; 1 eq.) of 2-tert-butyl-4-bromoaniline (prepared according to Example 1 a)) are dissolved in 150 ml of dichloromethane at about 0°C. 12.1 ml (0.087 mol; 1.1 eq.) of triethylamine are added, followed, after 15 minutes, by addition of 6.2 ml (0.087 mol; 1.1 eq.) of acetyl chloride. The mixture is warmed to room temperature at the end of addition and stirred for 2 hours. 70 ml of H₂O are added and the resulting mixture is allowed to settle. The aqueous phase is re-extracted with dichloromethane, and the organic phases are combined, washed to neutrality with H₂O, dried over sodium sulfate, filtered and concentrated on a rotavapor.

21 g of a beige-coloured crystalline powder are obtained, which product is slurried in heptane for 2 hours at room temperature, chilled and then filtered. After drying, 17.6 g of N-(2-tert-butyl-4-bromophenyl)acetamide are obtained (yield = 83%).

### b) (2-tert-Butyl-4-bromophenyl)ethylamine:

17.5 g (0.065 mol; 1 eq.) of N-(2-tert-butyl-4-bromophenyl)acetamide (obtained in step a)) are suspended in 100 ml of tetrahydrofuran.

162 ml (0.162 mol; 2.5 eq.) of 1 M borane-tetrahydrofuran complex are added and the mixture is heated to reflux. The conditions are maintained for about 12 hours. The reaction mixture is cooled to room temperature and 70 ml of methanol are added to destroy the excess borane. The mixture is stirred until the evolution of gas has ceased and is then concentrated on a rotavapor. The oil obtained is dissolved in ethyl acetate and washed with saturated aqueous ammonium chloride solution and then with H₂O. The resulting solution is concentrated and purified by filtration on silica; 13 g of (2-tert-butyl-4-bromophenyl)ethylamine are thus obtained in the form of a relatively colourless oil (yield = 78%).

### c) [2-tert-Butyl-4-(4,4,5,5-tetramethyl[1,3,2]dioxaborolan-2-yl)phenyl]ethylamine:

13 g (0.0507 mol; 1 eq.) of (2-tert-butyl-4-bromophenyl)ethylamine (obtained in step b)), 15.5 g (0.061 mol; 1.2 eq.) of bis(pinacolato)diborane and 15 g (0.152 mol; 3 eq.) of potassium acetate are suspended in 75 ml of dimethylformamide. After bubbling a stream of nitrogen through the reaction medium for 15 minutes, 1.66 g (2.03 mmol; 0.04 eq.) of catalyst (PdCl₂(dppf)) are added and the medium is heated at about 90°C for about 10 hours.

The reaction medium is cooled to room temperature and filtered through a sinter funnel packed with Celite; the filter cake is rinsed thoroughly with ethyl acetate, and H₂O is added to the filtrate and the phases are allowed to separate by settling. The organic phase obtained is thus concentrated on a rotavapor to give a residue, which is chromatographed on a column of silica. 7 g of [2-tert-butyl-4-(4,4,5,5-tetramethyl[1,3,2]-dioxaborolan-2-yl)phenyl]ethylamine are isolated in the form of an orange crystalline powder (yield = 45%).

### d) Ethyl [3'-bromo-4'-(3-hydroxypropoxy)]biphenyl-4-benzoate:

47 g (0.146 mol; 1 eq.) of ethyl [3'-bromo-4'-hydroxy]biphenyl-4-benzoate are suspended with 30.3 g (0.219 mol; 1.5 eq.) of potassium carbonate in 470 ml of methyl ethyl ketone. 13.5 ml (0.154 mol; 1.05 eq.) of 3-bromopropan-1-ol are added in a single portion and this suspension is brought to reflux. These conditions are maintained for 14 hours and the mixture is then cooled to room temperature. The medium is filtered and the filtrate is concentrated. The residue is taken up in ethyl acetate, washed with H₂O and then concentrated again. 60 g of a powder are isolated, and are recrystallized from a heptane/ethyl acetate mixture to give, after drying, 40 g of ethyl [3'-bromo-4'-(3-hydroxypropoxy)]biphenyl-4-benzoate in the form of an off-white crystalline powder (yield = 72%).

### e) Ethyl [3'-bromo-4'-(3-acetoxypropoxy)]biphenyl-4-benzoate:

2 g (5.27 mmol; 1 eq.) of ethyl [3'-bromo-4'-(3-hydroxypropoxy)]biphenyl-4-benzoate (obtained in step d)) are dissolved in 20 ml of dichloromethane. 64 mg (0.527 mmol; 0.1 eq.) of 4-dimethylaminopyridine and 430 µl (5.27 mmol; 1 eq.) of pyridine are added at room temperature. The mixture is stirred for 15 minutes at room temperature, and 750 µl (7.91 mmol; 1.5 eq.) of acetic anhydride are then added. The mixture is maintained at room temperature for 1 hour. H₂O is added, the phases are allowed to separate by settling and the organic phase is neutralized with aqueous 1M NHCO₃ solution. The resulting organic phase is washed with H₂O until neutral and concentrated on a rotavapor. 2.2 g of a powder are obtained, and are recrystallized from a heptane/ethyl acetate mixture. This gives, after drying, 1.9 g of ethyl [3'-bromo-4'-(3-acetoxypropoxy)]-biphenyl-4-benzoate in the form of a white crystalline powder (yield = 85%).

### f) Ethyl 4'-(3-acetoxypropoxy)-3"-tert-butyl-4"-ethylamino[1,1',3',1"]terphenyl-4-benzoate:

720 mg (2.3 mmol; 1 eq.) of [2-tert-butyl-4-(4,4,5,5-tetramethyl[1,3,2]dioxaborolan-2-yl)-phenyl]ethylamine (obtained in step c)) and 1 g (2.3 mmol; 1 eq.) of ethyl [3'-bromo-4'-(3-acetoxypropoxy)]biphenyl-4-benzoate (obtained in step e)) are dissolved in 10 ml of dimethylformamide. 2.5 ml (5 mmol; 2 eq.) of aqueous 2M tribasic potassium phosphate solution and 16.1 mg (0.046 mmol; 0.02 eq.) of 2-dicyclohexylphosphinobiphenyl are added at room temperature.

After bubbling a stream of nitrogen into the medium for about 10 minutes, 5.2 mg (0.023 mmol; 0.01 eq.) of palladium acetate are added and the mixture is heated at 80°C for about 5 hours. The reaction medium is filtered through a sinter funnel packed with Celite and rinsed thoroughly with ethyl acetate. Saturated aqueous ammonium chloride solution is then added to the filtrate, the phases are allowed to separate by settling and the organic phase is washed with H₂O and then dried over sodium sulfate. The organic phase is concentrated; the oil obtained is chromatographed on a column of silica, and 900 mg of ethyl 4'-(3-acetoxypropoxy)-3"-tert-butyl-4"-ethylamino[1,1',3',1"]terphenyl-4-benzoate are isolated (yield = 73%).

### g) 3"-tert-Butyl-4"-ethylamino-4'-(3-hydroxypropoxy)-[1,1',3',1"]terphenyl-4-carboxylic

### acid:

900 mg (1.74 mmol; 1 eq.) of ethyl 4'-(3-acetoxypropoxy)-3"-tert-butyl-4"-ethylamino-[1,1',3',1"]terphenyl-4-benzoate are dissolved in 10 ml of absolute ethanol. 312 mg (7.8 mmol; 4.5 eq.) of sodium hydroxide and 4 ml of H₂O are added at room temperature and the mixture is then heated to reflux. These conditions are maintained for about 1 hour 30 minutes. The reaction medium is concentrated to a small volume; H₂O is added to the precipitate formed, and the mixture is then acidified with acetic acid to pH ∼4-5. This suspension is fluidized by adding H₂O and stirred at room temperature for 1 hour. The resulting mixture is filtered through a sinter funnel, rinsed with H₂O until the filtrate is neutral, and oven-dried. 694 mg of 3"-tert-butyl-4"-ethylamino-4'-(3-hydroxypropoxy)-[1,1',3',1"]terphenyl-4-carboxylic acid are thus obtained in the form of a white powder (yield = 89%).

### EXAMPLE 55: TRANSACTIVATION TEST

The activation of receptors with an agonist (activator) in HeLa cells leads to the expression of a reporter gene, luciferase, which, in the presence of a substrate, generates light. The activation of the receptors may thus be measured by quantifying the luminescence produced after incubating the cells in the presence of a reference agonist. The inhibitory products displace the agonist from its site, thus preventing activation of the receptor. The activity is measured by quantifying the reduction in light produced. This measurement makes it possible to determine the inhibitory activity of the compounds according to the invention.

In this study, a constant is determined which represents the affinity of the molecule for the receptor. Since this value can fluctuate depending on the basal activity and the expression of the receptor, it is referred to as the Kd apparent (KdApp).

To determine this constant, "crossed curves" of the test product against a reference agonist, 4-[2-(5,5,8,8-tetramethyl-5,6,7,8-tetrahydro-2-naphthyl)propenyl]benzoic acid, are performed in 96-well plates. The test product is used at 10 concentrations and the reference agonist at 7 concentrations. In each well, the cells are in contact with a concentration of the test product and a concentration of the reference agonist, 4-[2-(5,5,8,8-tetramethyl-5,6,7,8-tetrahydro-2-naphthyl)propenyl]benzoic acid. Measurements are also taken for the total agonist (4-[2-(5,5,8,8-tetramethyl-5,6,7,8-tetrahydro-2-naphthyl)propenyl]benzoic acid) and inverse agonist, 4-{(E)-3-[4-(4-tert-butylphenyl)-5,5,8,8-tetramethyl-5,6,7,8-tetrahydro-2-naphthyl]-3-oxopropenyl}benzoic acid, controls.

These crossed curves make it possible to determine the AC₅₀ values (concentration at which 50% activation is observed) for the reference ligand at various concentrations of test product. These AC₅₀ values are used to calculate the Schild regression by plotting a straight line corresponding to the Schild equation *("*quantitation in receptor pharmacology" Terry P. Kenakin, Receptors and Channels, 2001, 7, 371-385*).*

The HeLa cell lines used are stable transfectants containing the plasmids ERE-βGlob-Luc-SV-Neo (reporter gene) and RAR (α, β, γ) ER-DBD-puro. These cells are inoculated into 96-well plates at a rate of 10 000 cells per well in 100 µl of DMEM medium without phenol red, and supplemented with 10% defatted calf serum. The plates are then incubated at 37°C and 7% CO₂ for 4 hours.

The various dilutions of the test products, of the reference ligand (4-[2-(5,5,8,8-tetramethyl-5,6,7,8-tetrahydro-2-naphthyl)propenyl]benzoic acid), of the 100% control (100 nM 4-[2-(5,5,8,8-tetramethyl-5,6,7,8-tetrahydro-2-naphthyl)propenyl]benzoic acid) and of the 0% control (500 nM 4-{(E)-3-[4-(4-*tert*-butylphenyl)-5,5,8,8-tetramethyl-5,6,7,8-tetrahydro-2-naphthyl]-3-oxopropenyl}benzoic acid) are added at a rate of 5 µl per well. The plates are then incubated for 18 hours at 37°C and 7% CO₂.

The culture medium is removed by turning over and 100 µl of a 1:1 PBS/luciferine mixture is added to each well. After 5 minutes, the plates are read using the luminescence reader.

| | **RARalpha Kdapp(nM)** | **RARbeta Kdapp (nM)** | **RARgamma Kdapp (nM)** |
|---|---|---|---|
| **Compound of Example 3** | 30 | 8 | 2 |
| **Compound of Example 5** | 8 | 8 | 0.5 |
| **Compound of Example 6** | 60 | 8 | 0.25 |
| **Compound of Example 8** | 60 | 4 | 0.12 |
| **Compound of Example 12** | 250 | 4 | 2 |
| **Compound of Example 13** | 500 | 4 | 0.5 |
| **Compound of Example 17** | 250 | 8 | 30 |
| **Compound of Example 19** | 60 | 4 | 1 |
| **Compound of Example 21** | 4 | 4 | 30 |
| **Compound of Example 23** | 60 | 8 | 2 |
| **Compound of Example 25** | 500 | 15 | 2 |
| **Compound of Example 33** | 60 | 8 | 0.5 |
| **Compound of Example 35** | 4000 | 500 | 8 |
| **Compound of Example 37** | 250 | 120 | 30 |
| **Compound of Example 47** | 120 | 250 | 120 |
| **Compound of Example 52** | 30 | 2 | 0.25 |
| **Compound of Example 53** | 2000 | 8000 | 60 |
| **Compound of Example 54** | 8 | 4 | 0.25 |

The results obtained with the compounds according to the invention clearly show Kdapp values of less than or equal to 1000 nM.

### EXAMPLE 56: FORMULATION EXAMPLES

This example illustrates various concrete formulations based on the compounds according to the invention.

### A - ORAL ROUTE

(a) 0.2 g tablet
   - Compound of Example 5 0.001 g
   - Starch 0.114 g
   - Dicalcium phosphate 0.020 g
   - Silica 0.020 g
   - Lactose 0.030 g
   - Talc 0.010 g
   - Magnesium stearate 0.005 g
(b) Drinkable suspension in 5 ml ampoules
   - Compound of Example 3 0.001 g
   - Glycerol 0.500 g
   - 70% sorbitol 0.500 g
   - Sodium saccharinate 0.010 g
   - Methyl para-hydroxybenzoate 0.040 g
   - Flavouring qs
   - Purified water qs 5 ml
(c) 0.8 g tablet
   - Compound of Example 4 0.500 g
   - Pregelatinized starch 0.100 g
   - Microcrystalline cellulose 0.115 g
   - Lactose 0.075 g
   - Magnesium stearate 0.010 g
(d) Drinkable suspension in 10 ml ampoules
   - Compound of Example 2 0.200 g
   - Glycerol 1.000 g
   - 70% sorbitol 1.000 g
   - Sodium saccharinate 0.010 g
   - Methyl para-hydroxybenzoate 0.080 g
   - Flavouring qs
   - Purified water qs 10 ml

### B - PARENTERAL ROUTE

(a) Composition
   - Compound of Example 3 0.002 g
   - Ethyl oleate qs 10 g
(b) Composition
   - Compound of Example 1 0.05%
   - Polyethylene glycol 20%
   - 0.9% NaCl solution qs 100
(c) Composition
   - Compound of Example 3 2.5%
   - Polyethylene glycol 400 20%
   - 0.9% NaCl solution qs 100
(d) Injectable cyclodextrin composition
   - Compound of Example 3 0.1 mg
   - β-Cyclodextrin 0.10 g
   - Water for injection qs 10.00 g

### C -TOPICAL ROUTE

(a) Ointment
   - Compound of Example 2 0.020 g
   - Isopropyl myristate 81.700 g
   - Liquid petroleum jelly oil 9.100 g
   - Silica ("Aerosil 200" sold by Degussa) 9.180 g
(b) Ointment
   - Compound of Example 5 0.300 g
   - White petroleum jelly codex qs 100 g
(c) Nonionic water-in-oil cream
   - Compound of Example 4 0.100 g
   - Mixture of emulsifying lanolin alcohols, waxes and oils ("Anhydrous Eucerin" sold by BDF) 39.900 g
   - Methyl para-hydroxybenzoate 0.075 g
   - Propyl para-hydroxybenzoate 0.075 g
   - Sterile demineralized water qs 100 g
(d) Lotion
   - Compound of Example 2 0.100 g
   - Polyethylene glycol (PEG 400) 69.900 g
   - 95% ethanol 30.000 g
(e) Hydrophobic ointment
   - Compound of Example 4 0.300 g
   - Isopropyl myristate 36.400 g
   - Silicone oil ("Rhodorsil 47 V 300" sold by Rhône-Poulenc) 36.400 g
   - Beeswax 13.600 g
   - Silicone oil ("Abil 300 000 cSt" sold by Goldschmidt) qs 100 g
(f) Nonionic oil-in-water cream
   - Compound of Example 5 1.000 g
   - Cetyl alcohol 4.000 g
   - Glyceryl monostearate 2.500 g
   - PEG 50 stearate 2.500 g
   - Shea butter 9.200 g
   - Propylene glycol 2.000 g
   - Methyl para-hydroxybenzoate 0.075 g
   - Propyl para-hydroxybenzoate 0.075 g
   - Sterile demineralized water qs 100 g

## Claims

1. Compounds **characterized in that** they correspond to formula (I) below: 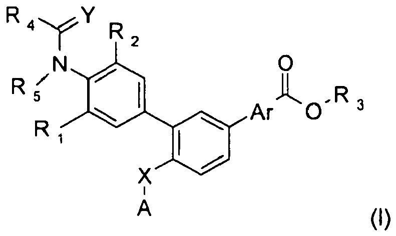 in which:
- R₁ is a hydrogen atom, an alkyl radical of 1 to 4 carbon atoms or a -CF₃ radical;
- R₂ is a hydrogen atom, an alkyl or alkoxy radical of 1 to 4 carbon atoms or a chlorine atom;
- R₃ is a hydrogen atom or a linear or branched alkyl radical of 1 to 10 carbon atoms optionally substituted with a methoxy group, or a linear or branched alkyl radical of 1 to 10 carbon atoms containing an ether function;
- R₄ is a hydrogen atom or an alkyl radical of 1 to 3 carbon atoms;
- R₅ is a hydrogen atom or an alkyl radical of 1 to 3 carbon atoms;
- or alternatively R₄ and R₅ form, together with the bond -N-C(=Y)-, a ring of pyrrolidine, pyrrolidinone, piperidine or piperidinone type;
- Y represents two hydrogen atoms or a hetero atom, for instance oxygen or sulfur;
- Ar represents a 1,4-phenyl, 2,5-pyridyl, 5,2-pyridyl or 2,5-thiophenyl ring;
- X represents an oxygen atom optionally substituted with an alkyl or alkylamine chain or a C-C single bond;
- A represents a hydrogen atom or the following formula: 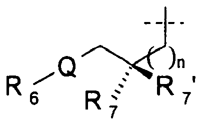 in which,
o Q is an oxygen atom or an -NH- bond;
o R₆ represents a hydrogen atom, an alkyl radical of 1 to 6 carbon atoms, a cycloalkyl radical of 3 to 6 carbon atoms or a -C(O)CH₃ or -C(O)CH₂CH₃ radical;
o R₇ and R₇' represent, independently of each other, a hydrogen atom or a hydroxyl group, on condition that R₇ and R₇' are not simultaneously a hydroxyl group;
o n is 0, 1, 2, 3, 4 or 5;
and the salts of the compounds of formula (I) when R₃ represents a hydrogen atom, OH and also the geometrical isomers of the said compounds of formula (I).

2. Compounds according to Claim 1, **characterized in that** they are in the form of alkali metal or alkaline-earth metal salts, zinc salts or salts of an organic amine or of an acidic partner when the compound is itself basic.

3. Compound according to Claim 1, **characterized in that** the alkyl radical of 1 to 3 carbon atoms is chosen from methyl, ethyl, i-propyl and n-propyl radicals.

4. Compound according to Claim 1, **characterized in that** the alkyl radical of 1 to 4 carbon atoms is chosen from methyl, ethyl, i-propyl, i-butyl and t-butyl radicals.

5. Compound according to Claim 1, **characterized in that** the alkyl radical of 1 to 10 carbon atoms is chosen from methyl, ethyl, propyl, i-propyl, butyl, i-butyl, t-butyl, pentyl, hexyl, heptyl, octyl, nonyl and dodecyl radicals.

6. Compound according to Claim 1, **characterized in that** the alkoxy radical containing from 1 to 4 carbon atoms is chosen from methoxy, ethoxy, isopropyloxy and tert-butoxy radicals.

7. Compound according to Claim 1, **characterized in that** the cycloalkyl radical of 3 to 5 carbon atoms is chosen from cyclopropyl, cyclopentyl and cyclohexyl radicals.

8. Compounds of formula (I) according to any one of the preceding claims, chosen from:
1. ethyl 3"-*tert*-butyl-4"-diethylamino-4'-hydroxy[1,1';3',1 "]terphenyl-4-carboxylate
2. ethyl 3"-*tert*-butyl-4"-diethylamino-4'-(4-hydroxybutoxy)-[1,1';3',1"]terphenyl-4-carboxylate
3. 3"-*tert*-butyl-4"-diethylamino-4'-(4-hydroxybutoxy)-[1,1';3',1"]terphenyl-4-carboxylic acid
4. ethyl 4"-(acetylethylamino)-3"-*tert*-butyl-4'-(4-hydroxybutoxy)-[1,1';3',1"]terphenyl-4-carboxylate
5. 4"-(acetytethylamino)-3"-*tert*-butyl-4'-(4-hydroxybutoxy)-[1,1';3',1"]terpheny)-4-carboxylic acid
6. 3"-*tert*-butyl-4"-diethylamino-4'-(2-hydroxyethoxy)-[1,1'; 3',1 "]terphenyl-4-carboxylic acid
7. ethyl 3"-*tert*-butyl-4"-diethylamino-4'-(3-hydroxypropoxy)-[1,1';3',1"]terphenyl-4-carboxylate
8. 3"-*tert*-butyl-4"-diethylamino-4'-(3-hydroxypropoxy)-[1,1';3',1"]terphenyl-4-carboxylic acid
9. ethyl 4"-diethylamino-4'-(3-hydroxypropoxy)-[1,1';3',1"]terphenyl-4-carboxylate
10. 4"-diethylamino-4'-(3-hydroxypropoxy)-[1,1';3',1"]terphenyl-4-carboxylic acid
11. ethyl 4"-diethylamino-3"-ethyl-4'-(3-hydroxypropoxy)-[1,1'; 3',1"]terphenyl-4-carboxylate
12. 4"-diethylamino-3"-ethyl-4'-(3-hydroxypropoxy)-[1,1';3',1"]terphenyl-4-carboxylic acid
13. 4"-diethylamino-3"-ethyl-4'-(2-hydroxyethoxy)-[1,1';3',1"]terphenyl-4-carboxylic acid
14. ethyl 4"-diethylamino-3"-ethyl-4'-(2-hydroxyethoxy)-[1,1'; 3',1"]terphenyl-4-carboxylate
15. ethyl 3"-*tert*-butyl-4"-diethylamino-4'-(2-hydroxyethoxy)-[1,1';3',1"]terphenyl-4-carboxylate
16. ethyl 4"-diethylamino-4'-(3-hydroxypropoxy)-3"-methyl[1,1';3',1"]terphenyl-4-carboxylate
17. 4"-diethylamino-4'-(3-hydroxypropoxy)-3"-methyl[1,1';3',1"]terphenyl-4-carboxylic acid
18. ethyl 3"-*tert*-butyl-4'-(4-hydroxybutoxy)-4"-pyrrolidin-1-yl[1,1';3',1"]terphenyl-4-carboxylate
19. 3"-*tert*-butyl-4'-(4-hydroxybutoxy)-4"-pyrrolidin-1-yl[1,1';3',1"]terphenyl-4-carboxylic acid
20. ethyl 4'-(4-hydroxybutoxy)-4"-pyrrolidin-1-yl-3"-trifluoromethyl[1,1';3',1"]terphenyl-4-carboxylate
21. 4'-(4-hydroxybutoxy)-4"-pyrrolidin-1-yl-3"-trifluoromethyl[1,1';3',1"]terphenyl-4-carboxylic acid
22. ethyl 3"-*tert*-butyl-4'-(3-hydroxypropoxy)-4"-pyrrolidin-1-yl[1,1';3',1"]terphenyl-4-carboxylate
23. 3"-*tert*-butyl-4'-(3-hydroxypropoxy)-4"-pyrrolidin-1-yl[1,1';3',1"]terphenyl-4-carboxylic acid
24. ethyl 3"-*tert*-butyl-4'-(2-hydroxyethoxy)-4"-pyrrolidin-1-yl[1,1';3',1"]terphenyl-4-carboxylate
25. 3"-*tert*-butyl-4'-(2-hydroxyethoxy)-4"-pyrrolidin-1-yl[1,1';3',1"]terphenyl-4-carboxylic acid
26. 4'-(3-hydroxypropoxy)-4"-pyrrolidin-1-yl[1,1';3',1"]terphenyl-4-carboxylic acid
27. 3"-*tert*-butyl-4"-diethylamino-4'-hydroxy[1,1'; 3',1"]terphenyl-4-carboxylic acid
28. ethyl 4"-diethylamino-4'-hydroxy-3"-trifluoromethyl[1,1';3',1"]terphenyl-4-carboxylate
29. 4"-diethylamino-4'-hydroxy-3"-trifluoromethyl[1,1';3',1"]terphenyl-4-carboxylic acid
30. 3"-*tert*-butyl-4"-diethylamino-4'-(4-isopropylaminobutoxy)-[1,1';3',1"]terphenyl-4-carboxylic acid
31. 3"-*tert*-butyl-4'-(4-isopropylaminobutoxy)-4"-pyrrolidin-1-yl[1,1';3',1"]terphenyl-4-carboxylic acid
32. ethyl 3"-*tert*-butyl-4"-diethylamino-4'-(3-hydroxypropyl)-[1,1';3',1"]terphenyl-4-carboxylate
33. 3"-*tert*-butyl-4"-diethylamino-4'-(3-hydroxypropyl)-[1,1';3',1"]terphenyl-4-carboxylic acid
34. ethyl 3"-*tert*-butyl-4"-diethylamino-4'-(2,3-dihydroxypropyl)-[1,1';3',1"]terphenyl-4-carboxylate
35. 3"-*tert*-butyl-4"-diethylamino-4'-(2,3-dihydroxypropyl)-[1,1';3',1"]terphenyl-4-carboxylic acid
36. ethyl 4"-(acetylethylamino)-3"-*tert*-butyl-4'-(3-hydroxypropyl)-[1,1';3',1"]terphenyl-4-carboxylate
37. 4"-(acetylethylamino)-3"-*tert*-butyl-4'-(3-hydroxypropyl)-[1,1';3',1"]terphenyl-4-carboxylic acid
38. ethyl 3"-*tert*-butyl-4'-(3-cyclopropylaminopropyl)-4"-diethylamino[1,1';3',1"]terphenyl-4-carboxylate
39. 3"-*tert*-butyl-4'-(3-cyclopropylaminopropyl)-4"-diethylamino[1,1';3',1"]terphenyl-4-carboxylic acid
40. ethyl 3"-*tert*-butyl-4'-(3-cyclopentylaminopropyl)-4"-diethylamino[1,1';3',1"]terphenyl-4-carboxylate
41. 3"-*tert*-butyl-4'-(3-cyclopentylaminopropyl)-4"-diethylamino[1,1';3',1"]terphenyl-4-carboxylic acid
42. ethyl 3"-*tert*-butyl-4'-(3-cyclohexylaminopropyl)-4"-diethylamino[1,1';3',1"]terphenyl-4-carboxylate
43. 3"-*tert*-butyl-4'-(3-cyclohexylaminopropyl)-4"-diethylamino[1,1'; 3',1 "]terphenyl-4-carboxylic acid
44. ethyl 3"-*tert*-butyl-4'-(3-tert-butylaminopropyl)-4"-diethylamino[1,1';3',1"]terphenyl-4-carboxylate
45. 3"-*tert*-butyl-4'-(3-tert-butylaminopropyl)-4"-diethylamino[1,1';3',1"]terphenyl-4-carboxylic acid
46. ethyl 4"-(acetylethylamino)-3"-*tert*-butyl-4'-(3-cyclopropylaminopropyl)-[1,1';3',1"]terphenyl-4-carboxylate
47. 4"-(acetylethylamino)-3"-*tert*-butyl-4'-(3-cyclopropylaminopropyl)-[1,1';3',l"]terphenyl-4-carboxylic acid
48. ethyl 3"-*tert*-butyl-4"-diethylamino-4'-(3-isopropylaminopropyl)-[1,1';3',1"]terphenyl-4-carboxylate
49. 3"-*tert*-butyl-4"-diethylamino-4'-(3-isopropylaminopropyl)-[1,1';3',1"]terphenyl-4-carboxylic acid
50. ethyl 4'-(3-aminopropyl)-3"-*tert*-butyl-4"-diethylamino[1,1';3',1"]terphenyl-4-carboxylate
51. 4'-(3-aminopropyl)-3"-*tert*-butyl-4"-diethylamino[1,1';3',1 "]terphenyl-4-carboxylic acid
52. [3"-*tert*-butyl-4-carboxy-4'-(3-hydroxypropyl)-[1,1';3',1"]terphenyl-4"-yl]diethylamine hydrochloride
53. 3"-*tert*-butyl-4'-(2-hydroxy-ethoxy)-4"-(2-oxo-pyrrolidin-1-yl)-[1,1'; 3',1"]terphenyl-4-carboxylic acid
54. 3"-tert-butyl-4"-ethylamino-4'-(3-hydroxy-propoxy)-[1,1';3',1"]terphenyl-4-carboxylic acid
55. 4'-(3-acetoxypropoxy)-3"-*tert*-butyl-4"-diethylamino[1,1';3',1"]terphenyl-4-carboxylic acid
56. 3"-*tert*-butyl-4"-diethylamino-4'-(3-propionyloxypropoxy)-[1,1';3',1"]terphenyl-4-carboxylic acid
57. methyl 3"-*tert*-butyl-4"-diethylamino-4'-(3-hydroxypropoxy)-[1,1';3',1"]terphenyl-4-carboxylate
58. isopropyl 3"-*tert*-butyl-4"-diethylamino-4'-(3-hydroxypropoxy)-[1,1';3',1"]terphenyl-4-carboxylate
59. isobutyl 3"-*tert*-butyl-4"-diethylamino-4'-(3-hydroxypropoxy)-[1,1';3',1"]terphenyl-4-carboxylate
60. 3"-*tert*-butyl-4"-diethylamino-4'-(3-hydroxypropoxy)-5"-methyl[1,1';3',1"]terphenyl-4-carboxylic acid
61. 4"-diethylamino-4'-(3-hydroxypropoxy)-3"-isopropyl-5"-methyl[1,1';3',1"]terphenyl-4-carboxylic acid
62. 3"-*tert*-butyl-5"-chloro-4"-diethylamino-4'-(3-hydroxypropoxy)-[1,1';3',l"]terphenyl-4-carboxylic acid
63. 4"-diethylamino-4'-(3-hydroxypropoxy)-3",5"-diisopropyl[1,1';3',1"]terphenyl-4-carboxylic acid
64. 3",5"-di-*tert*-butyl-4"-diethylamino-4'-(3-hydroxypropoxy)-[1,1';3',1"]terphenyl-4-carboxylic acid
65. 4"-diethylamino-4'-(3-hydroxypropoxy)-3"-trifluoromethyl[1,1';3',1"]terphenyl-4-carboxylic acid
66. 3"-*tert*-butyl-4"-(ethylmethylamino)-4'-(2-hydroxyethoxy)-[1,1';3',1"]terphenyl-4-carboxylic acid
67. 3"-*tert*-butyl-4"-dimethylamino-4'-(2-hydroxyethoxy)-[1,1';3',1"]terphenyl-4-carboxylic acid
68. 3"-*tert*-butyl-4"-(ethylisopropylamino)-4'-(2-hydroxyethoxy)-[1,1';3',1"]terphenyl-4-carboxylic acid
69. 3"-*tert*-butyl-4"-(ethylpropylamino)-4'-(2-hydroxyethoxy)-[1,1';3',1"]terphenyl-4-carboxylic acid
70. 3"-*tert*-butyl-4"-dipropylamino-4'-(2-hydroxyethoxy)-[1,1';3',1"]terphenyl-4-carboxylic acid
71. 3"-*tert*-butyl-4"-(ethylpropionylamino)-4'-(2-hydroxyethoxy)-[1,1'; 3',1"]terphenyl-4-carboxylic acid
72. 6-[3'-*tert*-butyl-4'-diethylamino-6-(2-hydroxyethoxy)biphenyl-3-yl]nicotinic acid
73. 5-[3'-*tert*-butyl-4'-diethylamino-6-(2-hydroxyethoxy)biphenyl-3-yl]pyridine-2-carboxylic acid
74. 5-[3'-*tert*-butyl-4'-diethylamino-6-(2-hydroxyethoxy)biphenyl-3-yl]thiophene-2-carboxylic acid
75. 3"-*tert*-butyl-4'-(2-hydroxyethoxy)-5"-methyl-4"-pyrrolidin-1-yl[1,1';3',1"]terphenyl-4-carboxylic acid
76. 3"-*tert*-butyl-5"-chloro-4'-(2-hydroxyethoxy)-4"-pyrrolidin-1-yl[1,1';3',1"]terphenyl-4-carboxylic acid
77. 4'-(2-hydroxyethoxy)-3"-isopropyl-4"-pyrrolidin-1-yl[1,1';3',1"]terphenyl-4-carboxylic acid
78. 3"-ethyl-4'-(2-hydroxyethoxy)-4"-pyrrolidin-1-yl[1,1';3',1"]terphenyl-4-carboxylic acid
79. 4'-(2-hydroxyethoxy)-3",5"-diisopropyl-4"-pyrrolidin-1-yl[1,1';3',1"]terphenyl-4-carboxylic acid
80. 3",5"-diethyl-4'-(2-hydroxyethoxy)-4"-pyrrolidin-1-yl[1,1';3',1"]terphenyl-4-carboxylic acid
81. 3",5"-dimethyl-4'-(2-hydroxyethoxy)-4"-pyrrolidin-1-yl[1,1';3',1"]terphenyl-4-carboxylic acid
82. 4'-(2-acetoxyethoxy)-3"-*tert*-butyl-4"-pyrrolidin-1-yl[1,1';3',1"]terphenyl-4-carboxylic acid
83. 4'-(2-propionyloxyethoxy)-3"-*tert*-butyl-4"-pyrrolidin-1-yl[1,1';3',1"]terphenyl-4-carboxylic acid
84. methyl 3"-*tert*-butyl-4'-(2-hydroxyethoxy)-4"-pyrrolidin-1-yl[1,1';3',1"]terphenyl-4-carboxylate
85. isopropyl 3"-*tert*-butyl-4'-(2-hydroxyethoxy)-4"-pyrrolidin-1-yl[1,1';3',1"]terphenyl-4-carboxylate
86. isobutyl 3"-*tert*-butyl-4'-(2-hydroxyethoxy)-4"-pyrrolidin-1-yl[1,1';3',1"]terphenyl-4-carboxylate
87. ethyl 3"-*tert*-butyl-4'-(3-hydroxypropoxy)-4"-pyrrolidin-1-yl[1,1';3',1"]terphenyl-4-carboxylate
88. ethyl 3"-*tert*-butyl-5"-chloro-4'-(2-hydroxyethoxy)-4"-pyrrolidin-1-yl[1,1';3',1"]terphenyl-4-carboxylate
89. 6-[3'-*tert*-butyl-6-(2-hydroxyethoxy)-4'-pyrrolidin-1-ylbiphenyl-3-y]nicotinic acid
90. 5-[3'-*tert*-butyl-6-(2-hydroxyethoxy)-4'-pyrrolidin-1-ylbiphenyl-3-yl]pyridine-2-carboxylic acid
91. ethyl 6-[3'-*tert*-butyl-6-(2-hydroxyethoxy)-4'-pyrrolidin-1-ylbiphenyl-3-yl]nicotinate
92. ethyl 3"-*tert*-butyl-4'-(3-hydroxypropyl)-4"-pyrrolidin-1-yl[1,1';3',1"]terphenyl-4-carboxylate
93. 3"-*tert*-butyl-4'-(3-hydroxypropyl)-4"-pyrrolidin-1-yl[1,1'; 3',1"]terphenyl-4-carboxylic acid
94. 3"-*tert*-butyl-4'-(2-hydroxyethoxy)-4"-(2-oxopyrrolidin-1-yl)-[1,1'; 3',1"]terphenyl-4-carboxylic acid
95. 3"-*tert*-butyl-4'-(2-hydroxyethoxy)-4"-(2-oxopiperid-1-yl)-[1,1';3',1"]terphenyl-4-carboxylic acid
96. 3"-*tert*-butyl-4'-(2-hydroxyethoxy)-4"-piperid-1-yl[1,1';3',1"]terphenyl-4-carboxylic acid
97. 5-[3'-*tert*-butyl-6-(2-hydroxyethoxy)-4'-pyrrolidin-1-ylbiphenyl-3-yl]thiophene-2-carboxylic acid.

9. Compounds according to Claim 1, **characterized in that** they have at least one of the following characteristics:
- R1 is a hydrogen atom or a t-butyl or i-propyl radical;
- R2 is a hydrogen atom or a t-butyl or i-propyl radical;
- R3 is a hydrogen atom or an ethyl radical;
- R4 and R5 are, independently of each other, a methyl or ethyl radical or together form a pyrrolidine ring;
- A is as defined above in which R₆ represents a hydrogen atom, an i-propyl or t-butyl radical, a cycloalkyl radical of 3 to 6 carbon atoms or a -C(O)CH₃ or -C(O)CH₂CH₃ radical.

10. Compounds according to Claim 9, **characterized in that** they have all of the following characteristics:
- R1 is a hydrogen atom or a t-butyl or i-propyl radical;
- R2 is a hydrogen atom or a t-butyl or i-propyl radical;
- R3 is a hydrogen atom or an ethyl radical;
- R4 and R5 are, independently of each other, a methyl or ethyl radical or together form a pyrrolidine ring;
- A is as defined above in which R₆ represents a hydrogen atom, an i-propyl or t-butyl radical, a cycloalkyl radical of 3 to 6 carbon atoms or a -C(O)CH₃ or -C(O)CH₂CH₃ radical.

11. Compounds according to any one of Claims 1 to 10, as medicaments.

12. Use of a compound according to any one of Claims 1 to 10 in the manufacture of a composition for treating:
- dermatological complaints associated with a keratinization disorder relating to cell differentiation and proliferation;
- ichthyosis, ichthyosiform conditions, Darier's disease, palmoplantar keratoderma, leukoplakia and leukoplakiform conditions, and cutaneous or mucous (buccal) lichen;
- dermatological complaints with an inflammatory immunoallergic component, with or without a cell proliferation disorder;
- skin disorders caused by exposure to UV radiation, photoinduced or chronological ageing of the skin, or actinic pigmentations and keratoses;
- pathologies associated with chronological or actinic ageing of the skin;
- benign or malignant dermal or epidermal proliferations, of viral or non-viral origin;
- proliferations that may be induced by ultraviolet radiation;
- precancerous skin lesions;
- immune dermatoses;
- immune bullous diseases;
- collagen diseases;
- dermatological complaints with an immunological component;
- ophthalmological disorders;
- stigmata of epidermal and/or dermal atrophy induced by local or systemic corticosteroids, or any other form of cutaneous atrophy;
- skin complaints of viral origin;
- skin disorders caused by exposure to UV radiation, photoinduced or chronological ageing of the skin, or actinic pigmentations and keratoses;
- pathologies associated with chronological or actinic ageing of the skin;
- disorders of sebaceous function;
- cicatrization disorders or stretch marks; or
- pigmentation disorders.

13. Use according to Claim 12, **characterized in that** the dermatological complaints associated with a keratinization disorder relating to cell differentiation and proliferation are chosen from common acne, comedones, polymorphonuclear leukocytes, acne rosacea, nodulocystic acne, acne conglobata, senile acne, and secondary acnes such as solar acne, medication-related acne or occupational acne.

14. Use according to Claim 12, **characterized in that** the dermatological complaints with an inflammatory immunoallergic component, with or without a cell proliferation disorder, are chosen from all forms of psoriasis, whether cutaneous, mucous or ungual psoriasis, and even psoriatic rheumatism, cutaneous atopy, such as eczema, or respiratory atopy, or even gingival hypertrophy.

15. Pharmaceutical composition, **characterized in that** it comprises, in a physiologically acceptable support, at least one of the compounds as defined in any one of Claims 1 to 10.

16. Composition according to Claim 15, **characterized in that** the concentration of compound(s) according to any one of Claims 1 to 10 is between 0.001 % and 10% by weight relative to the total weight of the composition.

17. Composition according to Claim 16, **characterized in that** the concentration of compound(s) according to one of Claims 1 to 10 is between 0.01% and 1% by weight relative to the total weight of the composition.

18. Cosmetic composition, **characterized in that** it comprises, in a physiologically acceptable medium, at least one of the compounds as defined in any one of Claims 1 to 10.

19. Composition according to Claim 18, **characterized in that** the concentration of compound(s) according to one of Claims 1 to 10 is between 0.001% and 3% by weight relative to the total weight of the composition.

20. Non-therapeutic use of a cosmetic composition as defined in one of Claims 1 to 10, for preventing and/or treating the signs of ageing and/or dry skin.

21. Non-therapeutic use of a cosmetic composition as defined in one of Claims 1 to 10, for body or hair hygiene.

22. Cosmetic process for enhancing the appearance of the skin, **characterized in that** a composition as defined in any one of Claims 1 to 10 is applied to the skin.

## Patentansprüche

1. Verbindungen, die **dadurch gekennzeichnet sind, dass** sie der Formel (I) unten entsprechen: 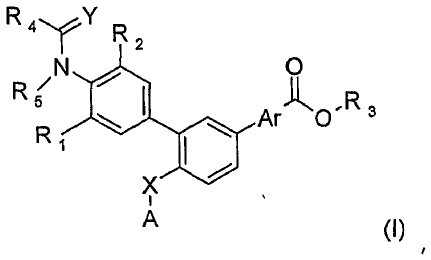 in der
- R₁ ein Wasserstoffatom, einen Alkylrest mit 1 bis 4 Kohlenstoffatomen oder einen -CF₃-Rest bedeutet;
- R₂ ein Wasserstoffatom, einen Alkyl- oder Alkoxyrest mit 1 bis 4 Kohlenstoffatomen oder ein Chloratom bedeutet;
- R₃ ein Wasserstoffatom oder einen geradkettigen oder verzweigten Alkylrest mit 1 bis 10 Kohlenstoffatomen, der gegebenenfalls durch eine Ethoxygruppe substituiert ist, oder einen geradkettigen oder verzweigten Alkylrest mit 1 bis 10 Kohlenstoffatomen, der eine Etherfunktion enthält, bedeutet;
- R₄ ein Wasserstoffatom oder einen Alkylrest mit 1 bis 3 Kohlenstoffatomen bedeutet;
- R₅ ein Wasserstoffatom oder einen Alkylrest mit 1 bis 3 Kohlenstoffatomen bedeutet;
- oder alternativ dazu R₄ und R₅ gemeinsam mit der Bindung -N-C(=Y)- einen Ring des Pyrrolidin-, Pyrrolidinon-, Piperidin- oder Piperidinontyps bilden;
- Y zwei Wasserstoffatome oder ein Heteroatom, zum Beispiel Sauerstoff oder Schwefel, bedeutet;
- Ar einen 1,4-Phenyl-, 2,5-Pyridyl-, 5,2-Pyridyl- oder 2,5-Thiophenylring bedeutet;
- X ein Sauerstoffatom, das gegebenenfalls durch eine Alkyl- oder Alkylaminkette substituiert ist, oder eine C-C-Einfachbindung bedeutet;
- A ein Wasserstoffatom oder die folgende Formel bedeutet: 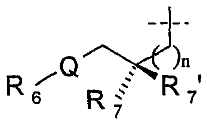
in der
o Q ein Sauerstoffatom oder eine -NH-Bindung bedeutet;
o R₆ ein Wasserstoffatom, einen Alkylrest mit 1 bis 6 Kohlenstoffatomen, einen Cycloalkylrest mit 3 bis 6 Kohlenstoffatomen oder einen -C(O)CH₃- oder - C(O)CH₂CH₃-Rest bedeutet;
o R₇ und R₇' unabhängig voneinander ein Wasserstoffatom oder eine Hydroxylgruppe bedeuten, mit der Maßgabe, dass R₇ und R₇' nicht zugleich eine Hydroxylgruppe bedeuten;
o n 0, 1, 2, 3, 4 oder 5 bedeutet;
sowie die Salze der Verbindungen der Formel (I), wenn R₃ ein Wasserstoffatom, OH bedeutet sowie die geometrischen Isomere der Verbindungen der Formel (I).

2. Verbindungen nach Anspruch 1, **dadurch gekennzeichnet, dass** sie in Form von Alkalimetall- oder Erdalkalimetallsalzen, Zinksalzen oder Salzen eines organischen Amins oder, wenn die Verbindung selbst basisch ist, eines sauren Partners vorliegen.

3. Verbindung nach Anspruch 1, **dadurch gekennzeichnet, dass** der Alkylrest mit 1 bis 3 Kohlenstoffatomen aus der Reihe Methyl-, Ethyl-, i-Propyl- und n-Propylreste ausgewählt ist.

4. Verbindung nach Anspruch 1, **dadurch gekennzeichnet, dass** der Alkylrest mit 1 bis 4 Kohlenstoffatomen, aus der Reihe Methyl-, Ethyl-, i-Propyl-, i-Butyl- und t-Butylreste ausgewählt ist.

5. Verbindung nach Anspruch 1, **dadurch gekennzeichnet, dass** der Alkylrest mit 1 bis 10 Kohlenstoffatomen aus der Reihe Methyl-, Ethyl-, Propyl-, i-Propyl-, Butyl-, i-Butyl-, t-Butyl-, Pentyl-, Hexyl-, Heptyl-, Octyl-, Nonyl- und Dodecylreste ausgewählt ist.

6. Verbindung nach Anspruch 1, **dadurch gekennzeichnet, dass** der Alkoxyrest, der 1 bis 4 Kohlenstoffatome enthält, aus der Reihe Methoxy-, Ethoxy-, Isopropyloxy- und tert.-Butoxyreste ausgewählt ist.

7. Verbindung nach Anspruch 1, **dadurch gekennzeichnet, dass** der Cycloalkylrest mit 3 bis 5 Kohlenstoffatomen aus der Reihe Cyclopropyl-, Cyclopentyl- und Cyclohexylreste ausgewählt ist.

8. Verbindungen der Formel (I) nach einem der vorhergehenden Ansprüche, die aus den folgenden ausgewählt sind:
1. Ethyl-3"-*tert*.-butyl-4"-diethylamino-4'-hydroxy[1,1';3',1"]terphenyl-4-carboxylat
2. Ethyl-3"-*tert*.-butyl-4"-diethylamino-4'-(4-hydroxybutoxy)-[1,1';3',1"]terphenyl-4-carboxylat
3. 3"-*tert*.-Butyl-4"-diethylamino-4'-(4-hydroxybutoxy)-[1,1';3',1"]terphenyl-4-carbonsäure
4. Ethyl-4"(acetylethylamino)-3"-*tert*.-butyl-4'-(hydroxybutoxy)-[1,1';3',1"]terphenyl-4-carboxylat
5. 4"-(cetylethylamino)-3"-*tert*.-butyl-4'-(4-hydroxybutoxy)-[1,1';3',1"]terphenyl-4-carbonsäure
6. 3"-*tert*.-Butyl-4"-diethylamino-4'-(2-hydroxyethoxy)-[1,1';3',1"]terphenyl-4-carbonsäure
7. Ethyl-3"-*tert*.-butyl-4"-diethylamino-4'-(3-hydroxypropoxy)-[1,1';3',1"]terphenyl-4-carboxylat
8. 3"-*tert*.-Butyl-4"-diethylamino-4'-(3-hydroxypropoxy)-[1,1';3',1"]terphenyl-4-carbonsäure
9. Ethyl-4"-diethylamino-4'-(3-hydroxypropoxy)-[1,1';3',1"]terphenyl-4-carboxylat
10. 4"-Diethylamino-4'-(3-hydroxypropoxy)-[1,1';3',1"]terphenyl-4-carbonsäure
11. Ethyl-4"-diethylamino-3"-ethyl-4'-(3-hydroxypropoxy)-[1,1';3',1"]terphenyl-4-carboxylat
12. 4"-Diethylamino-3"-ethyl-4'-(3-hydroxypropoxy)-[1,1';3',1"]terphenyl-4-carbonsäure
13. 4"-Diethylamino-3"-ethyl-4'-(2-hydroxyethoxy)-[1,1';3',1"]terphenyl-4-carbonsäure
14. Ethyl-4"-diethylamino-3"-ethyl-4'-(2-hydroxyethoxy)-[1,1';3',1"]terphenyl-4-carboxylat
15. Ethyl-3"-*tert*.-butyl-4"-(2-hydroxyethoxy)-[1,1';3',1"] terphenyl-4-carboxylat
16. Ethyl-4"-diethylamino-4'-(3-hydroxypropoxy)-3"-methyl[1,1';3',1"]terphenyl-4-carboxylat
17. 4"-Diethylamino-4'-(3-hydroxypropoxy)-3"-methyl[1,1';3',1"]terphenyl-4-carbonsäure
18. Ethyl-3"-*tert*.-butyl-4'-(4-hydroxybutoxy)-4"-pyrrolidin-1-yl[1,1';3',1"]terphenyl-4-carboxylat
19. 3"-*tert*.-Butyl-4'-(4-hydroxybutoxy)-4"-pyrrolidin-1-yl[1,1';3',1"]terphenyl-4-carbonsäure
20. Ethyl-4'-(4-hydroxybutoxy)-4"-pyrrolidin-1-yl-3"-trifluormethyl[1,1';3',1"]terphenyl-4-carboxylat
21. 4'-(4-Hydroxybutoxy)-4"-pyrrolidin-1-yl-3"-trifluormethyl[1,1';3',1"]terphenyl-4-carbonsäure
22. Ethyl-3"-*tert*.-butyl-4'-(3-hydroxypropoxy)-4"-pyrrolidin-1-yl[1,1';3',1"]terphenyl-4-carboxylat
23. 3"-*tert*.-Butyl-4'-(3-hydroxypropoxy)-4"-pyrrolidin-1-yl[1,1';3',1"]terphenyl-4-carbonsäure
24. Ethyl-3"-*tert*.-butyl-4'-(2-hydroxyethoxy)-4"-pyrrolidin-1-yl[1,1';3',1"]terphenyl-4-carboxylat
25. 3"-*tert*.-Butyl-4"-(2-hydroxyethoxy)-4"-pyrrolidin-1-yl[1,1';3',1"]terphenyl-4-carbonsäure
26. 4'-(3-Hydroxypropoxy)-4"-pyrrolidin-1-yl[1,1';3',1"]terphenyl-4-carbonsäure
27. 3"-*tert*.-Butyl-4"-diethylamino-4'-hydroxy[1,1';3',1"]terphenyl-4-carbonsäure
28. Ethyl-4"-diethylamino-4'-hydroxy-3"-trifluormethyl[1,1';3',1"]terphenyl-4-carboxylat
29. 4"-Diethylamino-4'-hydroxy-3"-trifluormethyl[1,1';3',1"]terphenyl-4-carbonsäure
30. 3"-*tert*.-Butyl-4"-diethylamino-4'-(4-isopropylaminobutoxy)-[1,1';3',1"]terphenyl-4-carbonsäure
31. 3"-*tert*.-Butyl-4'-(4-isopropylaminobutoxy)-4"-pyrrolidin-1-yl[1,1';3',1"]terphenyl-4-carbonsäure
32. Ethyl-3"-*tert*.-butyl-4"-diethylamino-4'-(3-hydroxypropyl)-[1,1';3',1"]terphenyl-4-carboxylat
33. 3"-*tert*.-Butyl-4"-diethylamino-4'-(3-hydroxypropyl)-[1,1';3',1"]terphenyl-4-carbonsäure
34. Ethyl-3"-*tert*.-butyl-4"-diethylamino-4'-(2,3-dihydroxypropyl)-[1,1';3',1"]terphenyl-4-carboxylat
35. 3"-*tert*.-Butyl-4"-diethylamino-4'-(2,3-dihydroxypropyl)-[1,1';3',1"]terphenyl-4-carbonsäure
36. Ethyl-4"-(acetylethylamino)-3"-*tert*.-butyl-4'-(3-hydroxypropyl)-[1,1';3',1"]terphenyl-4-carboxylat
37. 4"-(Acetylethylamino)-3"-*tert*.-butyl-4'-(3-hydroxypropyl)-[1,1';3',1"]terphenyl-4-carbonsäure
38. Ethyl-3"-*tert*.-butyl-4'-(3-cyclopropylaminopropyl)-4"-diethylamino[1,1';3',1"]terphenyl-4-carboxylat
39. 3"-*tert*.-Butyl-4'-(3-cyclopropylaminopropyl)-4"-diethylamino[1,1';3',1"]terphenyl-4-carbonsäure
40. Ethyl-3"-*tert*.-butyl-4'-(3-cyclopentylaminopropyl)-4"-diethylamino[1,1';3',1"]terphenyl-4-carboxylat
41. 3"-*tert*.-Butyl-4'-(3-cyclopentylaminopropyl)-4"-diethylamino[1,1';3',1"]terphenyl-4-carbonsäure
42. Ethyl-3"-*tert*.-butyl-4'-(3-cyclohexylaminopropyl)-4"-diethylamino[1,1';3',1"] terphenyl-4-carboxylat
43. 3"-*tert*.-Butyl-4'-(3-cyclohexylaminopropyl)-4"-diethylamino[1,1';3',1"]terphenyl-4-carbonsäure
44. Ethyl-3"-*tert*.-butyl-4'-(3-tert.-butylaminopropyl)-4"-diethylamino[1,1;3',1"]terphenyl-4-carboxylat
45. 3"-*tert*.-Butyl-4'-(3-tert.-butylaminopropyl)-4"-diethylamino[1,1';3',1"]terphenyl-4-carbonsäure
46. Ethyl-4"-(acetylethylamino)-3"-*tert*.-butyl-4'-(3-cyclopropylaminopropyl)-[1,1';3',1"]terphenyl-4-carboxylat
47. 4"-(Acetylethylamino)-3"-*tert*.-butyl-4'-(3-cyclopropylaminopropyl)-[1,1;3',1"]terphenyl-4-carbonsäure
48. Ethyl-3"-*tert*.-butyl-4"-diehtylamino-4'-(3-isopropylaminopropyl)-[1,1';3',1"]terphenyl-4-carboxylat
49. 3"-*tert*.-Butyl-4"-diethylamino-4'-(3-isopropylaminopropyl)-[1,1';3',1"]terphenyl-4-carbonsäure
50. Ethyl-4'-(3-aminopropyl)-3"-*tert*.-butyl-4"-diethylamino[1,1';3',1"]terphenyl-4-carboxylat
51. 4'-(3-Aminopropyl)-3"-*tert*.-butyl-4"-diethylamino[1,1';3',1"]terphenyl-4-carbonsäure
52. [3"-*tert*.-Butyl-4-carboxy-4'-(3-hydroxypropyl)-[1,1';3',1"]terphenyl-4"-yl]diethylamin-hydrochlorid
53. 3"-*tert*.-Butyl-4'-(2-hydroxyethoxy)-4"-(2-oxopyrrolidin-1-yl)-[1,1';3',1"]terphenyl-4-carbonsäure
54. 3"-*tert*.-Butyl-4"-ethylamino-4'-(3-hydroxypropoxy)-[1,1';3',1"]terphenyl-4-carbonsäure
55. 4'-(3-Acetoxypropoxy)-3"-*tert*.-butyl-4"-diethylamino[1,1';3',1"]terphenyl-4-carbonsäure
56. 3"-*tert*.-Butyl-4"-diethylamino-4'-(3-propionyloxypropoxy)-[1,1';3',1"] terphenyl-4-carbonsäure
57. Methyl-3"-*tert*.-butyl-4"-diethylamino-4'-(3-hydroxypropoxy)-[1,1';3',1"]terphenyl-4-carboxylat
58. Isopropyl-3"-*tert*.-butyl-4"-diethylamino-4'-(3-hydroxypropoxy)-[1,1';3',1"]terphenyl-4-carboxylat
59. Isobutyl-3"-*tert*.-butyl-4"-diethylamino-4'-(3-hydroxypropoxy)-[1,1';3',1"]terphenyl-4-carboxylat
60. 3"-*tert*.-Butyl-4"-diethylamino-4'-(3-hydroxypropoxy)-5"-methyl[1,1';3',1"]terphenyl-4-carbonsäure
61. 4"-Diethylamino-4'-(3-hydroxypropoxy)-3"-isopropyl-5"-methyl[1,1';3',1"]terphenyl-4-carbonsäure
62. 3"-*tert*.-Butyl-5"-chlor-4"-diethylamino-4'-(3-hydroxypropoxy)-[1,1';3',1"]terphenyl-4-carbonsäure
63. 4"-Diethylamino-4'-(3-hydroxypropoxy)-3" ,5"-diisopropyl [1,1';3',1"] terphenyl-4-carbonsäure
64. 3", 5"-Di-*tert*.-butyl-4"-diethylamino-4'-(3-hydroxypropoxy)-[1,1';3',1"]terphenyl-4-carbonsäure
65. 4"-Diethylamino-4'-(3-hydroxypropoxy)-3"-trifluormethyl[1,1';3',1"] terphenyl-4-carbonsäure
66. 3"-*tert*.-Butyl-4"-(ethylmethylamino)-4'-(2-hydroxyethoxy)-[1,1';3',1"]terphenyl-4-carbonsäure
67. 3"-*tert*.-Butyl-4"-dimethylamino-4'-(2-hydroxyethoxy)-[1,1';3',1"]terphenyl-4-carbonsäure
68. 3"-*tert*.-Butyl-4"-(ethylisopropylamino)-4'-(2-hydroxyethoxy)-[1,1';3',1"]terphenyl-4-carbonsäure
69. 3"-*tert*.-Butyl-4"-(ethylpropylamino)-4'-(2-hydroxyethoxy)-[1,1';3',1"]terphenyl-4-carbonsäure
70. 3"-*tert*.-Butyl-4"-dipropylamino-4'-(2-hydroxyethoxy)-[1,1';3',1']terphenyl-4-carbonsäure
71. 3"-*tert*.-Butyl-4"-(ethylpropionylamino)-4'-(2-hydroxyethoxy)-[1,1';3',1"]terphenyl-4-carbonsäure
72. 6-[3'-*tert*.-Butyl-4'-diethylamino-6-(2-hydroxyethoxy)biphenyl-3-yl]nicotinsäure
73. 5-[3'-*tert*.-Butyl-4'-diethylamino-6-(2-hydroxyethoxy)biphenyl-3-yl]pyridin-2-carbonsäure
74. 5-[3'-*tert*.-Butyl-4'-diethylamino-6-(2-hydroxyethoxy)biphenyl-3-yl]thiophen-2-carbonsäure
75. 3"-*tert*.-Butyl-4'-(2-hydroxyethoxy)-5"-methyl-4"-pyrrolidin-1-yl[1,1';3',1"]terphenyl-4-carbonsäure
76. 3"-*tert*.-Butyl-5"-chlor-4'-(2-hydroxyethoxy)-4"-pyrrolidin-1-yl[1,1';3',1"]terphenyl-4-carbonsäure
77. 4'-(2-Hydroxyethoxy)-3"-isopropyl-4"-pyrrolidin-1-yl [1,1';3',1"] terphenyl-4-carbonsäure
78. 3"-Ethyl-4'-(2-hydroxyethoxy)-4"-pyrrolidin-1-yl[1,1';3',1"]terphenyl-4-carbonsäure
79. 4'-(2-Hydroxyethoxy)-3",5"-diisopropyl-4"-pyrrolidin-1-yl[1,1';3',1"] terphenyl-4-carbonsäure
80. 3",5"-Diethyl-4'-(2-hydroxyethoxy)-4"-pyrrolidin-1-yl [1,1';3',1"] terphenyl-4-carbonsäure
81. 3",5"-Dimethyl-4'-(2-hydroxyethoxy)-4"-pyrrolidin-1-yl[1,1';3',1"] terphenyl-4-carbonsäure
82. 4'-(2-Acetoxyethoxy)-3"-*tert*.-butyl-4"-pyrrolidin-1-yl[1,1';3',1"]terphenyl-4-carbonsäure
83. 4'-(2-Propionyloxyethoxy)-3"-*tert*.-butyl-4"-pyrrolidin-1-yl[1,1';3',1"]terphenyl-4-carbonsäure
84. Methyl-3"-*tert*.-butyl-4'-(2-hydroxyethoxy)-4"-pyrrolidin-1-yl [1,1';3',1"] terphenyl-4-carboxylat
85. Isopropyl-3"-*tert*.-butyl-4'-(2-hydroxyethoxy)-4"-pyrrolidin-1-yl[1,1';3',1"]terphenyl-4-carboxylat
86. Isobutyl-3"-*tert*.-butyl-4'-(2-hydroxyethoxy)-4"-pyrrolidin-1-yl[1,1';3',1"]terphenyl-4-carboxylat
87. Ethyl-3"-*tert*.-butyl-4'-(3-hydroxypropoxy)-4"-pyrrolidin-1-yl[1,1';3',1"]terphenyl-4-carboxylat
88. Ethyl-3"-*tert*.-butyl-5"-chlor-4'-(2-hydroxyethoxy)-4"-pyrrolidin-1-yl[1,1';3',1"]terphenyl-4-carboxylat
89. 6-[3'-*tert*.-Butyl-6-(2-hydroxyethoxy)-4'-pyrrolidin-1-ylbiphenyl-3-yl]nicotinsäure
90. 5-[3'-*tert*.-Butyl-6-(2-hydroxyethoxy)-4'-pyrrolidin-1-ylbiphenyl-3-yl]pyridin-2-carbonsäure
91. Ethyl-6-[3'-*tert*.-butyl-6-(2-hydroxyethoxy)-4'-pyrrolidin-1-ylbiphenyl-3-yl]nicotinat
92. Ethyl-3"-*tert*.-butyl-4'-(3-hydroxypropyl)-4"-pyrrolidin-1-yl[1,1';3',1"]terphenyl-4-carboxylat
93. 3"-*tert*.-Butyl-4'-(3-hydroxypropyl)-4"-pyrrolidin-1-yl[1,1';3',1"]terphenyl-4-carbonsäure
94. 3"-*tert*.-Butyl-4'-(2-hydroxyethoxy)-4"-(2-oxopyrrolidin-1-yl)-[1,1';3',1"]terphenyl-4-carbonsäure
95. 3"-*tert*.-Butyl-4'-(2-hydroxyethoxy)-4"-(2-oxopiperid-1-yl)-[1,1';3',1"]terphenyl-4-carbonsäure
96. 3"-*tert*.-Butyl-4'-(2-hydroxyethoxy)-4"-piperid-1-yl[1,1';3',1"]terphenyl-4-carbonsäure
97. 5-[3'-*tert*.-Butyl-6-(2-hydroxyethoxy)-4'-pyrrolidin-1-ylbiphenyl-3-yl]thiophen-2-carbonsäure.

9. Verbindungen nach Anspruch 1, **dadurch gekennzeichnet, dass** sie mindestens eine der folgenden Eigenschaften aufweisen:
- R1 bedeutet ein Wasserstoffatom oder einen t-Butyl- oder i-Propylrest;
- R2 bedeutet ein Wasserstoffatom oder einen t-Butyl- oder i-Propylrest;
- R3 bedeutet ein Wasserstoffatom oder einen Ethylrest;
- R4 und R5 unabhängig voneinander bedeuten einen Methyl- oder Ethylrest oder bilden gemeinsam einen Pyrrolidinring;
- A ist wie oben definiert, wobei R₆ ein Wasserstoffatom, einen i-Propyl- oder t-Butylrest, einen Cycloalkylrest mit 3 bis 6 Kohlenstoffatomen oder einen -C(O)CH₃- oder -C(O)CH₂CH₃-Rest bedeutet.

10. Verbindungen nach Anspruch 9, **dadurch gekennzeichnet, dass** sie alle folgenden Eigenschaften aufweisen:
- R1 bedeutet ein Wasserstoffatom oder einen t-Butyl- oder i-Propylrest;
- R2 bedeutet ein Wasserstoffatom oder einen t-Butyl- oder i-Propylrest;
- R3 bedeutet ein Wasserstoffatom oder einen Ethylrest;
- R4 und R5 unabhängig voneinander bedeuten einen Methyl- oder Ethylrest oder bilden gemeinsam einen Pyrrolidinring;
- A ist wie oben definiert, wobei R₆ ein Wasserstoffatom, einen i-Propyl- oder t-Butylrest, einen Cycloalkylrest mit 3 bis 6 Kohlenstoffatomen oder einen -C(O)CH₃- oder -C(O)CH₂CH₃-Rest bedeutet.

11. Verbindungen nach einem der Ansprüche 1 bis 10 als Arzneimittel.

12. Verwendung einer Verbindung nach einem der Ansprüche 1 bis 10 in der Herstellung einer Zusammensetzung für die Behandlung der folgenden:
- dermatologische Leiden, die mit einer Keratinisierungsstörung bezüglich der Zelldifferenzierung und -proliferation einhergehen;
- Ichthyose, ichthyosiforme Leiden, Darier-Krankheit, palmoplantare Keratose, Leukoplakie und leukoforme Leiden sowie kutaner oder mukoser (bukkaler) Lichen;
- dermatologische Beschwerden mit entzündlicher immunallergischer Komponente, mit oder ohne Zellproliferationsstörung;
- Hautleiden, die durch Exposition durch UV-Strahlung verursacht sind, lichtinduzierte oder chronologische Hautalterung, oder aktinische Pigmentierungen und Keratosen;
- Pathologien, die mit der chronologischen oder aktinischen Hautalterung einhergehen;
- benigne oder maligne Haut- oder Epidermisproliferationen viralen oder nichtviralen Ursprungs;
- Proliferationen, die durch Ultraviolettstrahlung induziert werden können;
- prämaligne Hautläsionen;
- Immundermatosen;
- bullöse Immunkrankheiten;
- Kollagenosen;
- dermatologische Leiden mit immunologischer Komponente;
- ophthalmologische Erkrankungen;
- Stigmata von epidermaler und/oder dermaler Atrophierung, die durch lokale oder systemische Corticosteroide induziert sind, oder sonstige Formen von Hautatrophierung;
- Hautbeschwerden viralen Ursprungs;
- Hauterkrankungen, die durch Exposition durch UV-Strahlung verursacht sind, lichtinduzierte oder chronologische Hautalterung, oder aktinische Pigmentierungen und Keratosen;
- Pathologien, die mit der chronologischen oder aktinischen Hautalterung einhergehen;
- Störungen der Talgdrüsenfunktion;
- Vernarbungsstörungen oder Schwangerschaftsstreifen; oder
- Pigmentationsstörungen.

13. Verwendung nach Anspruch 12, **dadurch gekennzeichnet, dass** die dermatologischen Leiden, die mit einer Keratinisierungsstörung bezüglich der Zelldifferenzierung und -proliferation einhergehen, aus der Reihe Acne vulgaris, Komedonen, polymorphkernige neutrophile Granulozyten, Acne rosacea, nodulocystische Akne, Acne conglobata, Altersakne, sowie sekundäre Akneerkrankungen wie Acne solaris, arzneimittelbedingte Akne oder berufsbedingte Akne ausgewählt sind.

14. Verwendung nach Anspruch 12, **dadurch gekennzeichnet, dass** die dermatologischen Leiden mit entzündlicher immunallergischer Komponente, mit oder ohne Zellproliferationsstörung, aus allen Formen der Schuppenflechte, egal ob Schuppenflechte der Haut, der Schleimhaut oder des Nagels, ja sogar psoriatischer Rheumatismus, kutane Atopie wie Ekzem, oder Atmungsatopie, oder sogar Zahnfleischhypertrophie ausgewählt sind.

15. Pharmazeutische Zusammensetzung, **dadurch gekennzeichnet, dass** sie mindestens eine der Verbindungen wie in einem der Ansprüche 1 bis 10 definiert in einen physiologisch unbedenklichen Träger umfasst.

16. Zusammensetzung nach Anspruch 15, **dadurch gekennzeichnet, dass** die Konzentration an Verbindung(en) nach einem der Ansprüche 1 bis 10 zwischen 0,001 Gew.-% und 10 Gew.-% in Bezug auf das Gesamtgewicht der Zusammensetzung beträgt.

17. Zusammensetzung nach Anspruch 16, **dadurch gekennzeichnet, dass** die Konzentration an Verbindung(en) nach einem der Ansprüche 1 bis 10 zwischen 0,01 Gew.-% und 1 Gew.-% in Bezug auf das Gesamtgewicht der Zusammensetzung beträgt.

18. Kosmetikzusammensetzung, **dadurch gekennzeichnet, dass** sie mindestens eine der Verbindungen wie in einem der Ansprüche 1 bis 10 definiert in einem physiologisch unbedenklichen Medium umfasst.

19. Zusammensetzung nach Anspruch 18, **dadurch gekennzeichnet, dass** die Konzentration an Verbindung(en) nach einem der Ansprüche 1 bis 10 zwischen 0,001 Gew.-% und 3 Gew.-% in Bezug auf das Gesamtgewicht der Zusammensetzung beträgt.

20. Nichttherapeutische Verwendung einer Kosmetikzusammensetzung wie in einem der Ansprüche 1 bis 10 definiert für die Vorbeugung und/oder Behandlung von Alterungsanzeichen und/oder trockener Haut.

21. Nichttherapeutische Verwendung einer Kosmetikzusammensetzung wie in einem der Ansprüche 1 bis 10 definiert für die Körper- oder Haarhygiene.

22. Kosmetikverfahren zur Verbesserung des Hautbilds, **dadurch gekennzeichnet, dass** man eine Zusammensetzung wie in einem der Ansprüche 1 bis 10 definiert auf die Haut aufträgt.

## Revendications

1. Composés **caractérisés en ce qu'**ils correspondent à la formule (I) ci-après : 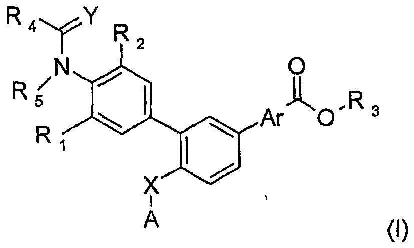 dans laquelle :
- R₁ est un atome d'hydrogène, un radical alkyle de 1 à 4 atomes de carbone ou un radical -CF₃ ;
- R₂ est un atome d'hydrogène, un radical alkyle ou alcoxy de 1 à 4 atomes de carbone ou un atome de chlore ;
- R₃ est un atome d'hydrogène ou un radical alkyle linéaire ou ramifié de 1 à 10 atomes de carbone éventuellement substitué par un groupement méthoxy, ou un radical alkyle linéaire ou ramifié de 1 à 10 atomes de carbone contenant une fonction éther ;
- R₄ est un atome d'hydrogène ou un radical alkyle de 1 à 3 atomes de carbone ;
- R₅ est un atome d'hydrogène ou un radical alkyle de 1 à 3 atomes de carbone ;
- ou de manière alternative R₄ et R₅ forment, conjointement avec la liaison -N-C(=Y)-, un cycle de type pyrrolidine, pyrrolidinone, pipéridine ou pipéridinone ;
- Y représente deux atomes d'hydrogène ou un hétéroatome, par exemple oxygène ou soufre ;
- Ar représente un cycle 1,4-phényle, 2,5-pyridyle, 5,2-pyridyle ou 2,5-thiophényle ;
- X représente un atome d'oxygène éventuellement substitué par une chaîne alkyle ou alkylamine ou une liaison simple C-C ;
- A représente un atome d'hydrogène ou la formule suivante : 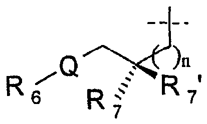
dans laquelle
o Q est un atome d'oxygène ou une liaison -NH- ;
o R₆ représente un atome d'hydrogène, un radical alkyle de 1 à 6 atomes de carbone, un radical cycloalkyle de 3 à 6 atomes de carbone ou un radical -C(O)CH₃ ou -C(O)CH₂CH₃ ;
o R₇ et R_{7'} représentent, indépendamment l'un de l'autre, un atome d'hydrogène ou un groupement hydroxyle, à condition que R₇ et R_{7'} ne soient pas simultanément un groupement hydroxyle ;
o n vaut 0, 1, 2, 3, 4 ou 5 ;
et les sels des composés de formule (I) lorsque R₃ représente un atome d'hydrogène, OH et aussi les isomères géométriques desdits composés de formule (I).

2. Composés selon la revendication 1, **caractérisés en ce qu'**ils sont sous la forme de sels de métaux alcalins ou de métaux alcalino-terreux, de sels de zinc ou de sels d'une amine organique ou d'un partenaire acide lorsque le composé est lui-même basique.

3. Composé selon la revendication 1, **caractérisé en ce que** le radical alkyle de 1 à 3 atomes de carbone est choisi parmi les radicaux méthyle, éthyle, i-propyle et n-propyle.

4. Composé selon la revendication 1, **caractérisé en ce que** le radical alkyle de 1 à 4 atomes de carbone est choisi parmi les radicaux méthyle, éthyle, i-propyle, i-butyle et t-butyle.

5. Composé selon la revendication 1, **caractérisé en ce que** le radical alkyle de 1 à 10 atomes de carbone est choisi parmi les radicaux méthyle, éthyle, propyle, i-propyle, butyle, i-butyle, t-butyle, pentyle, hexyle, heptyle, octyle, nonyle et dodécyle.

6. Composé selon la revendication 1, **caractérisé en ce que** le radical alcoxy contenant de 1 à 4 atomes de carbone est choisi parmi les radicaux méthoxy, éthoxy, isopropyloxy et tertio-butoxy.

7. Composé selon la revendication 1, **caractérisé en ce que** le radical cycloalkyle de 3 à 5 atomes de carbone est choisi parmi les radicaux cyclopropyle, cyclopentyle et cyclohexyle.

8. Composés de formule (I) selon l'une quelconque des revendications précédentes, choisis parmi :
1. le 3"-tertio-butyl-4"-diéthylamino-4'-hydroxy[1,1';3',1"]terphényl-4-carboxylate d'éthyle
2. le 3"-tertio-butyl-4"-diéthylamino-4'-(4-hydroxybutoxy)-[1,1';3',1"]terphényl-4-carboxylate d'éthyle
3. l'acide 3"-tertio-butyl-4"-diéthylamino-4'-(4-hydroxybutoxy)-[1,1';3',1"]terphényl-4-carboxylique
4. le 4"-(acétyléthylamino)-3"-tertio-butyl-4'-(4-hydroxybutoxy)-[1,1';3',1"]terphényl-4-carboxylate d'éthyle
5. l'acide 4"-(acétyléthylamino)-3"-tertio-butyl-4'-(4-hydroxybutoxy)-[1,1';3',1"]terphényl-4-carboxylique
6. l'acide 3"-tertio-butyl-4"-diéthylamino-4'-(2-hydroxyéthoxy)-[1,1';3',1"]terphényl-4-carboxylique
7. le 3"-tertio-butyl-4"-diéthylamino-4'-(3-hydroxypropoxy)-[1,1';3',1"]terphényl-4-carboxylate d'éthyle
8. l'acide 3"-tertio-butyl-4"-diéthylamino-4'-(3-hydroxypropoxy)-[1,1';3',1"]terphényl-4-carboxylique
9. le 4"-diéthylamino-4'-(3-hydroxypropoxy)-[1,1';3',1"]terphényl-4-carboxylate d'éthyle
10. l'acide 4"-diéthylamino-4'-(3-hydroxypropoxy)-[1,1';3',1"]terphényl-4-carboxylique
11. le 4"-diéthylamino-3"-éthyl-4'-(3-hydroxypropoxy)-[1,1';3',1"]terphényl-4-carboxylate d'éthyle
12. l'acide 4"-diéthylamino-3"-éthyl-4'-(3-hydroxypropoxy)-[1,1';3',1"]terphényl-4-carboxylique
13. l'acide 4"-diéthylamino-3"-éthyl-4'-(2-hydroxyéthoxy)-[1,1';3',1"]terphényl-4-carboxylique
14. le 4"-diéthylamino-3"-éthyl-4'-(2-hydroxyéthoxy)-[1,1';3',1"]terphényl-4-carboxylate d'éthyle
15. le 3"-tertio-butyl-4"-diéthylamino-4'-(2-hydroxyéthoxy)-[1,1';3',1"]terphényl-4-carboxylate d'éthyle
16. le 4"-diéthylamino-4'-(3-hydroxypropoxy)-3"-méthyl[1,1';3',1"]terphényl-4-carboxylate d'éthyle
17. l'acide 4"-diéthylamino-4'-(3-hydroxypropoxy)-3"-méthyl[1,1';3',1"]terphényl-4-carboxylique
18. le 3"-tertio-butyl-4'-(4-hydroxybutoxy)-4"-pyrrolidin-1-yl[1,1';3',1"]terphényl-4-carboxylate d'éthyle
19. l'acide 3"-tertio-butyl-4'-(4-hydroxybutoxy)-4"-pyrrolidin-1-yl[1,1';3',1"]terphényl-4-carboxylique
20. le 4'-(4-hydroxybutoxy)-4"-pyrrolidin-1-yl-3"-trifluorométhyl[1,1';3',1"]terphényl-4-carboxylate d'éthyle
21. l'acide 4'-(4-hydroxybutoxy)-4"-pyrrolidin-1-yl-3"-trifluorométhyl[1,1';3',1"]terphényl-4-carboxylique
22. le 3"-tertio-butyl-4'-(3-hydroxypropoxy)-4"-pyrrolidin-1-yl[1,1';3',1"]terphényl-4-carboxylate d'éthyle
23. l'acide 3"-tertio-butyl-4'-(3-hydroxypropoxy)-4"-pyrrolidin-1-yl[1,1';3',1"]terphényl-4-carboxylique
24. le 3"-tertio-butyl-4'-(2-hydroxyéthoxy)-4"-pyrrolidin-1-yl[1,1';3',1"]terphényl-4-carboxylate d'éthyle
25. l'acide 3"-tertio-butyl-4'-(2-hydroxyéthoxy)-4"-pyrrolidin-1-yl[1,1';3',1"]terphényl-4-carboxylique
26. l'acide 4'-(3-hydroxypropoxy)-4"-pyrrolidin-1-yl[1,1';3',1"]terphényl-4-carboxylique
27. l'acide 3"-tertio-butyl-4"-diéthylamino-4'-hydroxy[1,1';3',1"]terphényl-4-carboxylique
28. le 4"-diéthylamino-4'-hydroxy-3"-trifluorométhyl[1,1';3',1"]terphényl-4-carboxylate d'éthyle
29. l'acide 4"-diéthylamino-4'-hydroxy-3"-trifluorométhyl[1,1';3',1"]terphényl-4-carboxylique
30. l'acide 3"-tertio-butyl-4"-diéthylamino-4'-(4-isopropylaminobutoxy)-[1,1';3',1"]terphényl-4-carboxylique
31. l'acide 3"-tertio-butyl-4'-(4-isopropylaminobutoxy)-4"-pyrrolidin-1-yl[1,1';3',1"]terphényl-4-carboxylique
32. le 3"-tertio-butyl-4"-diéthylamino-4'-(3-hydroxy-propyl)-[1,1';3',1"]terphényl-4-carboxylate d'éthyle
33. l'acide 3"-tertio-butyl-4"-diéthylamino-4'-(3-hydroxypropyl)-[1,1';3',1"]terphényl-4-carboxylique
34. le 3"-tertio-butyl-4"-diéthylamino-4'-(2,3-dihydroxypropyl)-[1,1';3',1"]terphényl-4-carboxylate d'éthyle
35. l'acide 3"-tertio-butyl-4"-diéthylamino-4'-(2,3-dihydroxypropyl)-[1,1';3',1"]terphényl-4-carboxylique
36. le 4"-(acétyléthylamino)-3"-tertio-butyl-4'-(3-hydroxypropyl)-[1,1';3',1"]terphényl-4-carboxylate d'éthyle
37. l'acide 4"-(acétyléthylamino)-3"-tertio-butyl-4'-(3-hydroxypropyl)-[1,1';3',1"]terphényl-4-carboxylique
38. le 3"-tertio-butyl-4'-(3-cyclopropylaminopropyl)-4"-diéthylamino[1,1';3',1"]terphényl-4-carboxylate d'éthyle
39. l'acide 3"-tertio-butyl-4'-(3-cyclopropylaminopropyl)-4"-diéthylamino[1,1';3',1"]terphényl-4-carboxylique
40. le 3"-tertio-butyl-4'-(3-cyclopentylaminopropyl)-4"-diéthylamino[1,1';3',1"]terphényl-4-carboxylate d'éthyle
41. l'acide 3"-tertio-butyl-4'-(3-cyclopentylaminopropyl)-4"-diéthylamino[1,1';3',1"]terphényl-4-carboxylique
42. le 3"-tertio-butyl-4'-(3-cyclohexylaminopropyl)-4"-diéthylamino[1,1';3',1"]terphényl-4-carboxylate d'éthyle
43. l'acide 3"-tertio-butyl-4'-(3-cyclohexylaminopropyl)-4"-diéthylamino[1,1';3',1"]terphényl-4-carboxylique
44. le 3"-tertio-butyl-4'-(3-tertio-butylaminopropyl)-4"-diéthylamino[1,1';3',1"]terphényl-4-carboxylate d'éthyle
45. l'acide 3"-tertio-butyl-4'-(3-tertio-butylaminopropyl)-4"-diéthylamino[1,1';3',1"]terphényl-4-carboxylique
46. le 4"-(acétyléthylamino)-3"-tertio-butyl-4'-(3-cyclopropylaminopropyl)-[1,1';3',1"]terphényl-4-carboxylate d'éthyle
47. l'acide 4"-(acétyléthylamino)-3"-tertio-butyl-4'-(3-cyclopropylaminopropyl)-[1,1';3',1"]terphényl-4-carboxylique
48. le 3"-tertio-butyl-4"-diéthylamino-4'-(3-isopropylaminopropyl)-[1,1';3',1"]terphényl-4-carboxylate d'éthyle
49. l'acide 3"-tertio-butyl-4"-diéthylamino-4'-(3-isopropylaminopropyl)-[1,1';3',1"]terphényl-4-carboxylique
50. le 4'-(3-aminopropyl)-3"-tertio-butyl-4"-diéthylamino[1,1';3',1"]terphényl-4-carboxylate d'éthyle
51. l'acide 4'-(3-aminopropyl)-3"-tertio-butyl-4"-diéthylamino[1,1';3',1"]terphényl-4-carboxylique
52. le chlorhydrate de [3"-tertio-butyl-4-carboxy-4'-(3-hydroxypropyl)-[1,1';3',1"]terphényl-4"-yl]-diéthylamine
53. l'acide 3"-tertio-butyl-4'-(2-hydroxyéthoxy)-4"-(2-oxopyrrolidin-1-yl)-[1,1';3',1"]terphényl-4-carboxylique
54. l'acide 3"-tertio-butyl-4"-éthylamino-4'-(3-hydroxypropoxy)-[1,1';3',1"]terphényl-4-carboxylique
55. l'acide 4'-(3-acétoxypropoxy)-3"-tertio-butyl-4"-diéthylamino[1,1';3',1"]terphényl-4-carboxylique
56. l'acide 3"-tertio-butyl-4"-diéthylamino-4'-(3-propionyloxypropoxy)-[1,1';3',1"]terphényl-4-carboxylique
57. le 3"-tertio-butyl-4"-diéthylamino-4'-(3-hydroxypropoxy)-[1,1';3',1"]terphényl-4-carboxylate de méthyle
58. le 3"-tertio-butyl-4"-diéthylamino-4'-(3-hydroxypropoxy)-[1,1';3',1"]terphényl-4-carboxylate d'isopropyle
59. le 3"-tertio-butyl-4"-diéthylamino-4'-(3-hydroxypropoxy)-[1,1';3',1"]terphényl-4-carboxylate d'isobutyle
60. l'acide 3"-tertio-butyl-4"-diéthylamino-4'-(3-hydroxypropoxy)-5"-méthyl[1,1';3',1"]terphényl-4-carboxylique
61. l'acide 4"-diéthylamino-4'-(3-hydroxypropoxy)-3"-isopropyl-5"-méthyl[1,1';3',1"]terphényl-4-carboxylique
62. l'acide 3"-tertio-butyl-5"-chloro-4"-diéthylamino-4'-(3-hydroxypropoxy)-[1,1';3',1"]terphényl-4-carboxylique
63. l'acide 4"-diéthylamino-4'-(3-hydroxypropoxy)-3",5"-diisopropyl[1,1';3',1"]terphényl-4-carboxylique
64. l'acide 3",5"-di-tertio-butyl-4"-diéthylamino-4'-(3-hydroxypropoxy)-[1,1';3',1"]terphényl-4-carboxylique
65. l'acide 4"-diéthylamino-4'-(3-hydroxypropoxy)-3"-trifluorométhyl[1,1';3',1"]terphényl-4-carboxylique
66. l'acide 3"-tertio-butyl-4"-(éthylméthylamino)-4'-(2-hydroxyéthoxy)-[1,1';3',1"]terphényl-4-carboxylique
67. l'acide 3"-tertio-butyl-4"-diméthylamino-4'-(2-hydroxyéthoxy)-[1,1';3',1"]terphényl-4-carboxylique
68. l'acide 3"-tertio-butyl-4"-(éthylisopropylamino)-4'-(2-hydroxyéthoxy)-[1,1';3',1"]terphényl-4-carboxylique
69. l'acide 3"-tertio-butyl-4"-(éthylpropylamino)-4'-(2-hydroxyéthoxy)-[1,1';3',1"]terphényl-4-carboxylique
70. l'acide 3"-tertio-butyl-4"-dipropylamino-4'-(2-hydroxyéthoxy)-[1,1';3',1"]terphényl-4-carboxylique
71. l'acide 3"-tertio-butyl-4"-(éthylpropionylamino)-4'-(2-hydroxyéthoxy)-[1,1';3',1"]terphényl-4-carboxylique
72. l'acide 6-[3'-tertio-butyl-4'-diéthylamino-6-(2-hydroxyéthoxy)biphényl-3-yl]nicotinique
73. l'acide 5-[3'-tertio-butyl-4'-diéthylamino-6-(2-hydroxyéthoxy)biphényl-3-yl]pyridine-2-carboxylique
74. l'acide 5-[3'-tertio-butyl-4'-diéthylamino-6-(2-hydroxyéthoxy)biphényl-3-yl]thiophène-2-carboxylique
75. l'acide 3"-tertio-butyl-4'-(2-hydroxyéthoxy)-5"-méthyl-4"-pyrrolidin-1-yl[1,1';3',1"] terphényl-4-carboxylique
76. l'acide 3"-tertio-butyl-5"-chloro-4'-(2-hydroxy-éthoxy)-4"-pyrrolidin-1-yl[1,1';3',1"]terphényl-4-carboxylique
77. l'acide 4'-(2-hydroxyéthoxy)-3"-isopropyl-4"-pyrrolidin-1-yl[1,1';3',1"]terphényl-4-carboxylique
78. l'acide 3"-éthyl-4'-(2-hydroxyéthoxy)-4"-pyrrolidin-1-yl[1,1';3',1"]terphényl-4-carboxylique
79. l'acide 4'-(2-hydroxyéthoxy)-3",5"-diisopropyl-4"-pyrrolidin-1-yl[1,1';3',1"]terphényl-4-carboxylique
80. l'acide 3",5"-diéthyl-4'-(2-hydroxyéthoxy)-4"-pyrrolidin-1-yl[1,1';3',1"]terphényl-4-carboxylique
81. l'acide 3",5"-diméthyl-4'-(2-hydroxyéthoxy)-4"-pyrrolidin-1-yl[1,1';3',1"]terphényl-4-carboxylique
82. l'acide 4'-(2-acétoxyéthoxy)-3"-tertio-butyl-4"-pyrrolidin-1-yl[1,1';3',1"]terphényl-4-carboxylique
83. l'acide 4'-(2-propionyloxyéthoxy)-3"-tertio-butyl-4"-pyrrolidin-1-yl[1,1';3',1"]terphényl-4-carboxylique
84. le 3"-tertio-butyl-4'-(2-hydroxyéthoxy)-4"-pyrrolidin-1-yl[1,1';3',1"]terphényl-4-carboxylate de méthyle
85. le 3"-tertio-butyl-4'-(2-hydroxyéthoxy)-4"-pyrrolidin-1-yl[1,1';3',1"]terphényl-4-carboxylate d'isopropyle
86. le 3"-tertio-butyl-4'-(2-hydroxyéthoxy)-4"-pyrrolidin-1-yl[1,1';3',1"]terphényl-4-carboxylate d'isobutyle
87. le 3"-tertio-butyl-4'-(3-hydroxypropoxy)-4"-pyrrolidin-1-yl[1,1';3',1"]terphényl-4-carboxylate d'éthyle
88. le 3"-tertio-butyl-5"-chloro-4'-(2-hydroxyéthoxy)-4"-pyrrolidin-1-yl[1,1';3',1"]terphényl-4-carboxylate d'éthyle
89. l'acide 6-[3'-tertio-butyl-6-(2-hydroxyéthoxy)-4'-pyrrolidin-1-ylbiphényl-3-yl]nicotinique
90. l'acide 5-[3'-tertio-butyl-6-(2-hydroxyéthoxy)-4'-pyrrolidin-1-ylbiphényl-3-yl]pyridine-2-carboxylique
91. le 6-[3'-tertio-butyl-6-(2-hydroxyéthoxy)-4'-pyrrolidin-1-ylbiphényl-3-yl]nicotinate d'éthyle
92. le 3"-tertio-butyl-4'-(3-hydroxypropyl)-4"-pyrrolidin-1-yl[1,1';3',1"]terphényl-4-carboxylate d'éthyle
93. l'acide 3"-tertio-butyl-4'-(3-hydroxypropyl)-4"-pyrrolidin-1-yl[1,1';3',1"]terphényl-4-carboxylique
94. l'acide 3"-tertio-butyl-4'-(2-hydroxyéthoxy)-4"-(2-oxopyrrolidin-1-yl)-[1,1';3',1"]terphényl-4-carboxylique
95. l'acide 3"-tertio-butyl-4'-(2-hydroxyéthoxy)-4"-(2-oxopipérid-1-yl)-[1,1';3',1"]terphényl-4-carboxylique
96. l'acide 3"-tertio-butyl-4'-(2-hydroxyéthoxy)-4"-pipérid-1-yl[1,1';3',1"]terphényl-4-carboxylique
97. l'acide 5-[3'-tertio-butyl-6-(2-hydroxyéthoxy)-4'-pyrrolidin-1-ylbiphényl-3-yl]thiophène-2-carboxylique.

9. Composés selon la revendication 1, **caractérisés en ce qu'**ils possèdent au moins l'une des caractéristiques suivantes :
- R₁ est un atome d'hydrogène ou un radical t-butyle ou i-propyle ;
- R₂ est un atome d'hydrogène ou un radical t-butyle ou i-propyle ;
- R₃ est un atome d'hydrogène ou un radical éthyle ;
- R₄ et R₅ sont, indépendamment l'un de l'autre, un radical méthyle ou éthyle ou forment ensemble un cycle pyrrolidine ;
- A est tel que défini ci-dessus, où R₆ représente un atome d'hydrogène, un radical i-propyle ou t-butyle, un radical cycloalkyle de 3 à 6 atomes de carbone ou un radical -C(O)CH₃ ou -C(O)CH₂CH₃.

10. Composés selon la revendication 9, **caractérisés en ce qu'**ils possèdent l'ensemble des caractéristiques suivantes :
- R₁ est un atome d'hydrogène ou un radical t-butyle ou i-propyle ;
- R₂ est un atome d'hydrogène ou un radical t-butyle ou i-propyle ;
- R₃ est un atome d'hydrogène ou un radical éthyle ;
- R₄ et R₅ sont, indépendamment l'un de l'autre, un radical méthyle ou éthyle ou forment ensemble un cycle pyrrolidine ;
- A est tel que défini ci-dessus, où R₆ représente un atome d'hydrogène, un radical i-propyle ou t-butyle, un radical cycloalkyle de 3 à 6 atomes de carbone ou un radical -C(O)CH₃ ou -C(O)CH₂CH₃.

11. Composés selon l'une quelconque des revendications 1 à 10, comme médicaments.

12. Utilisation d'un composé selon l'une quelconque des revendications 1 à 10, dans l'élaboration d'une composition destinée à traiter :
- les affections dermatologiques associées à un trouble de kératinisation lié à la différenciation et à la prolifération cellulaires ;
- l'ichtyose, les conditions ichtyosiformes, la maladie de Darier, la kératodermie palmo-plantaire, la leucoplasie et les conditions leucoplasiformes, et le lichen cutané ou muqueux (buccal) ;
- les affections dermatologiques à composante immuno-allergique inflammatoire, avec ou sans trouble de la prolifération cellulaire ;
- les troubles cutanés provoqués par une exposition au rayonnement UV, le vieillissement de la peau photo-induit ou chronologique, ou les kératoses et les pigmentations actiniques ;
- les pathologies associées à un vieillissement chronologique ou actinique de la peau ;
- les proliférations dermiques ou épidermiques bénignes ou malignes, d'origine virale ou non virale ;
- les proliférations pouvant être induites par un rayonnement ultraviolet ;
- les lésions cutanées précancéreuses ;
- les dermatoses immunes ;
- les maladies bulleuses immunes ;
- les maladies du collagène ;
- les affections dermatologiques à composante immunologique ;
- les troubles ophtalmiques ;
- les stigmates d'une atrophie épidermique et/ou dermique induite par des corticostéroïdes locaux ou systémiques, ou toute autre forme d'atrophie cutanée ;
- les affections de la peau d'origine virale ;
- les troubles cutanés provoqués par une exposition au rayonnement UV, le vieillissement de la peau photo-induit ou chronologique, ou les kératoses et les pigmentations actiniques ;
- les pathologies associées à un vieillissement chronologique ou actinique de la peau ;
- les troubles de la fonction sébacée ;
- les troubles de la cicatrisation ou les vergetures ; ou
- les troubles de pigmentation.

13. Utilisation selon la revendication 12, **caractérisée en ce que** les affections dermatologiques associées à un trouble de kératinisation lié à la différenciation et à la prolifération cellulaires sont choisies parmi l'acné commune, les comédons, les leucocytes polynucléaires, l'acné rosacée, l'acné nodulokystique, l'acné conglobata, l'acné sénile, et les acnés secondaires telles que l'acné solaire, les acnés médicamenteuses, ou l'acné professionnelle.

14. Utilisation selon la revendication 12, **caractérisée en ce que** les affections dermatologiques à composante immuno-allergique inflammatoire, avec ou sans trouble de la prolifération cellulaire, sont choisies parmi toutes les formes de psoriasis, qu'il s'agisse de psoriasis cutané, muqueux ou unguéal, voire de psoriasis rhumatoïde, l'atopie cutanée, telle que l'eczéma, ou l'atopie respiratoire, voire l'hypertrophie gingivale.

15. Composition pharmaceutique, **caractérisée en ce qu'**elle comprend, dans un support physiologiquement acceptable, au moins l'un des composés tels que définis selon l'une quelconque des revendications 1 à 10.

16. Composition selon la revendication 15, **caractérisée en ce que** la concentration en composé(s) selon l'une quelconque des revendications 1 à 10 est comprise entre 0,001% et 10% en poids par rapport au poids total de la composition.

17. Composition selon la revendication 16, **caractérisée en ce que** la concentration en composé(s) selon l'une quelconque des revendications 1 à 10 est comprise entre 0,01% et 1% en poids par rapport au poids total de la composition.

18. Composition cosmétique, **caractérisée en ce qu'**elle comprend, dans un milieu physiologiquement acceptable, au moins l'un des composés tels que définis selon l'une quelconque des revendications 1 à 10.

19. Composition selon la revendication 18, **caractérisée en ce que** la concentration en composé(s) selon l'une quelconque des revendications 1 à 10 est comprise entre 0,001% et 3% en poids par rapport au poids total de la composition.

20. Utilisation non thérapeutique d'une composition cosmétique telle que définie selon l'une quelconque des revendications 1 à 10, pour empêcher et/ou traiter les signes de peau vieillissante et/ou sèche.

21. Utilisation non thérapeutique d'une composition cosmétique telle que définie selon l'une quelconque des revendications 1 à 10, pour l'hygiène du corps ou des cheveux.

22. Procédé cosmétique destiné à améliorer l'aspect de la peau, caractérisé en qu'une composition telle que définie selon l'une quelconque des revendications 1 à 10 est appliquée à la peau.
